# EUROPÄISCHE PATENTSCHRIFT

(11) **EP 2 313 403 B1**
(45) Veröffentlichungstag und Bekanntmachung des Hinweises auf die Patenterteilung: **08.04.2015**
(21) Anmeldenummer: 09768890.7
(22) Anmeldetag: 28.05.2009
(51) Int. Cl.: C07D 417/04, C07D 417/14, A61K 31/427, A61P 35/00

(54) **THIAZOLYL-PIPERIDINDERIVATE**
THIAZOLYL PIPERDINE DERIVATIVES
DÉRIVÉS DE THIAZOLYL-PIPERIDINE

(30) Priorität: 23.06.2008 DE 102008029734
(43) Veröffentlichungstag der Anmeldung: 27.04.2011
(73) Patentinhaber: Merck Patent GmbH, 64293 Darmstadt (DE)
(72) Erfinder: STIEBER, Frank, 69121 Heidelberg (DE); HEINRICH, Timo, 64823 Gross-Umstadt (DE); WIENKE, Dirk, 64287 Darmstadt (DE)
(86) Internationale Anmeldenummer: PCT/EP2009/003817
(87) Internationale Veröffentlichungsnummer: WO 2009/156041

(56) Entgegenhaltungen:
- WO-A2-2007/020213
- WO-A2-2007/064553
- TERVO, A.J. ET AL.: "Discovering Inhibitors of Human Sirtuin type 2: Novel Structural Scaffolds" JOURNAL OF MEDICINAL CHEMISTRY, Bd. 49, Nr. 24, 9. Februar 2006 (2006-02-09), Seiten 7239-7241, XP002573020

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, zur Behandlung von Krankheiten, die mit einer Erhöhung des Sphingosinphosphatspiegels einhergehen, ferner pharmazeutische Zusammensetzungen, die diese Verbindungen enthalten.

Im einzelnen betrifft die vorliegende Erfindung Verbindungen der Formel I, die bevorzugt das Enzym Sphingosinkinase 1 hemmen, das den Sphingosinphosphatspiegel durch Phosphorylierung von Sphingosin reguliert, Zusammensetzungen, die diese Verbindungen enthalten, sowie Verfahren zu ihrer Verwendung zur Behandlung von Krankheiten und Leiden wie Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Herzerkrankungen, Wundheilung oder Transplantatabstossung. Insbesondere eignen sich die erfindungsgemäßen Verbindungen zur Therapie von Krebserkrankungen.

Sphingosinphosphat gehört zu der Molekülfamilie der Sphingolipide, die, neben ihrer Rolle als strukturelle Bausteine von Zellmembranen, auch wichtige Funktionen als extra- und intrazelluläre Signalmoleküle ausüben. Sphingosinphosphat (S1P) entsteht in der Zelle aus Sphingomyelin, das zunächst zu Ceramid und Sphingosin abgebaut und Letzteres durch Sphingosinkinasen phosphoryliert wird. Von den zwei bislang identifizierten Sphingosinkinasen wird Sphingosinkinase 1 (SphK1) die größere Bedeutung bei der Bildung von S1 P im Serum zugeschrieben (Zemann et al., 2006 Blood Vol 107 Seite 1454). Während Ceramid und Sphingosin Zelltod und Zellwachstumshemmung induzieren (Kolesnick 2002, J Clin Invest Vol 110, Seite 3; Ogretmen et al. 2004 Nat Rev Cancer Vol 4, Seite 604), hat Sphingosinphosphat einen gegenteiligen Effekt auf die Zelle und steigert die Widerstandsfähigkeit gegen Apoptose, das Zellwachstum und den Ausstoß von Botenstoffen, die die Durchblutung des Gewebes und damit auch von Tumoren fördern (Cuvilier et al. 1996, Nature Vol 381, Seite 800; Perez et al. 1997, Nat Med Vol 3, Seite 1228). Das Verhältnis von Ceramid und Sphingosin auf der einen Seite und S1P auf der anderen entscheidet folglich über das Zellwachstum und durch Inhibierung der SphK 1 kann somit nicht nur die Bildung des wachstumsfördernden Sphingosinphosphats unterbunden, sondern auch die zelluläre Konzentration der wachstumshemmenden Moleküle Ceramid und Sphingosin erhöht werden.
Eine Vielzahl von zellulären Effekten, die durch S1 P ausgelöst werden, wird durch Sekretion des S1P und dessen Bindung an bislang 5 verschiedene G-Protein-gekoppelte Rezeptoren (bezeichnet als S1P₁₋₅) vermittelt. Die Signalweiterleitung erfolgt wiederum über verschiedene G-Proteine (Gᵢ, G_{q}, G_{12/13}), so dass eine Reihe unterschiedlicher zellulärer Signalwege, wie z.B. ERK oder PI3K aktiviert werden, die insbesondere bei Krebsentstehung und -wachstum wichtig sind. Eine wachsende Zahl an Publikationen zeigt außerdem, dass S1P ein wichtiger Faktor bei der tumoralen Angiogense ist. Angiogenese ist ein wichtiger Vorgang beim Tumorwachstum, durch den ausgehend von bereits bestehenden Blutgefäßen neue gebildet werden und somit die Versorgung des Tumors mit Nährstoffen gesichert wird. Aus diesem Grund ist die Hemmung der Angiogenese ein wichtiger Ansatzpunkt der Krebs- und Tumortherapie. (Folkman, 2007, Nature Reviews Drug Discovery Vol. 6, Seite 273-286). S1P stimuliert chemotaktische Bewegung von Endothelzellen und induziert die Differenzierung zu multizellulären Strukturen, beides frühe Schritte bei der Bildung von neuen Blutgefäßen (Lee et al. 1999 Biochem Biophys Res Commun Vol 264 Seite 325; Argraves et al. 2004 J Biol Chem Vol 279 Seite 50580). Außerdem fördert S1 P die Wanderung von knochenmarkstämmigen Endothelvorläuferzellen zu neovaskulären Initiationsstellen (Annabi et al. 2003 Exp Hematology Vol 31 Seite 640) und transaktiviert den Rezeptor von VEGF, einer der wichtigsten proangiogenen Faktoren insbesondere in der Tumorbiologie (Tanimoto et al. 2002 J Biol Chem Vol 277 Seite 42997; Endo et al. 2002 J Biol Chem Vol 277 Seite 23747). Ein direkter Beweis der Aktivität von S1P in der Tumorangiogenese konnte durch Experimente mit einem Antikörper, der an S1 P spezifisch bindet, erbracht werden. Der S1P-Antikörper inhibierte die Wanderung und die Gefäßbildung von Endothelzellen in vitro, blockierte die S1P-abhängige Sekretion von proangiogenen Faktoren wie VEGF, IL-8 und IL-6 in vitro und in vivo und reduzierte signifikant das Wachstum von Tumormodellen der Brust, Lunge und Ovarien in Maus-Xenograftexperimenten (Visentin 2006 Cancer Cell Vol 9 Seite 225).
Daneben hat S1 P auch intrazelluläre Funktionen, wie zum Beispiel die Aktivierung des Transkriptionsfaktors NF-κB, der bei der Apoptoseresistenz von Krebszellen eine große Rolle spielt (Xia et al. 2002 J Biol Chem Vol 277 Seite 7996). Allerdings sind die intrazellulären Interaktionspartner von S1 P noch nicht identifiziert.
Daraus folgert, dass im Gegensatz zu einer ebenfalls denkbaren Intervention mit der krebsfördernden Wirkung von S1 P durch pharmakologische Blockade der extrazellulären Rezeptoren eine Inhibierung des für die S1P-Bildung verantwortlichen Enzyms SphK1 den Vorteil aufweist, damit auch die intrazellulären Aktivitäten von S1P zu unterbinden. Unterstützt wird dieser Ansatz durch Untersuchungen von Xia et al. (2000 Curr Biol Vol 10 Seite 1527), die zeigen, dass nichttumorgene Fibroblasten durch ektopische Expression von SphK1 transformiert werden und in Mäusen Tumore bilden können. SphK1 kann somit als Onkogen klassifiziert werden. In verschiedene Expressionstudien konnten erhöhte SphK1-mRNA-Konzentrationen in Tumorgeweben des Gehirns, der Brust, der Lunge, der Eierstöcke, des Magens, der Gebärmutter, der Nieren sowie des Dünn- und des Dickdarms gegenüber gesundem Gewebe festgestellt werden (French et al. 2003 Cancer Research Vol. 63 Seite 5962; Johnson et al. 2005 J Histochem Cytochem Vol 53 Seite 1159; Van Brocklyn et al. 2005 J Neuropathol Exp Neurol Vol 64 Seite 695). Darüber hinaus korreliert eine erhöhte Expression von SphK1 mit schlechterer Prognose in Patienten mit Glioblastoma Multiform (Van Brocklyn et al. 2005 J Neuropathol Exp Neurol Vol 64 Seite 695).
Eine wichtige Rolle kommt SphK1 bei der Modulierung der durch Chemotherapeutika induzierten Apoptose von Krebszellen zu. So erhöht sich durch Überexpremierung von SphK1 die Resistenz von Brustkrebs-, Prostatakrebs und Leukämiezellen gegenüber Chemotherapeutika wie Anthrazyklinen, Docetaxel, Camptothecin oder Doxorubicin (Nava et al. 2002 Exp Cell Res Vol 281 Seite 115; Pchejetski 2005 Cancer Res Vol 65 Seite 11667; Bonhoure 2006 Leukemia Vol 20 Seite 95). Es konnte gezeigt werden, dass die vermehrte Anwesenheit von SphK1 zu einer Verschiebung des Ceramid/S1P-Gleichgewichts in Richtung des apoptoseresistenzvermittelnden S1P führt. Ein möglicher Mechanismus ist dabei die Inhibierung des mitochondrialen Cytochrome C-Ausstoßes durch SphK1, was normalerweise ein frühes Ereignis beim programmierten Zelltod darstellt (Cuvilier et al. 2001 Blood Vol 98 Seite 2828; Bonhoure 2006 Leukemia Vol 20 Seite 95).
In umgekehrter Weise kann durch gezielte Blockade der SphK1-Expremierung mittels siRNA in Tumorzellmodellen verschiedener Indikationen wie Leukämie, Brustkrebs, Glioblastoma oder Prostatakrebs Apoptose ausgelöst oder die Wirkung von Chemotherapeutika gesteigert werden (Bonhoure 2006 Leukemia Vol 20 Seite 95; Taha et al. 2004 J Biol Chem Vol 279 Seite 20546; Taha et al. 2006 FASEB J Vol 20 Seite 482; Van Brocklyn et al. 2005 J Neuropathol Exp Neurol Vol 64 Seite 695; Pchejetski 2005 Cancer Res Vol 65 Seite 11667).
In einem Mausmodell konnte gezeigt werden, dass durch Überexpression von SphK1 degenerative Veränderungen von Kardiomyozyten und myocardiale Fibrose ausgelöst wird, die sich mit zunehmendem Alter der Versuchstiere verstärkte. Untermauert wird eine Funktion des S1P-Signalweges in Herzerkrankungen auch dadurch, dass in Mäuse, in denen die Expression des S1P3-Rezeptors gezielt unterbunden wurde, die Bildung von cardiovaskulären Fibrosen stark inhibiert ist (Takuwa 2008 Biochimica and Biophysica Acta in press*).* Auch in anderen Organen, wie zum Beispiel der Lunge kommt S1 P eine Rolle bei der Differenzierung von Fibroblasten hin zu Myofibroblasten und somit bei der Entstehung und Progression von fibrosen Erkrankungen zu (Kono et al. 2007 Am J Respir Cell Mol Biol Seite 395).

Es wurde gefunden, dass die erfindungsgemäßen Verbindungen eine spezifische Inhibierung der Sphingosinkinase 1, aber nicht der Sphingosinkinase 2 bewirken. Die erfindungsgemäßen Verbindungen zeigen bevorzugt eine vorteilhafte biologische Aktivität, die in den, zum Beispiel hierin beschriebenen, Tests nachweisbar ist. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.
Generell können alle soliden und nicht soliden Tumore mit den Verbindungen der Formel I behandelt werden, wie z.B. die Monozytenleukämie, Hirn-, Urogenital-, Lymphsystem-, Magen-, Kehlkopf- Ovarial- und Lungenkarzinom, darunter Lungenadenokarzinom und kleinzelliges Lungenkarzinom. Zu weiteren Beispielen zählen Prostata-, Bauchspeicheldrüsen- und Brustkarzinom.
Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch Inhibierung von SphK1 beeinflusst werden.
Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.
Der Wirt oder Patient kann jeglicher Säugerspezies angehören, z.B. einer Primatenspezies, besonders Menschen; Nagetieren, einschließlich Mäusen, Ratten und Hamstern, Kaninchen, Pferden, Rindern, Hunden, Katzen usw. Tiermodelle sind für experimentelle Untersuchungen von Interesse, wobei sie ein Modell zur Behandlung einer Krankheit des Menschen darstellen.
Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, die intrazelluläre S1P-Konzentration zu senken und darüber hinaus die Sekretion von angiogenesefördernden Substanzen zu blockieren oder Zelltod zu induzieren. Zum Testen in vitro können kultivierte Zellen aus einer Biopsieprobe oder etablierte Krebszelllinien, in denen SphK1 überexpremiert wir, verwendet werden.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden können.

### Verwendung

Wie in der Einleitung beschrieben sind SphK1, S1P und dessen Zelloberflächenrezeptoren S1P₁₋₅ in einer Vielzahl physiologischer und pathophysiologischer Prozesse involviert. Aus diesem Grund ist zu erwarten, dass die Hemmung der SphK1 durch die hier beschriebenen Substanzen bei verschiedenen Erkrankungen zu therapeutischen Zwecken genutzt werden kann.
Die Bildung von S1P durch SphK1 und die damit verbundene Verschiebung des Ceramid/S1P-Gleichgewichts führt, wie oben ausgeführt, dazu, dass die Zellen stärker proliferieren und widerstandsfähiger gegen apoptotische Stimuli werden. Daraus lässt sich eine generelle Funktion von SphK1 bei hyperproliferativen Erkrankungen wie Krebs, Psoriasis, Restenosen und Arteriosklerose ableiten. Die dieser Erfindung zugrunde liegenden Verbindungen der Formel I, die SphK1 hemmen und damit den S1P-Spiegel regulieren und/oder modulieren, Zusammensetzungen, die diese Verbindungen enthalten, sowie die beschriebenen Verfahren können somit zur Behandlung dieser Krankheiten eingesetzt werden. Generell können alle soliden und nicht soliden Tumore mit den Verbindungen der Formel X behandelt werden, wie z.B. die Monozytenleukämie, Hirn-, Urogenital-, Lymphsystem-, Magen-, Kehlkopf- Ovarial- und Lungenkarzinom, darunter Lungenadenokarzinom und kleinzelliges Lungenkarzinom. Zu weiteren Beispielen zählen Prostata-, Bauchspeicheldrüsen- und Brustkarzinom.
Neben der Funktion beim Zellwachstum spielt S1P auch eine Rolle bei der Neubildung von Blutgefäßen (Angiogenese). Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculares Glaukom. Die dieser Erfindung zugrunde liegenden Verbindungen der Formel I, die SphK1 hemmen und damit den S1 P-Spiegel regulieren und/oder modulieren, Zusammensetzungen, die diese Verbindungen enthalten, sowie die beschriebenen Verfahren können somit zur Behandlung dieser Krankheiten eingesetzt werden.
Des Weiteren beeinflussen SphK1 und S1 P die Proliferation, Differenzierung, Migration und Sekretion von Immunzellen (Rosen und Goetzl 2005 Nat Rev Immunol Vol 5 Seite 560) und sind somit an verschiedenen Funktionen des Immunsystems und an Entzündungsprozessen beteiligt. Stimulierung des Immunsystems steigert die Bildung und den Ausstoß von S1P in Mastzellen, Blutplättchenzellen und einigen mononukleären Phagozyten (Stunff et al. 2004 J Cell Biochem Vol 92 Seite 882; Olivera und Rivera 2005 j Immunol Vol 174 Seite 1153). Die Aktivität von SphK1 wird insbesondere durch Faktoren wie Tumour-Necrosis-Factor (TNF) und Vernetzung von IgG-Rezeptoren stark erhöht (Stunff et al. 2004 J Cell Biochem Vol 92 Seite 882; Delon et al. 2004 J Biol Chem Vol 279 Seite 44763). Es konnte außerdem gezeigt werden, dass SphK1 und S1P für die TNF-abhängige Bildung proinflammatorischer Enzyme wie Cyclooxygenase-2 (COX-2) und Nitric Oxide Synthase (NOS) wichtig sind (Pettus et al. 2003 FASEB J Vol 17 Seite 1411; Kwon et al.2001 J Biol Chem Vol 276 Seite 10627-33). Die dieser Erfindung zugrunde liegenden Verbindungen der Formel I, die SphK1 hemmen und damit den S1P-Spiegel regulieren und/oder modulieren, Zusammensetzungen, die diese Verbindungen enthalten, sowie die beschriebenen Verfahren können somit zur Behandlung von entzündungsbedingten Krankheiten wie Arthrose, Arteriosklerose, Psoriasis, Multiple Sklerose, chronisch-entzündlichen Darmerkrankungen (Morbus Crohn, Colitis ulcerosa) Asthma und andere allergische Erkrankungen eingesetzt werden.

Weiterhin können die Verbindungen der Formel I zur Isolierung und zur Untersuchung der Aktivität oder Expression von Sph-Kinase verwendet werden. Außerdem eigenen sie sich insbesondere zur Verwendung in diagnostischen Verfahren zu Erkrankungen im Zusammenhang mit unregulierter oder gestörter Sph-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-Gonzälez, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELlSA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

In der WO 2007/064553 A2 sind andere Thiazolderivate als CXCR3 Rezeptormodulatoren beschrieben.
In der WO 2007/020213 A2 sind andere Thiazol-piperidin-derivate als H3 Rezeptormodulatoren beschrieben.
In der WO 2007/019251 sind andere Thiazolderivate als Sphingosinkinase-Inhibitoren beschrieben. Dort ist die Verwendung der offenbarten Thiazolderivate zur Behandlung von hyperproliferativen, inflammatorischen und angiogenen Erkrankungen beschrieben.

### ZUSAMMENFASSUNG DER ERFINDUNG

Die Erfindung betrifft die Verwendung von Verbindungen der Formel I 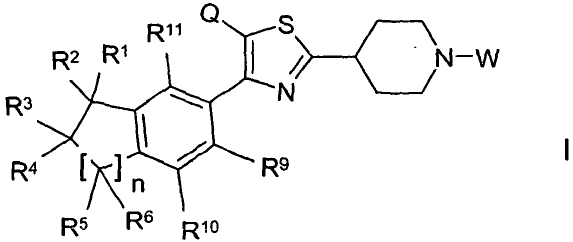 worin
- R¹, R², R³, R⁴, R⁵, R⁶: jeweils unabhängig voneinander H oder A',
- Q: H, A, Hal, COOR⁷ oder CON(R⁷R^{7'}),
- W: [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, SO₂-[C(R⁸R^{8'})]ₚZ, CO[C(R⁸R^{8'})]ₚNHCOOA oder [C(R⁸R^{8'})_{]}pNHCOOA,
- R⁷, R^{7'}: jeweils unabhängig voneinander H oder A',
- R⁸, R^{8'}: jeweils unabhängig voneinander H, A, OA, NAA' oder Het,
- R⁹: H oder A',
- R¹⁰, R¹¹: jeweils unabhängig voneinander H, A' oder OH,
- Z: Het, Ar oder A,
- A: unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, S, SO, SO₂, CO, COO, NR⁷, NR⁷CO, CONR⁷, CH=CH und/oder CH≡CH-Gruppen ersetzt sein können
oder
cyclisches Alkyl mit 3-7 C-Atomen,
- Ar: unsubstituiertes oder ein-, zwei-, drei-, vier- oder fünffach durch Hal, A, [C(R⁷R^{7'})]ₚOR⁷, [C(R⁷R^{7'})]ₚN(R⁷)₂, SR⁷, NO₂, CN, CHO, COOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R^{7'}), NHCOO[C(R⁷R^{7'})]ₚN(R⁷)₂, NHCOO[C(R⁷R⁷)]ₚHet, NHCONH[C(R⁷R^{7'})]ₚN(R⁷)₂, NHCONH[C(R⁷R^{7'})]ₚHet, OCONH[C(R⁷R^{7'})]ₚN(R⁷)₂, OCONH[C(R⁷R^{7'})]ₚHet, CONR⁷[C(R⁷R^{7'})]ₚN(R⁷)₂, CONR⁷[C(R⁷R^{7'})]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet und/oder S(O)ₘ[C(R⁷R^{7'})]ₚAr' substituiertes Phenyl, Naphthyl oder Biphenyl,
- Ar': unsubstituiertes oder ein-, zwei-, drei-, vier- oder fünffach durch Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA und/oder COA substituiertes Phenyl,
- A': unverzweigtes oder verzweigtes Alkyl mit 1-8 C-Atomen, worin 1-7 H-Atome durch F, Cl und/oder Br ersetzt sein können,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR⁷, [C(R⁷R^{7'})]ₚAr, [C(R⁷R^{7'})]ₚHet', COA, CO[C(R⁷R^{7'})]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R^{7'}), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR⁷SO₂A, NR⁷SO₂Ar, NR⁷SO₂Het', SO₂N(R⁷)A, SO₂N(R⁷)Ar, SO₂N(R⁷)Het', CHO, =S, =NH, =NA, Oxy (-O⁻) und/oder =O (Carbonylsauerstoff) substituiert sein kann,
- Het': einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
- m: 0, 1 oder 2,
- n: 1, 2 oder 3,
- p: 0, 1, 2, 3 oder 4,
sowie ihrer pharmazeutisch verwendbaren Salze, Tautomeren und Stereoisomeren, einschließlich deren Mischungen in allen Verhältnissen,
zur Herstellung eines Arzneimittels zur Behandlung von Krankheiten,
wobei die zu behandelnden Krankheiten ausgewählt sind aus der Gruppe hyperproliferative Erkrankung, inflammatorische Erkrankung, angiogene Erkrankung, fibrotische Erkrankung der Lunge, Niere, Leber und des Herzens.

Die Erfindung betrifft weiterhin Verbindungen der Formel I 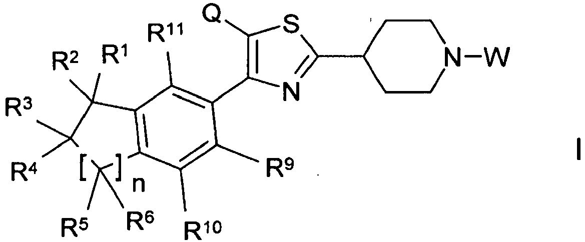 worin
- R¹, R², R³, R⁴, R⁵, R⁶: jeweils unabhängig voneinander H oder A',
- Q: H, A, Hal, COOR⁷ oder CON(R⁷R^{7'}),
- W: [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, SO₂-[C(R⁸R^{8'})]ₚZ, CO[C(R⁸R^{8'})]ₚNHCOOA oder [C(R⁸R^{8'})]ₚNHCOOA,
- R⁷, R^{7'}: jeweils unabhängig voneinander H oder A',
- R⁸, R^{8'}: jeweils unabhängig voneinander H, A, OA, NAA' oder Het,
- R⁹: H oder A',
- R¹⁰, R¹¹: jeweils unabhängig voneinander H, A' oder OH,
- Z: Het, Ar oder A,
- A: unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, S, SO, SO₂, CO, COO, NR⁷, NR⁷CO, CONR⁷, CH=CH und/oder CH≡CH-Gruppen ersetzt sein können
oder
cyclisches Alkyl mit 3-7 C-Atomen,
- Ar: unsubstituiertes oder ein-, zwei-, drei-, vier- oder fünffach durch Hal, A, [C(R⁷R^{7'})]ₚOR⁷, [C(R⁷R^{7'})]ₚN(R⁷)₂, SR⁷, NO₂, CN, CHO, COLOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R^{7'}), NHCOO[C(R⁷R^{7'})]ₚN(R⁷)2, NHCOO[C(R⁷R⁷)]ₚHet, NHCONH[C(R⁷R^{7'})]ₚ^{N}(R⁷)₂, NHCONH[C(R⁷R^{7'})]ₚHet, OCONH[C(R⁷R^{7'})]ₚN(R⁷)₂, OCONH[C(R⁷R^{7'})]ₚHet, CONR⁷[C(R⁷R^{7'})]ₚN(R⁷)2, CONR⁷[C(R⁷R^{7'})]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet und/oder S(O)ₘ[C(R⁷R^{7'})]ₚAr' substituiertes Phenyl, Naphthyl oder Biphenyl,
- Ar': unsubstituiertes oder ein-, zwei-, drei-, vier- oder fünffach durch Hal, A, OH, OA', NH₂, NHA, NAA', CN, CHO, COOR⁷, CON(R⁷R^{7'}) , NR⁷COA, NR⁷SO₂A, SO₂N(R⁷R^{7'}), S(O)ₘA und/oder COA substituiertes Phenyl,
- A': unverzweigtes oder verzweigtes Alkyl mit 1-8 C-Atomen, worin 1-7 H-Atome durch F, Cl und/oder Br ersetzt sein können,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR⁷, [C(R⁷R^{7'})]ₚAr, [C(R⁷R^{7'})]ₚHet', COA, CO[C(R⁷R^{7'})]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R^{7'}), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR'SO₂A, NR⁷SO₂Ar, NR⁷SO₂Het', SO₂N(R⁷)A, SO₂N(R⁷)Ar, SO₂N(R⁷)Het', CHO, =S, =NH, =NA, Oxy (-O⁻) und/oder =O (Carbonylsauerstoff) substituiert sein kann,
- Het': einen einkernigen gesättigten Heterocyclus mit 1 bis 2 N und/oder O-atomen, der ein- oder zweifach durch A, OA, OH, Hal und/oder =O (Carbonylsauerstoff) substituiert sein kann,
- m: 0, 1 oder 2,
- n: 1, 2 oder 3,
- p: 0, 1, 2, 3 oder 4,
bedeuten,
sowie ihre pharmazeutisch verwendbaren Salze, Tautomere und Stereoisomere, einschließlich deren Mischungen in allen Verhältnissen;
wobei Verbindungen der Formel I ausgeschlossen sind, worin
a) W = COCH₂Het bedeutet;
b) n = 2 und W = unsubstituiertes Alkyl mit 1-10 C-Atomen oder Cycloalkyl mit 3-7 C-Atomen bedeuten.

Gegenstand der Erfindung sind auch die Vorstufen der Verbindungen der Formel I (Verbindungen der Serie "C"), Arzneimittel, die diese Verbindungen enthalten, sowie deren Verwendung zu Behandlung von Krankheiten, wie für die Verbindungen der Formel I beschrieben.

Unter Verbindungen der Formel I versteht man auch die Hydrate und Solvate dieser Verbindungen, ferner pharmazeutisch verwendbare Derivate. Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch verwendbaren Derivaten versteht man z.B. sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1,1:2,1:3,1:4,1:5,1:10,1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man
a) eine Verbindung der Formel II 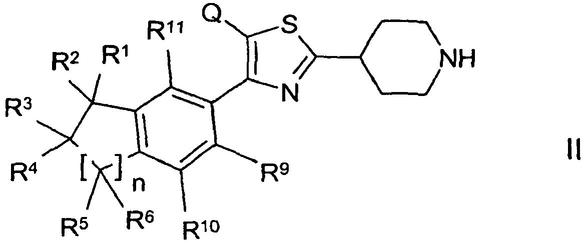 worin R¹, R², R³, R⁴, R⁵, R⁶, R⁹, R¹⁰, R¹¹, Q und n die in Anspruch 1 angegebenen Bedeutungen haben,
   mit einer Verbindung der Formel III

   L-W III,

   worin W die in Anspruch 1 angegebene Bedeutung hat und
   L Cl, Br, I oder eine freie oder reaktionsfähig funktionell abgewandelte OH-Gruppe bedeutet,
   umsetzt,
   oder
b) zur Herstellung von Verbindungen der Formel I, worin
   W [C(R⁸R^{8'})]ₚZ oder [C(R⁸R^{8'})]ₚNHCOOA,
   R⁸ H,
   p 1, 2, 3 oder 4
   bedeuten,
   R^{8'}, Z, A die in Anspruch 1 angegebenen Bedeutungen haben, eine Verbindung der Formel II
   i) mit einer Verbindung der Formel IVa

      R^{8'}-CO-[C(R⁸R^{8'})]ₚ₋₁Z IVa,

      worin
      p 1, 2, 3 oder 4 bedeutet und
      R^{8'}, Z die in Anspruch 1 angegebenen Bedeutungen haben,
      in einer reduktiven Aminierung umsetzt,
      oder
   ii) mit einer Verbindung der Formel IVb

      R^{8'}-CO-[C(R⁸R^{8'})]ₚ₋₁NHCOOA IVb,

      worin
      p 1, 2, 3 oder 4 bedeutet und
      R⁸, A die in Anspruch 1 angegebenen Bedeutungen haben,
      in einer reduktiven Aminierung umsetzt,
   oder
b) daß man sie aus einem ihrer funktionellen Derivate durch Behandeln mit einem solvolysierenden oder hydrogenolysierenden Mittel in Freiheit setzt,
und/oder
eine Base oder Säure der Formel I in eines ihrer Salze umwandelt.

Vor- und nachstehend haben die Reste R¹, R², R³, R⁴, R⁵, R⁶, R⁹, R¹⁰, R¹¹, Q, W und n die bei der Formel I angegebenen Bedeutungen, sofern nicht ausdrücklich etwas anderes angegeben ist.

Der Ausdruck "Carbamoyl" bedeutet "Aminocarbonyl" und umgekehrt.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
A bedeutet weiterhin vorzugsweise unverzweigtes oder verzweigtes Alkyl mit 1-10 C-Atomen, worin 1-7 H-Atome durch F, Cl, Br, OH und/oder OCH₃ ersetzt sein können, und/oder worin eine oder zwei nicht-benachbarte CH₂-Gruppen durch O, NR⁷, SO₂, NR⁷CO und/oder CONR⁷-Gruppen ersetzt sein können.
A bedeutet daher z.B. auch Dimethylaminoethyl, Aminocarbonylmethyl, Hydroxyethyl, 2,3-Dihydroxypropyl, 3-Hydroxypropyl, 3-Methoxy-2-hydroxypropyl, N,N-Diethylaminocarbonylmethyl oder 2-Methansulfonyl-1-methyl-ethyl.
Cyclisches Alkyl (Cycloalkyl) bedeutet vorzugsweise Cyclopropyl, Cyclobutyl, Cylopentyl, Cyclohexyl oder Cycloheptyl.
A' bedeutet vorzugsweise Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

R¹, R² bedeuten vorzugsweise, jeweils unabhängig voneinander H oder A'. R³, R⁴ bedeuten vorzugsweise H.
R⁵, R⁶ bedeuten vorzugsweise, jeweils unabhängig voneinander H oder A'. Q bedeutet vorzugsweise H.
W bedeutet vorzugsweise [C(R⁸R^{8'})]ₚZ, CO-[C(R⁸R^{8'})]ₚZ,
CO-N(R⁸)-[C(R⁸R⁸)]ₚZ, CO-O-[C(R⁸R^{8'})]ₚZ, CO[C(R⁸R^{8'})]ₚNHCOOA oder [C(R⁸R^{8'})]ₚNHCOOA.

R⁷, R^{7'} bedeuten vorzugsweise, jeweils unabhängig voneinander H oder A'. R⁸, R^{8'} bedeuten vorzugsweise, jeweils unabhängig voneinander H, A oder Het.
R⁹ bedeutet vorzugsweise H oder A', besonders bevorzugt H.
R¹⁰, R¹¹ bedeuten vorzugsweise, jeweils unabhängig voneinander H, A' oder OH, besonders bevorzugt H.
Z bedeutet vorzugsweise Het oder A.
Z bedeutet besonders bevorzugt Morpholinyl, Ethyl-piperidinyl, Methylpyrrolidinyl, Piperidinyl, Pyrrolidinyl, 2,4-Dioxo-tetrahydrochinazolinyl, 2-Oxooxazolidinyl, Methyl, Ethyl, 4-(Methoxyphenyl)-piperazinyl, Pyridyl, 4-(tert.-Butyloxycarbonyl)-piperazinyl, 4-(tert.-Butyloxycarbonyl)-morpholinyl, Piperazinyl, Hydroxy-pyrrolidinyl, N-tert.-Butyloxycarbonyl-hydroxy-pyrrolidinyl, Dimethylaminoethyl, Aminocarbonylmethyl, Hydroxyethyl, 2,3-Dihydroxypropyl, 3-Hydroxypropyl, 3-Methoxy-2-hydroxypropyl, N,N-Diethylaminocarbonylmethyl oder 2-Methansulfonyl-1-methyl-ethyl, CH(NH₂)CH₂CONH₂ oder CH(NH₂)CH₂OH.

Ar bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methytsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, o-, m- oder p-(Morpholin-4-ylcarbonyl)-phenyl, o-, m- oder p-(3-Oxo-morpholin-4-yl)-phenyl, o-, m- oder p-(Piperidinyl-carbonyl)-phenyl, o-, m- oder p-[2-(Morpholin-4-yl)ethoxyl-phenyl, o-, m- oder p-[3-(N,N-Diethylamino)propoxy]-phenyl, o-, m- oder p-[3-(3-Diethylaminopropyl)-ureido]-phenyl, o-, m- oder p-(3-Diethylaminopropoxy-carbonylamino)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibrom-phenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6-oder 3,4,5-Trichlorphenyl, 2,4,6-Trimethoxyphenyl, 2-Hydroxy-3,5-dichlor-phenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.
Ar bedeutet ganz besonders bevorzugt unsubstituiertes oder ein-, zwei-, drei-, vier- oder fünffach durch Hat und/oder [C(R⁷R^{7'})]ₚOR⁷ substituiertes Phenyl.
Ar' bedeutet vorzugsweise Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Aminophenyl, o-, m- oder p-(N-Methylamino)-phenyl, o-, m- oder p-(N-Methylamino-carbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, der p-(Methylsulfonamido)-phenyl, o-, m-oder p-(Methylsulfonyl)-phenyl, o-, m- oder p-Methylsulfanylphenyl, o-, m-oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino-oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-lsothiazolyl, 2-, 3- oder 4-Pyridyl, 2-, 4-, 5- oder 6-Pyrimidinyl, weiterhin bevorzugt 1,2,3- Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzo-thiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-y! oder Dibenzofuranyl. Die heterocyclischen Reste können auch teilweise oder vollständig hydriert sein.
Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5-oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, - 6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)-phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet vorzugsweise einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann
Het bedeutet besonders bevorzugt unsubstituiertes oder ein-, zwei- oder dreifach durch A, OR⁷, [C(R⁷R^{7'})ₚAr, COOR⁷ und/oder =O (Carbonylsauerstoff) substituiertes Pyrrolidinyl, Piperidinyl, Morpholinyl, Oxazolidinyl, Tetrahydrochinazolinyl, Piperazinyl, Thiazolyl, Furyl, Thienyl, Pyrrolyl, Imidazolyl, Pyrazolyl, Oxazolyl, Isoxazolyl, Isothiazolyl, Pyridyl, Pyrimidinyl, Triazolyl, Tetrazolyl, Oxadiazolyl oder Thiadiazolyl.
Het bedeutet vorzugsweise einen einkernigen gesättigten Heterocyclus mit 1 bis 2 N und/oder O-Atomen, der ein- oder zweifach durch A substituiert sein kann; ganz besonders bevorzugt Piperidinyl, Pyrrolidinyl, Morpholinyl oder Piperazinyl, die ein- oder zweifach durch A substituiert sein können.

Hal bedeutet vorzugsweise F, Cl oder Br, aber auch I, besonders bevorzugt F oder Cl.
n bedeutet vorzugsweise 1 oder 2.

Für die gesamte Erfindung gilt, daß sämtliche Reste, die mehrfach auftreten, gleich oder verschieden sein können, d.h. unabhängig voneinander sind.
Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I und deren Verwendung, in denen mindestens einer der genannten Reste eine der vorstehend angegebenen bevorzugten Bedeutungen hat. Einige bevorzugte Gruppen von Verbindungen können durch die folgenden Teilformeln la bis Ij ausgedrückt werden, die der Formel I entsprechen und worin die nicht näher bezeichneten Reste die bei der Formel I angegebene Bedeutung haben, worin jedoch
- in la: R¹, R² jeweils unabhängig voneinander H oder A',
R³, R⁴ H,
R⁵, R⁶ jeweils unabhängig voneinander H oder A', bedeuten;
- in Ib: Q H bedeutet;
- in Ic: W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R⁸)]ₚNHCOOA oder [C(R⁸R^{8'})]ₚNHCOOA, bedeutet;
- in Id: R⁷, R^{7'} jeweils unabhängig voneinander H oder A',
R⁸, R^{8'} H, A oder Het, bedeuten;
- in le: Z Het oder A bedeutet;
- in If: A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, NR⁷, SO₂, NR⁷CO und/oder CONR⁷-Gruppen ersetzt sein können" bedeutet;
- in Ig: Ar unsubstituiertes oder ein-, zwei-, drei-, vier- oder fünffach durch Hai und/oder [C(R⁷R^{7'})]ₚOR⁷ substituiertes Phenyl, bedeutet;
- in Ih: Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann, bedeutet;
- in li: Het unsubstituiertes oder ein-, zwei- oder dreifach durch A, OR⁷, [C(R⁷R^{7'})]ₚAr, COOR⁷ und/oder =O (Carbonylsauerstoff) substituiertes Pyrrolidinyl, Piperidinyl, Morpholinyl, Oxazolidinyl, Tetrahydrochinazolinyl, Piperazinyl, Thiazolyl, Furyl, Thienyl, Pyrrolyl, Imidazolyl, Pyrazolyl, Oxazolyl, Isoxazolyl, Isothiazolyl, Pyridyl, Pyrimidinyl, Triazolyl, Tetrazolyl, Oxadiazolyl oder Thiadiazolyl, bedeutet;
- in Ij: R¹, R² jeweils unabhängig voneinander H oder A',
R³, R⁴ H,
R⁵, R⁶ jeweils unabhängig voneinander H oder A',
R⁷, R^{7'} jeweils unabhängig voneinander H oder A',
R⁸, R^{8'} jeweils unabhängig voneinander H, A oder Het,
R⁹ H oder A',
R¹⁰, R¹¹ jeweils unabhängig voneinander H, A' oder OH,
Q H,
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA oder [C(R⁸R^{8'})]ₚNHCOOA,
Z Het oder A,
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, NR⁷, SO₂, NR⁷CO und/oder CONR⁷-Gruppen ersetzt sein können,
A' unverzweigtes oder verzweigtes Alkyl mit 1-8 C-Atomen, worin 1-7 H-Atome durch F, Cl und/oder Br ersetzt sein können,
Ar unsubstituiertes oder ein-, zwei-, drei-, vier- oder fünffach durch Hal und/oder [C(R⁷R^{7'})]ₚOR⁷ substituiertes Phenyl,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann,
n 1 oder 2,
p 0, 1, 2, 3 oder 4, bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II und III sind in der Regel bekannt. Sind sie neu, so können sie aber nach an sich bekannten Methoden hergestellt werden.

Verbindungen der Formel können vorzugsweise erhalten werden, indem man eine Verbindung der Formel II mit einer Verbindung der Formel III umsetzt.
In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Acetonitril, Dichlormethan und/oder DMF.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Umsetzung einer Verbindung der Formel II mit einer Verbindung der Formel IVa oder IVb erfolgt unter Bedingungen einer reduktiven Aminierung wie sie dem Fachmann bekannt sind und in Standardwerken der Organischen Chemie beschrieben sind.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanotamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intra-dermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von sphingosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Prostatakrebs, Darmkrebs, Bauchspeicheldrüsenkrebs, Ovarialkarzinom, Nierenkrebs, Leberkarzinom, Glioblastome und Brustkarzinom.
Ebenfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allzu großen Aufwand bestimmt werden.
Die vorliegende Erfindung umfasst auch die Verwendung von Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Neben den Verbindungen der Formel I können auch deren Vorstufen (Verbindungen der Serie "C") zur Behandlung der genannten Erkrankungeen verwendet werden.

Die Verbindungen der Formel I können an Patienten zur Behandlung von Krebs, insbesondere schnell wachsenden Tumoren, verabreicht werden.

Gegenstand der Erfindung ist somit die Verwendung von Verbindungen der Formel I, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Sph-Kinase.

Bevorzugt ist die Verwendung von Verbindungen der Formel I, sowie ihrer pharmazeutisch verwendbaren Derivate, Solvate und Stereoisomere, einschließlich deren Mischungen in allen Verhältnissen,
zur Herstellung eines Arzneimittels zur Behandlung von Krankheiten, die durch Inhibierung der SphK 1 durch die Verbindungen nach Anspruch 1 beeinflußt werden.

Die zu behandelnden Krankheiten sind vorzugsweise ausgewählt aus der Gruppe
hyperproliferative Erkrankung, inflammatorische Erkrankung, angiogene Erkrankung.

Die hyperproliferative Erkrankung ist vorzugsweise ausgewählt aus der Gruppe
Krebs (Tumorerkrankung), Atherosclerose, Restenose, proliferative Erkrankung der Mesangiumzellen, Psoriasis.

Die Tumorerkrankung ist vorzugsweise ausgewählt aus der Gruppe Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Die proliferative Erkrankung der Mesangiumzellen ist vorzugsweise ausgewählt aus der Gruppe
Glomerulonephritis, diabetische Nephropathie, maligne Nephrosclerose, thrombotisches Mikroangiopathie-Syndrom, Transplantatabstossung, Glomerulopathie.

Die inflammatorische Erkrankung ist vorzugsweise ausgewählt aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis,
durch T-Zellen - vermittelte Immunerkrankung.

Die entzündliche Darmerkrankung ist vorzugsweise ausgewählt aus der Gruppe
ulcerative Colitis, Morbus Crohn, unbestimmte Colitis.

Die durch T-Zellen - vermittelte Immunerkrankung ist vorzugsweise ausgewählt aus der Gruppe
allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt iaus der Gruppe rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe
Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe
Psoriasis, atopische Dermatitis, Kontaktempfindlichkeit, Akne.

Die angiogene Erkrankung ist vorzugsweise ausgewählt aus der Gruppe diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthe |
| | Tetraplatin | BBR-3464 (Hoffrnann-La Roche) |
| | Ormiplatin | |
| | Iproplatin | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche |
| | Idatrexate | Ethinylcytidin (Taiho ) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| | | |
| Antitumor-Antibiotik | Dactinomycin (Actinomycin | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubici | GPX-100 (Gem Pharmaceuticals) |
| | Mitoxantron (Novantron) | |
| | | |
| Antimitotische Mitte | Paclitaxel | SB 408075 (GlaxoSmithKlin |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881 A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharma) |
| | Vinflunin (Fabre) | |
| | Auristatin PE (Teikoku Hormone) | Azaepothilon B (BMS) |
| | | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransferas Inhibitoren | Arglabin (NuOncology Labs) | Tipifarnib (Johnson & Johnson) |
| | Ionafarnib (Schering-Plough | |
| | BAY-43-9006 (Bayer) | Perillylalkohol (DOR BioPharma) |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransferase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidredul se-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten / Antagonisten | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatorer | Interferon | Dexosom-Therapie ((Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa: | |
| | | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologie | |
| | Motexafin-Gadolinium (Pharmacyclics) | Lutetium-Texaphyrin (Pharmacyclics) |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| Verschiedene Mitte | SR-27897 (CCK-A-Inhibitor, Sanofi-Synthelabo) | BCX-1777 (PNP-Inhibitor, BioCryst) |
| | Tocladesin (cyclisches-AMP Agonist, Ribapharm) | Ranpirnase (Ribonuclease-Stimulans, Alfacell) |
| | Alvocidib (CDK-Inhibitor, Aventis) | Galarubicin (RNA-Synthese Inhibitor, Dong-A) |
| | CV-247 (COX-2-Inhibitor, Iv Medical) | Tirapazamin (Reduktionsmittel, SRI International) |
| | P54 (COX-2-Inhibitor, Phytopharm) | |
| | | N-Acetylcystein (Reduktionsmittel, Zambon) |
| | CapCell™ (CYP450-Stimulans, Bavarian Nordic) | |
| | | R-Flurbiprofen (NF-kappaB-Inhibitor, Encore) |
| | GCS-IOO (gal3-Antagonist, GlycoGenesys) | |
| | | 3CPA (NF-kappaB-Inhibitor. Active Biotech) |
| | G17DT-Immunogen (Gastrir Inhibitor, Aphton) | |
| | | Seocalcitol (Vitamin-D-Rezeptor-Agonist, Leo) |
| | Efaproxiral (Oxygenator, All Therapeutics) | |
| | | 131-I-TM-601 (DNA-Antagonist, TransMolecular) |
| | PI-88 (Heparanase-Inhibitor Progen) | |
| | | Eflornithin (ODC-Inhibitor, ILEX Oncology) |
| | Tesmilifen (Histamin-Antagonist, YM BioSciences | |
| | | Minodronsäure (Osteoclaste Inhibitor, Yamanouchi) |
| | Histamin (Histamin-H2-Rezeptor- Agonist, Maxim) | |
| | | Indisulam (p53-Stimulans, Eisai) |
| | Tiazofurin (IMPDH-Inhibitor, Ribapharm) | |
| | | Aplidin (PPT-Inhibitor, PharmaMar) |
| | Cilengitid (Integrin-Antagoni Merck KGaA) | |
| | | Rituximab (CD20-Antikörpe Genentech) |
| | SR-31747 (IL-1-Antagonist, Sanofi-Synthelabo) | |
| | | Gemtuzumab (CD33-Antikörper, Wyeth Ayerst) |
| | CCI-779 (mTOR-Kinase-Inhibitor, Wyeth) | |
| | | PG2 (Hämatopoese-Verstärker, Pharmagenesis) |
| | Exisulind (PDE-V-Inhibitor, Cell Pathways) | |
| | | Immunol™ (Triclosan-Oralspülung, Endo) |
| | CP-461 (PDE-V-Inhibitor, C Pathways) | |
| | | Triacetyluridin (Uridin-Prodr Wellstat) |
| | AG-2037 (GART-Inhibitor, Pfizer) | |
| | | SN-4071 (Sarkom-Mittel, Signature BioScience) |
| | WX-UK1 | |
| | (Plasminogenaktivator-Inhibitor, Wilex) | TransMID-107™ (Immunotoxin, KS Biomedix |
| | PBI-1402 (PMN-Stimulans, ProMetic LifeSciences) | PCK-3145 (Apoptose-Förderer, Procyon) |
| | Bortezomib (Proteasom-Inhibitor, Millennium) | Doranidazol (Apoptose-Förderer, Pola) |
| | SRL-172 (T-Zell-Stimulans, SR Pharma) | CHS-828 (cytotoxisches Mittel, Leo) |
| | TLK-286 (Glutathion-S-Transferase-Inhibitor, Telik) | trans-Retinsäure (Differentiator, NIH) |
| | PT-100 (Wachstumsfaktor-Agonist, Point Therapeutics | MX6 (Apoptose-Förderer, MAXIA) |
| | Midostaurin (PKC-Inhibitor, Novartis) | Apomin (Apoptose-Förderer ILEX Oncology) |
| | Bryostatin-1 (PKC-Stimulan: GPC Biotech) | Urocidin (Apoptose-Fördere Bioniche) |
| | CDA-II (Apoptose-Förderer, Everlife) | Ro-31-7453 (Apoptose-Förderer, La Roche) |
| | SDX-101 (Apoptose-Fördere Salmedix) | Brostallicin (Apoptose-Förderer, Pharmacia) |
| | Ceflatonin (Apoptose-Förderer, ChemGenex) | |
| | | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthe |
| | Tetraplatin | BBR-3464 (Hoffrnann-La Roche) |
| | Ormiplatin | |
| | Iproplatin | SM-11355 (Sumitomo) |
| | | AP-5280 (Access) |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche |
| | Idatrexate | Ethinylcytidin (Taiho ) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothecin | |
| | | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| | | |
| Antitumor-Antibiotik | Dactinomycin (Actinomycin | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubici | GPX-100 (Gem Pharmaceuticals) |
| | Mitoxantron (Novantron) | |
| Antimitotische Mitte | Paclitaxel | SB 408075 (GlaxoSmithKlin |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient |
| | Vinflunin (Fabre) | NeuroPharma) |
| | Auristatin PE (Teikoku Hormone) | Azaepothilon B (BMS) |
| | | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransferase Inhibitoren | Arglabin (NuOncology Labs) | Tipifarnib (Johnson & Johnson) |
| | lonafarnib (Schering-Plough | |
| | BAY-43-9006 (Bayer) | Perillylalkohol (DOR BioPharma) |
| Pumpen-Inhibitorer | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransfe se-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidredul se-Inhibitoren | Neovastat (Aeterna | CMT -3 (CollaGenex) |
| | Laboratories) | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten/Antagon en | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa | |
| Hormonelle und antihormonelle Mitt | *Östrogene* | *Prednison* |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologie | |
| | Motexafin-Gadolinium (Pharmacyclics) | Lutetium-Texaphyrin (Pharmacyclics) |
| | | Hypericin |
| | | |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| | | |
| Verschiedene Mitte | SR-27897 (CCK-A-Inhibitor Sanofi-Synthelabo) | BCX-1777 (PNP-Inhibitor, BioCryst) |
| | Tocladesin (cyclisches-AM Agonist, Ribapharm) | Ranpirnase (Ribonuclease-Stimulans, Alfacell) |
| | Alvocidib (CDK-Inhibitor, Aventis) | Galarubicin (RNA-Synthese-Inhibitor, Dong-A) |
| | CV-247 (COX-2-Inhibitor, | Tirapazamin (Reduktionsmitt |
| | Medical) | SRI International) |
| | P54 (COX-2-Inhibitor, Phytopharm) | N-Acetylcystein (Reduktionsmittel, Zambon) |
| | CapCell™ (CYP450-Stimulans, Bavarian Nordic | R-Flurbiprofen (NF-kappaBlnhibitor, Encore) |
| | GCS-IOO (gal3-Antagonist, GlycoGenesys) | 3CPA (NF-kappaB-Inhibitor, Active Biotech) |
| | G17DT-Immunogen (Gastri Inhibitor, Aphton) | Seocalcitol (Vitamin-D-Rezeptor-Agonist, Leo) |
| | Efaproxiral (Oxygenator, All Therapeutics) | 131-I-TM-601 (DNA-Antagonist, TransMolecular) |
| | PI-88 (Heparanase-Inhibito Progen) | Eflornithin (ODC-Inhibitor, IL Oncology) |
| | Tesmilifen (Histamin-Antagonist, YM BioScience | Minodronsäure (Osteoclaste Inhibitor, Yamanouchi) |
| | Histamin (Histamin-H2-Rezeptor- Agonist, Maxim) | Indisulam (p53-Stimulans, Eisai) |
| | Tiazofurin (IMPDH-Inhibitor Ribapharm) | Aplidin (PPT-Inhibitor, PharmaMar) |
| | Cilengitid (Integrin-Antagonist, Merck KGaA) | Rituximab (CD20-Antikörper. Genentech) |
| | SR-31747 (IL-1-Antagonist, Sanofi-Synthelabo) | Gemtuzumab (CD33-Antikörper, Wyeth Ayerst) |
| | CCI-779 (mTOR-Kinase-Inhibitor, Wyeth) | PG2 (Hämatopoese-Verstärker, Pharmagenesis) |
| | Exisulind (PDE-V-Inhibitor, Cell Pathways) | Immunol™ (Triclosan-Oralspülung, Endo) |
| | CP-461 (PDE-V-Inhibitor, C Pathways) | Triacetyluridin (Uridin-Prodru Wellstat) |
| | AG-2037 (GART-Inhibitor, Pfizer) | SN-4071 (Sarkom-Mittel, Signature BioScience) |
| | WX-UK1 (Plasminogenaktivator-Inhibitor, Wilex) | TransMID-107™ (Immunotoxin, KS Biomedix) |
| | | PCK-3145 (Apoptose-Förder Procyon) |
| | PBI-1402 (PMN-Stimulans, ProMetic LifeSciences) | |
| | | Doranidazol (Apoptose-Förderer, Pola) |
| | Bortezomib (Proteasom-Inhibitor, Millennium) | |
| | | CHS-828 (cytotoxisches Mitt Leo) |
| | SRL-172 (T-Zell-Stimulans, SR Pharma) | |
| | | trans-Retinsäure (Differentiator, NIH) |
| | TLK-286 (Glutathion-S-Transferase-Inhibitor, Telik) | |
| | | MX6 (Apoptose-Förderer, MAXIA) |
| | PT-100 (Wachstumsfaktor-Agonist, Point Therapeutics | |
| | | Apomin (Apoptose-Förderer, ILEX Oncology) |
| | Midostaurin (PKC-Inhibitor, Novartis) | |
| | | Urocidin (Apoptose-Förderer Bioniche) |
| | Bryostatin-1 (PKC-Stimulan | |
| | GPC Biotech) | Ro-31-7453 (Apoptose-Förderer, La Roche) |
| | CDA-II (Apoptose-Förderer Everlife) | |
| | | Brostallicin (Apoptose-Förderer, Pharmacia) |
| | SDX-101 (Apoptose-Förderer, Salmedix) | |
| | Ceflatonin (Apoptose-Förderer, ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I können in den unten beschriebenen Assays auf eine kinasehemmende Wirkung geprüft werden. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Tests für die Inhibierung der SphK1-Aktivität

### Testbeschreibung

### Biochemischer Assay

Der Kinaseassay wird als 384-well Flashplate assay durchgeführt. 5 nM modifizierte SphK1, 800 nM omega-biotinyl-D-erythro-Sphingosine und 1 µM ATP (mit 0.3µCi ³³P-ATP/well) werden in einem Gesamtvolumen von 50µl ( 25 mM HEPES, 5 mM MgCl₂, 1mM Dithiothreitol, 0.01 % Brij35, 0.1 % BSA (fettsäurefrei), pH 7.4) ohne oder mit Prüfsubstanz (5-10 Konzentrationen) für 120 Min bei 30°C inkubiert. Die Reaktion wird mit 25 µl 200 mM EDTA-Lösung gestoppt, nach 30 Min bei Raumtemperatur abgesaugt und die Kavitäten 3mal mit 100 µl 0.9%-iger NaCl-Lösung gewaschen. Der unspezifische Anteil der Kinasereaktion (Blank) wird mit 0,5 mM NaCl bestimmt. Radioaktivität wird im Topcount gemessen. IC₅₀-Werte werden mit RS1 berechnet.

Neben der Überprüfung der Aktivität der Substanz auf das gereinigte SphK1-Enzym ist es im nächsten Schritt erforderlich zu untersuchen, ob die Substanzen SphK1 auch in seiner physiologischen Umgebung, d.h. im Cytoplasma der Zelle, hemmen.
Zu diesem Zweck wird die Bildung von S1 P in U2OS Osteosarkomazellen, die durch Einführung von modifizierter SphK1-cDNA das Enzym überproduzierten, mit zwei verschiedenen Methoden gemessen:
1. Die Zellen werden für 1 Stunde mit Substanzen und anschließend 15 min mit tritiummarkiertem Sphingosin inkubiert. Das radioaktiv markierte Sphingosin wird in dieser Zeit von den Zellen aufgenommen und von SphK1 zu S1 P umgesetzt. Die Zellen werden dann gewaschen und mit Ammoniaklösung lysiert. Um S1P von nicht umgesetzten Sphingosin zu trennen, erfolgt eine Extraktion durch Zugabe eines Chloroform-Methanol-Gemischs. Während Sphingosin zum größten Teil in die organische Phase übergeht, reichert sich S1 P in der wässrigen Phase an und wird mit Hilfe eines Szintillationszählers quantifiziert.
2. Die Zellen werden für 1 Stunde mit Substanzen und anschließend 15 min mit Sphingosin inkubiert. Das Sphingosin wird in dieser Zeit von den Zellen aufgenommen und von SphK1 zu S 1 P umgesetzt. Die Zellen werden dann gewaschen und mit Methanol lysiert. Die Methanollösung wird nun eingedampft und das S1P in lipidfreiem Serum aufgenommen. Die Quantifizierung des S1 P erfolgt unter Verwendung eines S1P-spezifischen Antikörpers mit Hilfe eines kompetitiven ELISA-Assays. Der mit Biotin verknüpfte S1P-Antikörper wird mit der Probenlösung inkubiert und dieses Gemisch wird in ein Well übertragen, dessen Boden mit S1 P beschichtet ist. Nur die Antikörper, die noch kein S1P aus der Probenlösung gebunden haben, binden an das auf der Platte immobilisierte S1P und können nach einem Waschschritt durch Zugabe von Meerrettich-Peroxidase gekoppeltem Streptavidin quantifiziert werden. Dazu wird das Substrat TMB zugefügt, das nach Umsetzung durch die Peroxidase bei einer Wellenlänge von 450 nm absorbiert und gemessen werden kann. Ein hohes Signal entspricht folglich einer niedrigen S1P-Konzentration in der Probenlösung und ein niedriges Signal entsprechend einer hohen S1P-Konzentration.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | El (Elektronenstoß-Ionisation) M⁺ |
| | FAB (Fast Atom Bombardment) (M+H)⁺ |
| | ESI (Electrospray lonization) (M+H)⁺ |

APCI-MS (atmospheric pressure chemical ionization - mass spectrometry) (M+H)⁺.

### HPLC-Methode:

Gradient: 4.2 min
Fluss: 2 ml/min 99:01 - 0:100 Wasser + 0.1%(Vol.) TFA : Acetonitril + 0.1%(Vol.) TFA
0.0 bis 0.2 min: 99:01
0.2 bis 3.8 min: 99:01---> 0:100
3.8 bis 4.2 min: 0:100
Säule: Chromolith Performance RP18e; 100 mm lang, Innendurchmesser 3 mm Wellenlänge: 220nm
Retentionszeit Rt. in Minuten [min].

### Methode B

Gradient: 4.2 min
Fluss: 2 ml/min 99:01 - 0:100 Wasser + 0.1%(Vol.) Ameisensäure: Acetonitril + 0.1 %(Vol.) Ameisensäure
0.0 bis 0.2 min: 99:01
0.2 bis 3.8 min: 99:01---> 0:100
3.8 bis 4.2 min: 0:100
Säule: Chromolith Performance RP18e; 100 mm lang, Innendurchmesser 3 mm Wellenlänge: 220 nm

### Beispiele zur Herstellung von Edukten

### Herstellung der Bromcarbonylverbindungen:

### 2-Brom-1-(5,6,7,8-tetrahydro-naphthalin-2-yl)-ethanon:

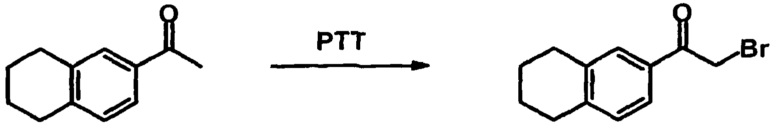

25 g (143 mmol) 1-(5,6,7,8-Tetrahydro-naphthalin-2-yl)-ethanon werden in 750 ml THF gelöst, mit 64.7 g (172 mmol) Phenyltrimethylammoniumtribromid versetzt und 15 h bei Raumtemperatur gerührt. Der entstandene Niederschlag wird filtriert und das Filtrat zum Rückstand eingedampft. Der Rückstand wird in Ethylacetat aufgenommen, 2 x mit gesättigter Natriumhydrogencarbonatlösung und 1 x mit gesättigter Natriumchloridlösung gewaschen, die organische Phase wird über Natriumsulfat getrocknet und zum Rückstand eingedampft.
Ausbeute: 62 g, weißer Feststoff; HPLC: Rt.= 3.06 min.

Analog der oben genannten Vorschrift werden folgende Verbindungen hergestellt. In einigen Fällen ist eine Aufreinigung mittels Säulenchromatographie an Kieselgel erforderlich:

| Edukt | Produkt | Rt. In min |
|---|---|---|
| 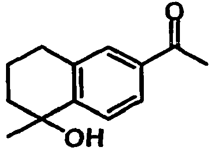 | 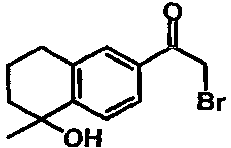 | |
| Synthetic Communications 20 877-892 | | |
| 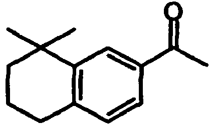 | 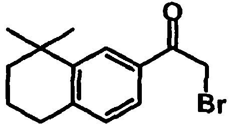 | 3.42 |
| Journal of Organic Chemistry 1984, 51(26), 5265-5267 | | |
| 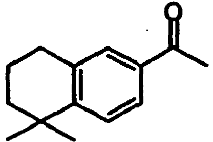 | 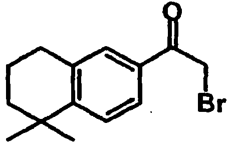 | 3.41 |
| US 2005/0148590 | | |
| 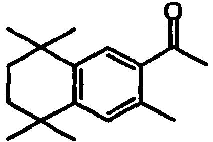 | 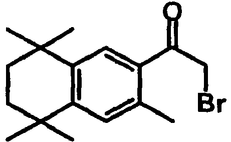 | |
| US 2005/0148590 | | |
| 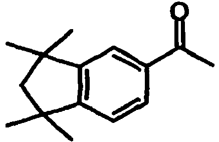 | 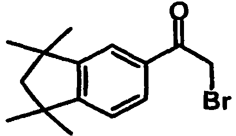 | 3.59 |
| US 2005/0148590 | | |
| 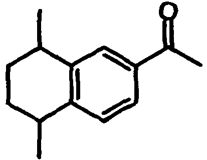 | 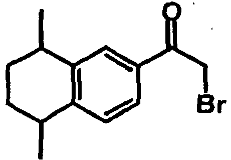 | 3.53 |
| Journal of Organic Chemistry 1963, 29(9), 2248-2255 | | |
| 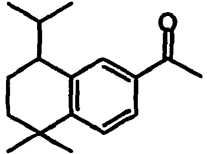 | 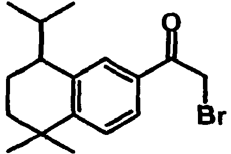 | |
| Journal of Organic Chemistry 1963, 29(9), 2248-2255 | | |
| 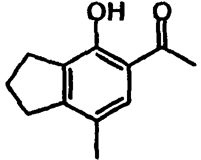 | 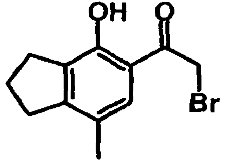 | |
| 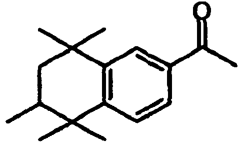 | 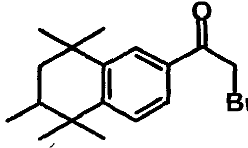 | 3.99 |
| 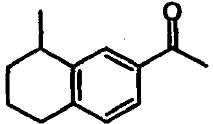 | 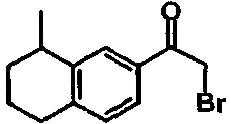 | 3.40 (Meth. B) |

### Herstellung der 2-(Piperidin-4-yl)thiazolverbindungen:

### 4-[4-(5,5,8,8-Tetramethyl-5,6,7,8-tetrahydro-naphthalin-2-yl)-thiazol-2-yl]-piperidin Hydrobromid ("C1 "):

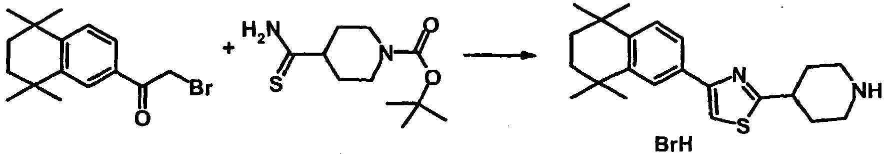

14.2 g (31.2 mmol) 2-Brom-1-(5,5,8,8-tetramethyl-5,6,7,8-tetrahydronaphthalin-2-yl)-ethanon und 7.6 g (31.2 mmol) 4-Thiocarbamoyl-piperidin-1-carbonsäure-tert.-butylester werden in 210 ml Ethanol suspendiert und 15 h refluxiert. Das Reaktionsgemisch wird auf Raumtemperatur abgekühlt und eingedampft. Der ölige Rückstand wird mit 50 ml Acetonitril verrührt, der Feststoff wird abgesaugt und mit Acetonitril und Diethylether gewaschen. Der Rückstand wird im Vakuum getrocknet.
Ausbeute: 10.3 g, weißer Feststoff. Das Produkt liegt als Hydrobromid vor; ESI: 355 (M+H); HPLC: Rt.=2.93 min.

### 4-[4-(5,5,8,8-Tetramethyl-5,6,7,8-tetrahydro-naphthalin-2-yl)-thiazol-2-yl]-piperidin Hydrochlorid:

¹H NMR (400 MHz, DMSO-d₆) δ [ppm] 9.12 (s, 1 H), 8.87 (s, 1 H), 7.97 (s, 1 H), 7.87 (d, J = 1.8, 1 H), 7.66 (dd, J = 1.9, 8.2, 1 H), 7.38 (d, J = 8.3, 1 H), 3.53 - 3.37 (m, 3H), 3.30 - 3.50 (überlagert, 3H), 3.06 (q, J = 11.9, 2H), 2.51 (dt, J = 1.8, 3.6, 5H), 2.38 - 2.17 (m, 2H), 2.13 - 1.88 (m, 2H), 1.67 (s, 4H), 1.28 (d, J = 13.7, 12H).

Analog der oben genannten Vorschrift werden folgende Verbindungen hergestellt. In einigen Fällen ist eine Aufreinigung mittels Säulenchromatographie an Kieselgel oder mittels präparativer HPLC erforderlich:

| Edukt | Produkt | Rt. in min / HPLC-MS |
|---|---|---|
| 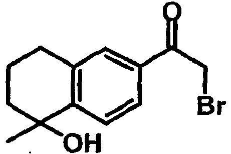 | 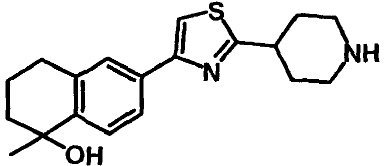 | |
| 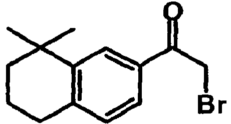 | 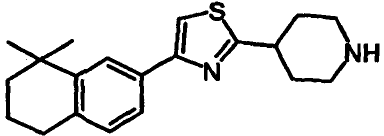 | |
| | ("C2") Hydrochlorid | |
| | ¹H NMR (500 MHz, DMSO-d₆) δ [ppm]8.99 (s, 1H), 8.73 (s, 1H), 7.98 (s, 1H), 7.91 (s, 1H), 7.68 - 7.57 (m, 1H), 7.09 (d, J = 8.0, 1H), 3.34 - 3.46 (m, 3H), 3.07 (dd, J = 9.9, 13.0, 2H), 2.74 (t, J = 6.3, 2H), 2.26 (d, J = 11.9, 2H), 1.97 (dd, J = 11.7, 22.1, 2H), 1.81 - 1.72 (m, 2H), 1.72 - 1.59 (m, 2H), 1.30 (s, 6H) | 2.94 |
| | | 327 [M+H] |
| 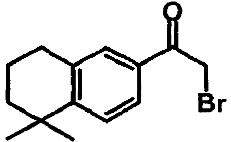 | 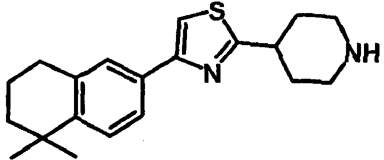 | |
| | ("C3") Trifluoracetat | 2.88 |
| | ¹H NMR (400 MHz, DMSO-d₆) δ [ppm] 8.30 (s, 1H), 7.86 (s, 1H), 7.66 (d, J = 8.2, 1H), 7.59 (s, 1H), 7.39 (d, J = 8.2, 1H), 3.1 - 3.5 (überlagert, 4H), 2.87 - 2.73 (m, 4H), 2.10 (d, J = 12.7, 2H), 1.60-1.80 (m, 6H), 1.26 (s, 6H) | 327 [M+H] |
| 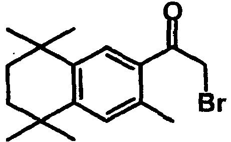 | 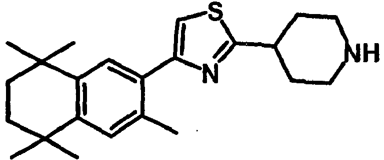 | |
| | ("C4") | |
| 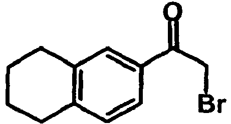 | 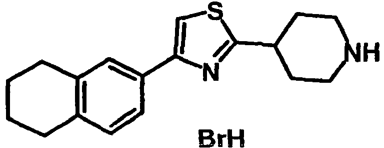 | 2.59 |
| | | 299 [M+H] |
| | ("C5") | |
| 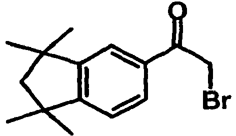 | 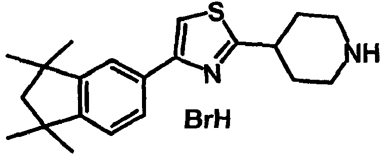 | |
| | ("C6") | |
| | ¹H NMR (400 MHz, DMSO-d₆) δ [ppm] | 2.98 |
| | 9.24 (s, 1H), 8.97 (s, 1H), 7.94 (s, 1H), 7.75 (dd, J = 1.6, 7.9, 1H), 7.69 (d, J = 1.3, 1H), 7.20 (d, J = 7.9, 1H), 3.51 - 3.23 (m, 3H), 3.04 (q, J = 12.4, 2H), 2.24 (d, J = 11.8, 2H), 2.07 - 1.81 (m, 4H), 1.30 (d, J = 9.8, 12H) | 341 [M+H] |
| 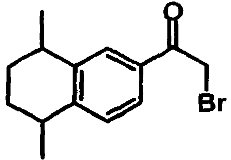 | 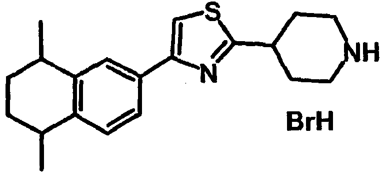 | 2.55 (Meth. B) |
| | ("C7") | 327 [M+H] |
| 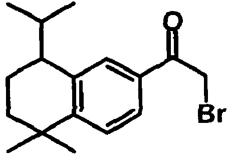 | 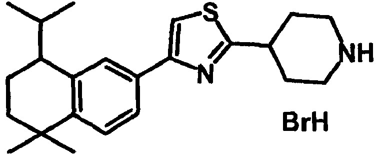 | |
| | ("C8") | |
| 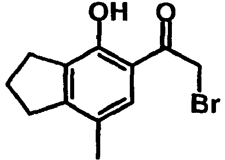 | 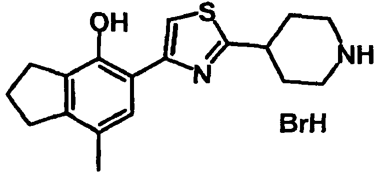 | |
| | ("C9") | |
| 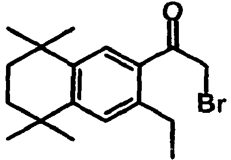 | 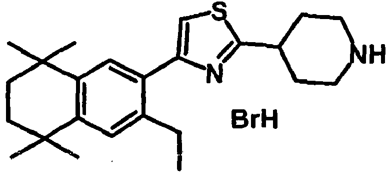 | 3.20 |
| | | 383 [M+H] |
| | ("C10") | |
| 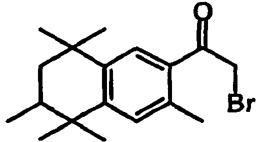 | 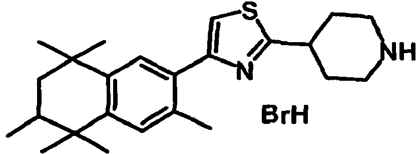 | 3.18 |
| | | 383 [M+H] |
| | ("C11") | |
| 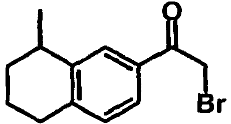 | 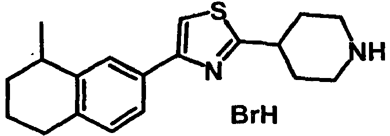 | 2.48 (Meth. B) |
| | | 313 [M+H] |
| | ("C12") | |

### Beispiel 1

### Herstellung von 4-(2-Methyl-3-{4-[4-(5,5,8,8-tetramethyl-5,6,7,8-tetrahydro-naphthalin-2-yl)-thiazol-2-yl]-piperidin-1-yl}-propyl)-morpholin, ("A1")

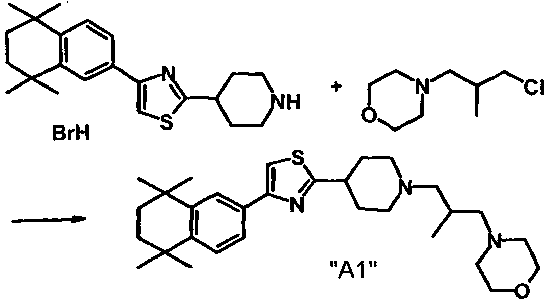

200 mg (0.46 mmol) 4-[4-(5,5,8,8-Tetramethyl-5,6,7,8-tetrahydro-naphthalin-2-yl)-thiazol-2-yl]-piperidin Hydrobromid werden mit 122 mg (0.69 mmol) 4-(3-Chlor-2-methyl-propyl)-morpholin in 5 ml Ethanol und 320 µl (2.3 mmol) Triethylamin 2 h bei 160°C in der Mikrowelle bestrahlt. Das Reaktionsgemisch wird eingedampft und mittels Säulenchromatographie an Kieselgel aufgereinigt. Das Produkt wird noch mittels präparativer HPLC weiter aufgereinigt.
Ausbeute: 49 mg "A1" Trifluoracetat, gelbliches Öl; ESI: 496 g/mol [M+H], HPLC: Rt. = 1.94 min.

### Beispiel 2

### Herstellung von 1'-Ethyl-4-[4-(5,5,8,8-tetramethyl-5,6,7,8-tetrahydronaphthalin-2-yl)-thiazol-2-yl]-[1,3']bipiperidinyl ("A2")

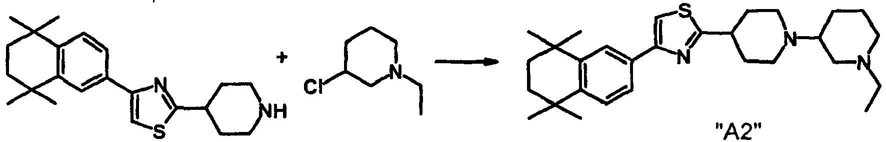

500 mg (1.41 mmol) 4-[4-(5,5,8,8-Tetramethyl-5,6,7,8-tetrahydronaphthalin-2-yl)-thiazol-2-yl]-piperidin werden mit 210 mg (1.42 mmol) 3-Chlor-1-ethyl-piperidin, 360 mg (4.29 mmol) Natriumhydrogencarbonat und 210 mg (1.40 mmol) Natriumiodid in 40 ml DMF suspendiert und 6 h bei 120°C in der Mikrowelle bestrahlt. Das Reaktionsgemisch wird eingedampft und der Rückstand in Etylacetat und 0.1 N NaOH gelöst.
Die organische Phase wirde abgetrennt, eingedampft und mittels Säulenchromatographie an Kieselgel aufgereinigt.
Ausbeute: 45 mg "A2", Hydrochlorid, braune Kristalle, ESI: 466 g/mol [M+H];
¹H NMR (500 MHz, DMSO-d₆) δ [ppm] 11.19 (b, 1H), 7.93 (s, 1H), 7.65 (s, 2H), 7.11 (d, J = 8.4, 1H), 3.0 - 4.0 (überlagert, 7H), 2.76 (d, J = 19.6, 4H), 2.15 - 2.45 (b, 5H), 1.85 - 2.10 (m, 4H), 1.73 - 1.82 (m, 4H), 1.20 - 1.38 (m, 7H).

Analog den oben genannten Vorschriften werden folgende Verbindungen hergestellt:

| Verbindung Nr. | Struktur | ESI (M+H) | HPLC Rt. in min. |
|---|---|---|---|
| "A3" | 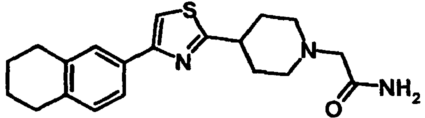 | 356 | |
| "A4" | 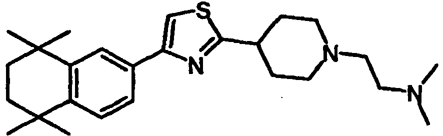 | 426 | |
| | Hydrochlorid | | |
| "A5" | 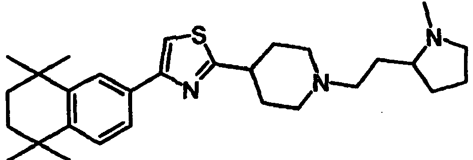 | 466 | |
| | Hydrochlorid | | |
| "A6" | 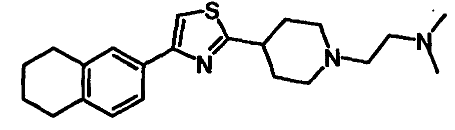 | 370 | |
| | Hydrochlorid | | |
| ¹H NMR (500 MHz, DMSO-d₆) δ [ppm] 7.86 (s, 1H), 7.60 - 7.66 (m, 2H), 7.10 (d, J = 8.5, 1H), 3.01 - 3.12 (m, 3H), 2.87 - 2.96 (m, 2H), 2.76 (d, J = 19.4, 4H), 2.64 (t, J = 6.3, 2H), 2.56 (s, 6H), 2.24 (t, J = 10.7, 2H), 2.08 (d, J = 11.5, 2H), 1.89 - 1.68 (m, 6H) | | | |
| "A7" | 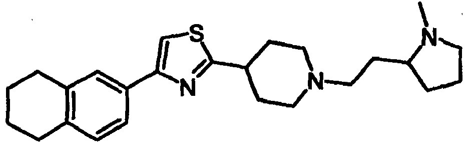 | 410 | |
| | Hydrochlorid | | |
| "A8" | 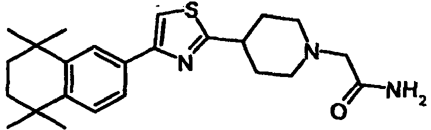 | 411 | |
| | Hydrochlorid | | |
| "A9" | 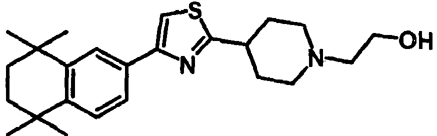 | 399 | |
| | Hydrochlorid | | |
| ¹H NMR (500 MHz, DMSO-d₆) δ 7.92 (s, 1H), 7.85 (d, J = 1.8, 1H), 7.65 (dd, J = 1.8, 8.2, 1H), 7.36 (d, J = 8.3, 1H), 5.01 (b, 1H), 3.55 - 3.75 (m, 2H), 3.10 - 3.50 (überlagert, 4H), 2.50 - 2.90 (m, 3H), 2.08 - 2.22 (m, 2H), 1.82 - 2.00 (m, 2H), 1.66 (s, 4H), 1.27 (d, J = 17.6, 12H) | | | |
| "A10" | 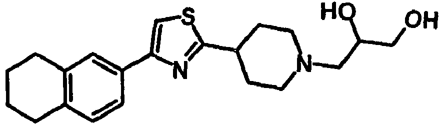 | 373 | |
| "A11" | 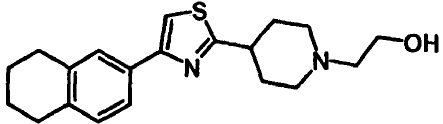 | 343 | |
| | Hydrochlorid | | |
| "A12" | 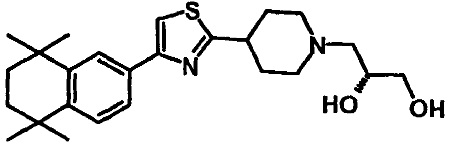 | 429 | |
| | Hydrochlorid | | |
| "A13" | 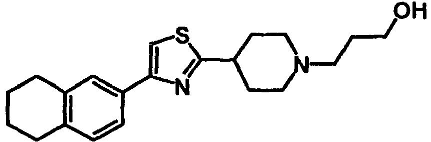 | 357 | |
| "A14" | 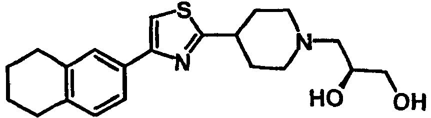 | 373 | |
| "A15" | 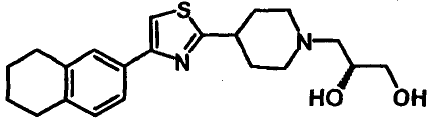 | 373 | |
| "A16" | 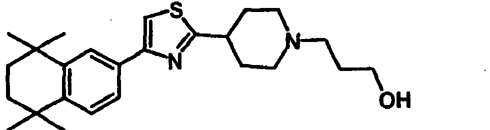 | 413 | 2.94 |
| | Hydrochlorid | | |
| ¹H NMR (500 MHz, DMSO-d₆) δ [ppm] 10.02 (s, 1H), 7.96 (s, 1H), 7.86 (s, 1H), 7.65 (d, J = 8.2, 1H), 7.37 (d, J = 8.3, 1H), 3.30 - 3.80 (überlagert, 5H), 3.00 - 3.20 (m, 4H), 2.39 - 2.18 (m, 3H), 2.03 - 2.17 (m, 2H), 1.87 (s, 2H), 1.67 (s, 4H), 1.27 (d, J = 16.8, 12H) | | | |
| "A17" | 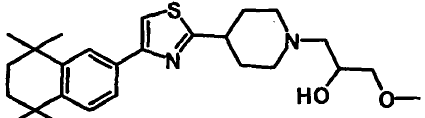 | 443 | 3.00 |
| | Hydrochlorid | | |
| "A18" | 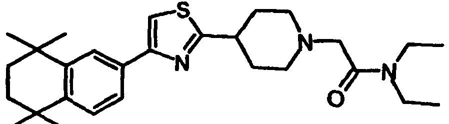 | 468 | 2.88 |
| | Hydrochlorid | | |
| "A19" | 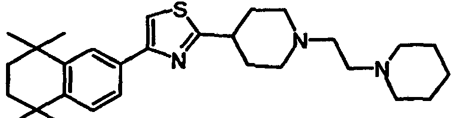 | 466 | 2.87 |
| | Hydrochlorid | | |
| "A20" | 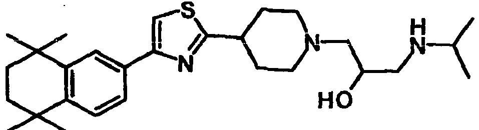 | 470 | 2.76 |
| | Hydrochlorid | | |
| "A21" | 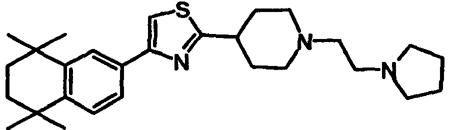 | 452 | 2.80 |
| | Hydrochlorid | | |
| "A22" | 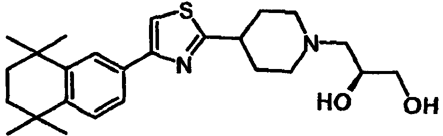 | 429 | 2.88 |
| | Trifluoracetat | | |
| "A23" | 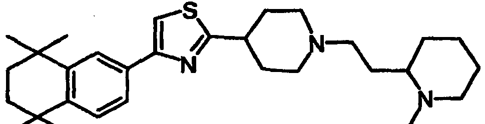 | 480 | 2.83 |
| | Hydrochlorid | | |
| "A24" | 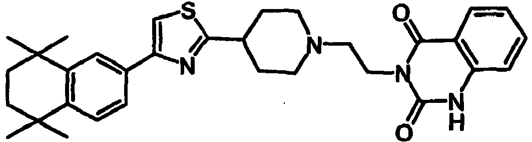 | 543 | 3.11 |
| | Hydrochlorid | | |
| "A25" | 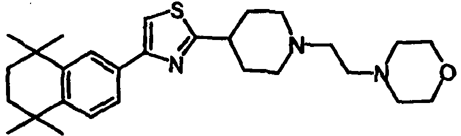 | 468 | 2.07 |
| | Hydrochlorid | | |
| "A26" | 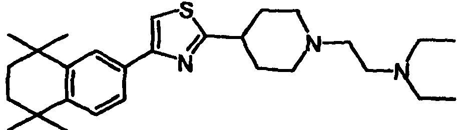 | 454 | 2.87 |
| | Hydrochlorid | | |
| "A27" | 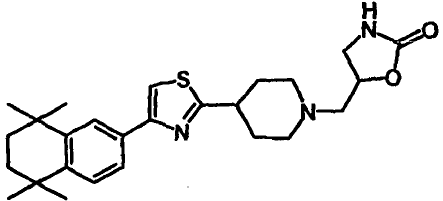 | 454 | 3.00 |
| "A28" | 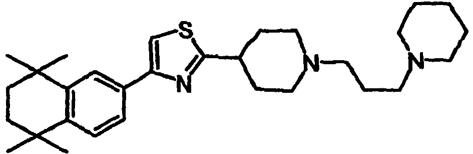 | 480 | 2.82 |
| | Hydrochlorid | | |
| "A29" | 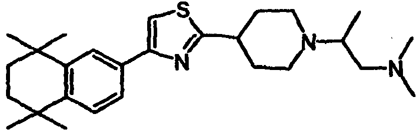 | 440 | 2.85 |
| | Trifluoracetat | | |
| "A30" | 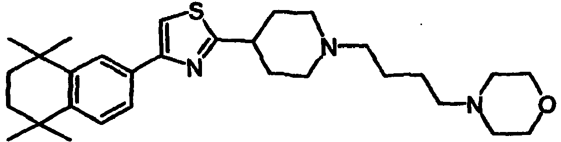 | 496 | 2.80 |
| | Hydrochlorid | | |
| "A31" | 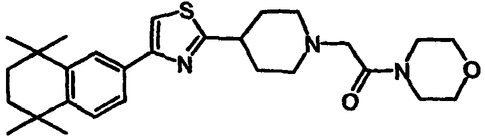 | 482 | 3.08 |
| | Hydrochlorid | | |
| "A32" | 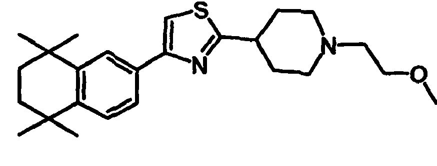 | 413 | 2.19 |
| ¹H NMR (500 MHz, DMSO-d₆) δ [ppm] 7.90 (s, 1H), 7.86 (d, J = 1.8, 1H), 7.65 (dd, J = 1.8, 8.2, 1H), 7.37 (d, J = 8.3, 1H), 2.80 - 3.60 (m, 7H), 2.50 - 2.70 (überlagert, 2H), 2.00 - 2.40 (m, 5H), 1.59 - 1.83 (m, 6H), 1.28 (d, J = 17.1, 12H) | | | |
| "A33" | 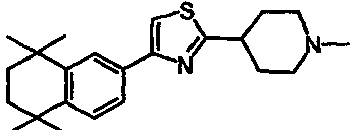 | 369 | 3.01 |
| | Trifluoracetat | | |
| ¹H NMR (400 MHz, DMSO-d₆) δ [ppm]10.18 (s, 1H), 7.98 (s, 1H), 7.86 (d, J = 1.7, 1H), 7.65 (d, J = 8.2, 1H), 7.37 (d, J = 8.3, 1H), 3.52 (m, 2H), 3.35 (m, 1H), 3.10 (m, 2H), 2.77 (t, J = 6.3, 3H), 2.31 (d, J = 13.9, 2H), 2.03 (d, J = 12.7, 2H), 1.66 (s, 4H), 1.27 (d, J = 13.4, 12H) | | | |
| "A34" | 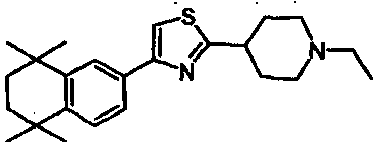 | 383 | 3.06 |
| | Hydrochlorid | | |
| ¹H NMR (500 MHz, DMSO-d₆) δ [ppm] 9.70 (s, 1H), 7.96 (s, 1H), 7.86 (d, J = 1.7, 1H), 7.65 (dd, J = 1.8, 8.2, 1H), 7.37 (d, J = 8.3, 1H), 3.55 - 3.62 (m, 2H), 3.33 - 3.44 (m, 1H), 3.19 - 2.99 (m, 4H), 2.32 (d, J = 13.9, 2H), 2.04 (d, J = 12.9, 2H), 1.67 (s, 4H), 1.35 - 1.18 (m, 15H) | | | |
| "A35" | 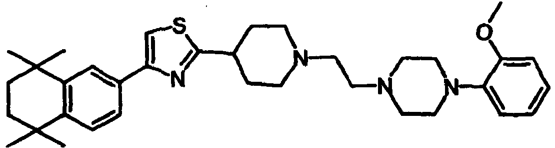 | 573 | 2.97 |
| "A36" | 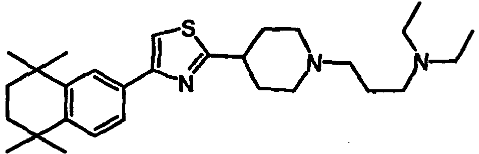 | 468 | 2.80 |
| "B1" | 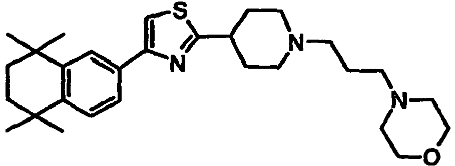 | 482 (M+H) | 3.03 |
| "B2" | 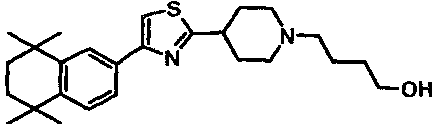 | 427 (M+H) | 2.77 (Meth. B) |
| | Trifluoracetat | | |
| | ¹H NMR (400 MHz, DMSO-d₆) δ [ppm] 8.14 (s, 1H), 7.89 (s, 1H), 7.84 (d, J = 1.8, 1H), 7.64 (dd, J = 1.8, 8.2, 1H), 7.35 (d, J = 8.2, 1H), 4.06 (b, 1H), 3.40 (t, J = 6.2, 2H), 3.04 (m, 1H), 2.98 (d, J = 11.6, 2H), 2.35 (t, J = 6.9, 2H), 2.10 (dd, J = 13.4, 26.1, 4H), 1.73 (dd, J = 12.1, 24.1, 2H), 1.66 (s, 4H), 1.56 - 1.39 (m, 4H), 1.27 (d, J = 17.2, 12H) | | |
| "B3" | 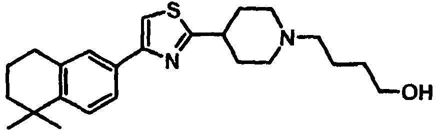 | 399 (M+H) | 2.89 |
| | ¹H NMR (400 MHz, DMSO-d₆) δ [ppm] 7.8 (s, 1H), 7.65 (d, J = 8.2, 1H), 7.59 (s, 1H), 7.38 (d, J = 8.2, 1H), 4.52 (b, 1H), 3.40 (t, J = 6.0, 2H), 2.99 (dt, J = 6.0, 7.8, 1H), 2.93 (d, J = 11.8, 2H), 2.76 (t, J = 6.3, 2H), 2.30 (t, J = 6.9, 2H), 2.03 (t, J = 10.8, 4H), 1.81 - 1.58 (m, 6H), 1.54 - 1.38 (m, 4H), 1.25 (s, 6H) | | |
| "B4" | 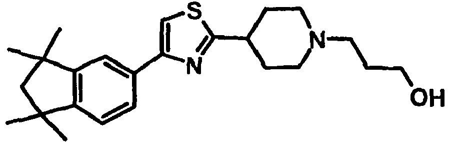 | 399 (M+H) | 3.05 |
| | Hydrochlorid | | |
| "B5" | 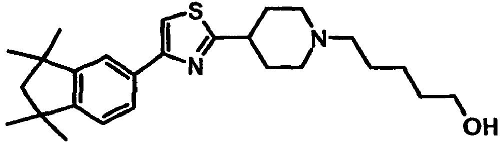 | 427 (M+H) | 3.09 |
| | Hydrochlorid | | |
| "B6" | 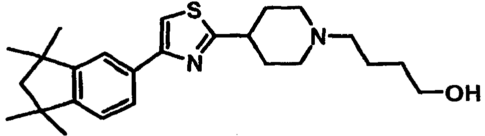 | 413 (M+H) | 3.13 |
| | Hydrochlorid | | |

### Beispiel 3

### Herstellung von (2-{4-[4-(5,5,8,8-Tetramethyl-5,6,7,8-tetrahydronaphthalin-2-yl)-thiazol-2-yl]-piperidin-1-yl}-ethyl)-carbaminsäure-tert.-butylester ("A37")

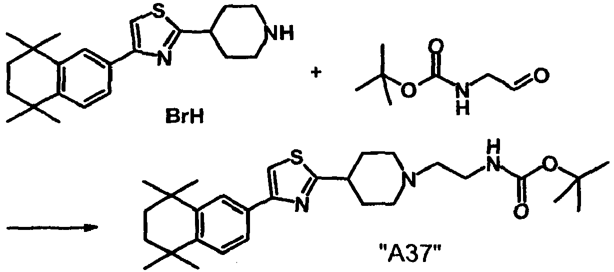

200 mg (0.46 mmol) 4-[4-(5,5,8,8-Tetramethyl-5,6,7,8-tetrahydronaphthalin-2-yl)-thiazol-2-yl}-piperidin Hydrobromid werden mit 10 ml THF und 200 µl Eisessig versetzt und 154 mg (0.92 mmol) (2-Oxoethyl)-carbaminsäure-*tert*.-butylester werden zugegeben. Anschließend werden 195 mg (0.92 mmol) Natriumtrisacetoxyborhydrid zugegeben und das Reaktionsgemisch wird 24 h bei Raumtemperatur gerührt. Das Reaktionsgemisch wird filtriert, die Mutterlauge wird eingedampft und der Rückstand mittels präparativer HPLC aufgereinigt. Man erhält "A37"; ESI: 498 (M+H), HPLC: 3.34 min.

Analog der oben genannten Vorschrift werden folgende Verbindungen hergestellt:

| Verbindung Nr. | Struktur | ESI (M+H) | HPLC Rt. in min. |
|---|---|---|---|
| "A38" | 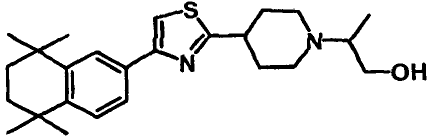 | 413 | 3.05 |
| "A39" | 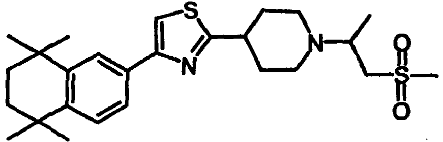 | 474 | 3.71 |
| "A40" | 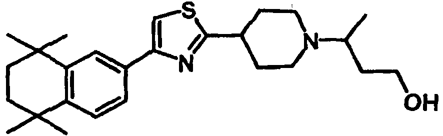 | 427 | 3.04 |
| ¹H NMR (400 MHz, DMSO-d₆) δ [ppm] 7.93 (s, 1H), 7.86 (d, *J* = 1.8, 1H), 7.66 (dd, *J* = 1.8, 8.2, 1H), 7.37 (d, *J* = 8.3, 1H), 2.80 - 3.60 (m, 10H), 1.7 - 2.3 (m, 6H), 1.67 (b, 4H), 1.3 - 1.66 (m, 2H), 1.28 (d, *J* = 13.5, 12H) | | | |
| "A71" | 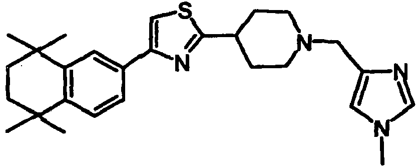 | 449 | 2.78 |
| "A72" | 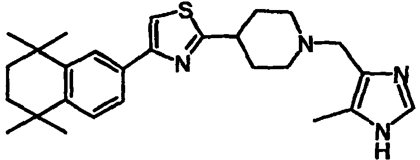 | 449 | 2.84 |
| | Trifluoracetat | | |
| ¹H NMR (500 MHz, DMSO-d₆) δ [ppm] 15.53 - 12.89 (m, 1H), 10.87 - 9.73 (m, 1H), 8.93 (s, 1H), 7.96 (s, 1H), 7.85 (d, *J* = 1.8, 1H), 7.63 (dd, *J* = 1.8, 8.2, 1H), 7.37 (d, *J* = 8.3, 1 H), 4.46 (s, 2H), 3.78 - 2.94 (m, 5H), 2.43 - 2.21 (m, 5H), 1.96 (m, 2H), 1.66 (s, 4H), 1.27 (d, *J* = 14.0, 12H) | | | |
| "A73" | 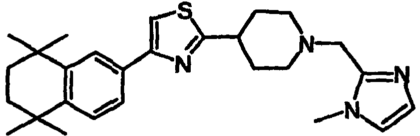 | 449 | 2.98 |
| "A74" | 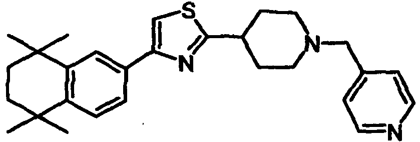 | 446 | 2.83 |
| "A75" | 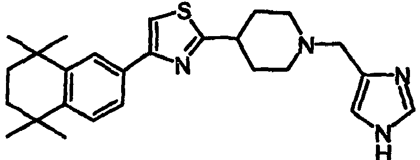 | 435 | 2.78 |
| | Trifluoracetat | | |
| ¹H NMR (500 MHz, DMSO-d₆) δ [ppm] 9.22 (s, 1H), 7.80 - 8.00 (m, 3H), 7.63 (d, *J* = 8.2, 1H), 7.36 (d, *J* = 8.1, 1H), 4.53 (s, 2H), 3.59 (b, 2H), 3.47 - 3.34 (m, 1H), 3.18 (b, 2H), 2.32 (m, 2H), 2.10 - 1.90 (m, 2H), 1.66 (s, 4H), 1.26 (d, *J* = 15.6, 12H) | | | |
| "A76" | 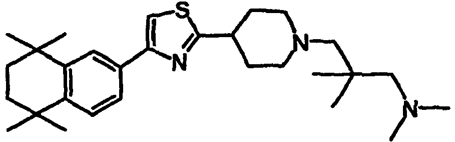 | 468 | 2.78 |
| "A77" | 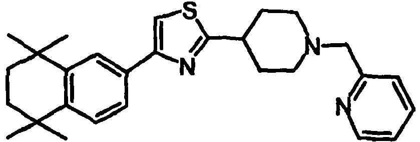 | 446 | 3.19 |
| "B7" | 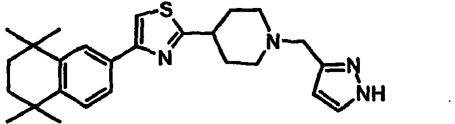 | 435 (M+H) | 3.08 |
| | Trifluoracetat | | |
| | ¹H NMR (400 MHz, DMSO-d₆) δ [ppm] 9.73 (s, 1H), 7.95 (s, 1H), 7.85 (m, 2H), 7.63 (d, J = 8.2, 1H), 7.36 (d, J = 8.3, 1H), 6.46 (d, J = 2.1, 1H), 4.36 (s, 2H), 3.56 (d, J = 12.1, 2H), 3.20 - 3.40 (m, 2H), 3.14 (d, J = 10.6, 2H), 2.41 - 2.18 (m, 3H), 2.06 - 1.89 (m, 2H), 1.66 (s, 4H), 1.27 (d, J = 15.1, 12H). | | |
| "B8" | 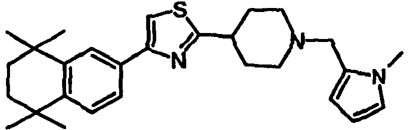 | 448 (M+H) | 3.29 |
| | Trifluoracetat | | |
| "B9" | 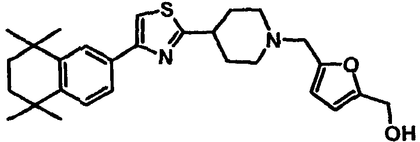 | 465 (M+H) | 3.09 |
| | Trifluoracetat | | |
| "B10" | 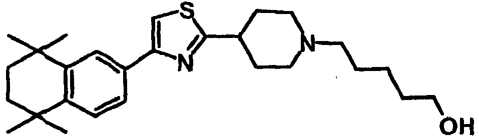 | 441 (M+H) | 3.07. |
| | Trifluoracetat | | |
| | ¹H NMR (400 MHz, DMSO-d₆) δ [ppm] 9.06 (s, 1H), 7.96 (s, 1H), 7.86 (d, *J* = 1.8, 1H), 7.64 (dd, *J =* 1.8, 8.2, 1H), 7.37 (d, *J* = 8.3, 1H), 4.42 (b, 1 H), 3.62 (d, *J =* 11.0, 2H), 3.41 (überlagert, 2H), 3.15 - 3.02 (m, 4H), 2.20 - 2.40 (m, 3H), 1.98 (q, *J* = 12.6, 2H), 1.67 (m, 6H), 1.47 (m, 2H), 1.35 (m, 2H), 1.27 (d, *J* = 16.1, 12H) | | |
| "B11" | 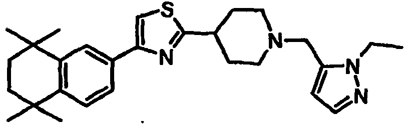 | 463 (M+H) | 3.15 |
| | Trifluoracetat | | |
| "B12" | 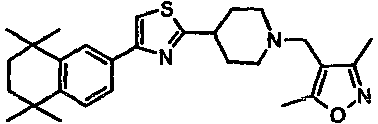 | 464 (M+H) | 3.20 |
| | Trifluoracetat | | |
| "B13" | 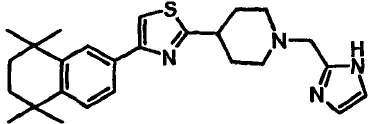 | 435 (M+H) | 3.05 |
| | Hydrochlorid | | |
| | ¹H NMR (400 MHz, DMSO-d₆) δ [ppm] 7.98 (s, 1H), 7.85 (s, 1H), 7.72 (s, 2H), 7.64 (d, J = 10.0, 1H), 7.37 (d, J = 8.3, 1H), 4.49 (b, 2H), 3.0-4.0 (überlagert, 8H), 2.33 (s, 2H), 2.19 - 2.00 (m, 2H), 1.66 (s, 4H), 1.27 (d, J = 12.3, 12H) | | |
| "B14" | 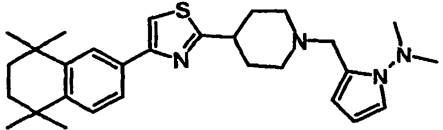 | 477 (M+H) | 3.38 |
| | Trifluoracetat | | |

### Beispiel 4

### Herstellung von (2-{4-[4-(5,5,8,8-Tetramethyl-5,6,7,8-tetrahydro-naphthalin-2-yl)-thiazol-2-yl]-piperidin-1-yl}-ethyl)-carbaminsäure-tert.-butylester ("A41 ")

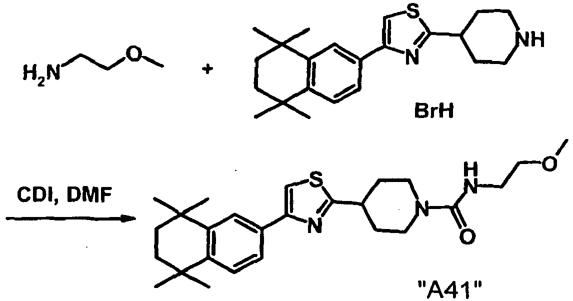

30 µl (0.35 mmol) 2-Methoxyethyamin werden in 3 ml DMF gelöst, mit 56 mg (0.35 mmol) 1,1'-Carbonyldiimidazol versetzt und 2 h bei Raumtemperatur gerührt. Anschließend werden 100 mg (0.23 mmol) 4-[4-(5,5,8,8-Tetramethyl-5,6,7,8-tetrahydro-naphthalin-2-yl)-thiazol-2-yl]-piperidin Hydrobromid und 64 µl (0.46 mmol) Triethylamin zugegeben und das Reaktionsgemisch wird 15 h bei Raumtemperatur gerührt. Das Reaktionsgemisch wird eingedampft und der Rückstand wird mittels Säulenchromatographie an Kieselgel aufgereinigt.
Ausbeute: 13 mg "A41", weißer Feststoff; ESI: 456 (M+H), HPLC: 3.71 min.

Analog der oben genannten Vorschrift werden folgende Verbindungen hergestellt:

| Verbindung Nr. | Struktur | ESI (M+H) | HPLC Rt. in min. |
|---|---|---|---|
| "A42" | 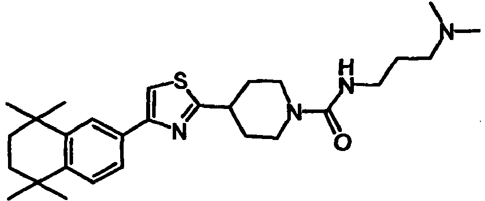 | 484 | 3.16 |
| "A43" | 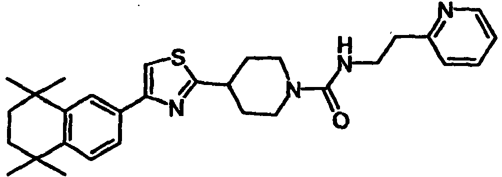 | 504 | 3.17 |
| "A44" | 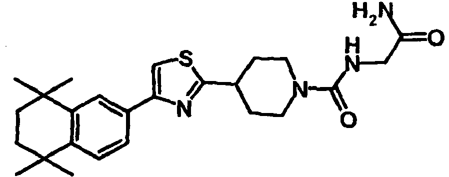 | 456 | 3.33 |
| "A45" | 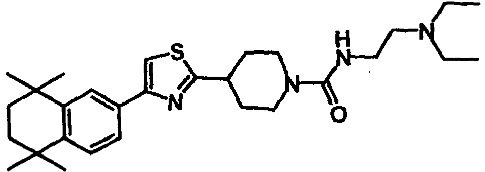 | 498 | 3.26 |
| "A78" | 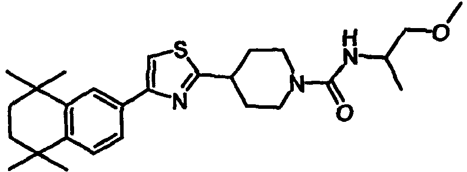 | 470 | 3.76 |
| "A79" | 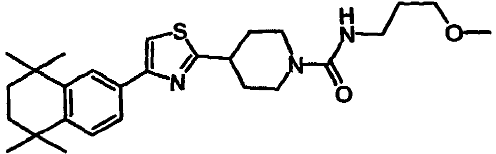 | 470 | 3.74 |

### Beispiel 5

### Herstellung von 4-[4-(5,5,8,8-Tetramethyl-5,6,7,8-tetrahydro-naphthalin-2-yl)-thiazol-2-yl]-piperidin-1-carbonsäure-3-methoxy-propylester ("A46")

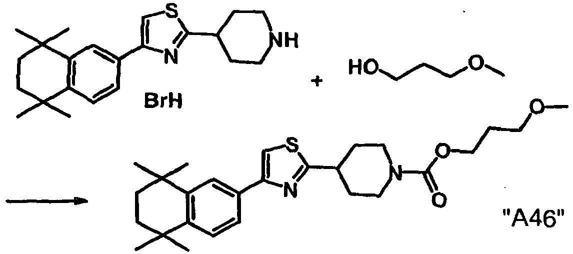

41 µl (0.42 mmol) 3-Methoxy-1-propanol werden in 3 ml DMF gelöst, mit 69 mg (0.42 mmol) 1,1'-Carbonyldiimidazol versetzt und 2 h bei Raumtemperatur gerührt. Anschließend werden 122 mg (0.28 mmol) 4-[4-(5,5,8,8-Tetramethyl-5,6,7,8-tetrahydro-naphthalin-2-yl)-thiazol-2-yl]-piperidin Hydrobromid zugegeben und das Reaktionsgemisch wird 3 Tage bei Raumtemperatur gerührt. Das Reaktionsgemisch wird eingedampft, der Rückstand wird in Ethylacetat aufgenommen und mit 1 N HCl, gesättigter Natriumhydrogencarbonatlösung und gesättigter Natriumchloridlösung gewaschen. Das Rohgemisch wird mittels Säulenchromatographie an Kieselgel aufgereinigt. Ausbeute: 90 mg "A46", ESI: 471 (M+H), HPLC: 4.20 min.

Analog der oben genannten Vorschrift werden folgende Verbindungen hergestellt:

| Verbindung Nr. | Struktur | ESI (M+H) | HPLC Rt. in min. |
|---|---|---|---|
| "A47" | 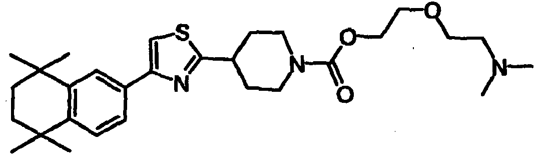 | 514 | 3.27 |
| | Trifluoracetat | | |
| "A48" | 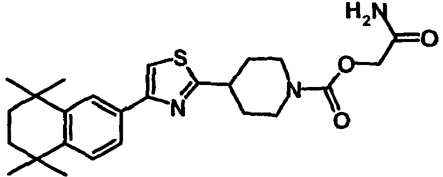 | 504 | 3.17 |
| "A49" | 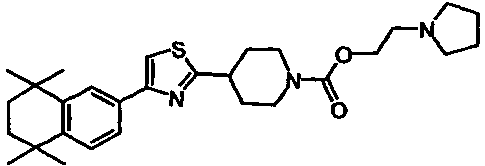 | 496 | 3.28 |
| "A50" | 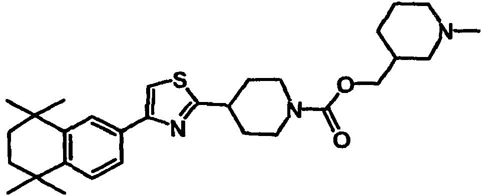 | 510 | 3.32 |
| | Trifluoracetat | | |
| "B15" | 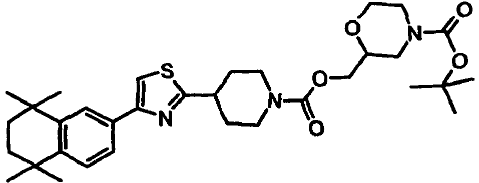 | 598 (M+H) | 4.25 |

### Beispiel 6

### Herstellung von 2-Pyridin-4-yl-1-{4-[4-(5,5,8,8-tetramethyl-5,6,7,8-tetrahydro-naphthalin-2-yl)-thiazol-2-yl]-piperidin-1-yl}-ethanon ("A51")

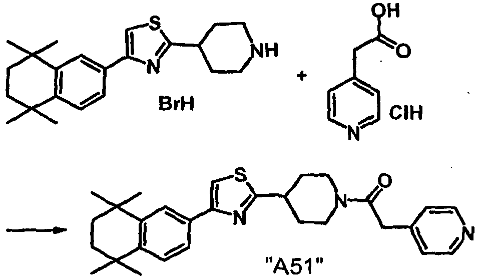

200 mg (0.46 mmol) 4-[4-(5,5,8,8-Tetramethyl-5,6,7,8-tetrahydro-naphthalin-2-yl)-thiazol-2-yl]-piperidin Hydrobromid, 81 mg (0.46 mmol) Pyridin-4-yl-essigsäure, 202 µl (1.84 mmol) 4-Methylmorpholin, 361 mg (1.84 mmol) EDCI und 135 mg (0.73 mmol) HOBt werden in 10 ml THF gelöst und 24 h bei Raumtemperatur gerührt. Das Reaktionsgemisch wird auf gesättigte Ammoniumchloridlösung gegeben und mit Ethylacetat extrahiert. Die organische Phase wird abgetrennt, über Natriumsulfat getrocknet und zum Rückstand eingedampft. Das Rohgemisch wird mittels Säulenchromatographie an Kieselgel aufgereinigt.
Ausbeute: 70 mg "A51 ", ESI: 474 (M+H), HPLC: 3.18 min.

Analog der oben genannten Vorschrift werden folgende Verbindungen hergestellt:

| Verbindung Nr. | Struktur ESI | ESI (M+H) | HPLC Rt. in min. |
|---|---|---|---|
| "A52" | 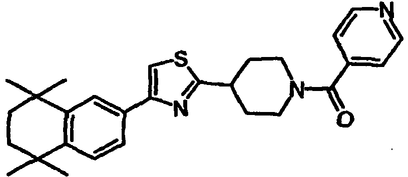 | 460 | 3.24 |
| "A53" | 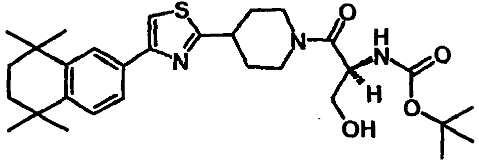 | 542 | |
| "A54" | 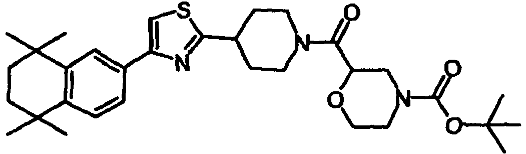 | 568 | |
| "A55" | 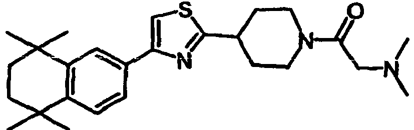 | 440 | |
| | Hydrochlorid | | |
| "A56" | 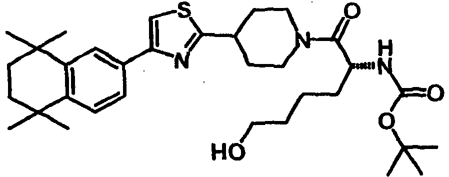 | 584 | |
| "A57" | 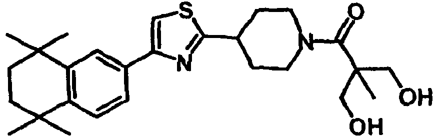 | 471 | |
| "A58" | 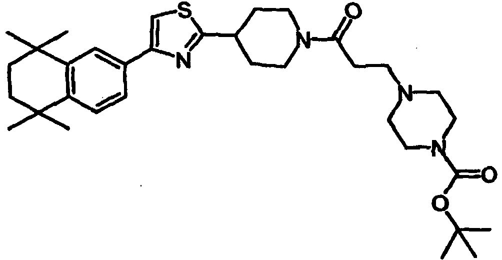 | 595 | |
| "A59" | 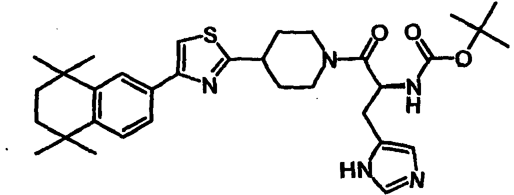 | 592 | |
| "A60" | 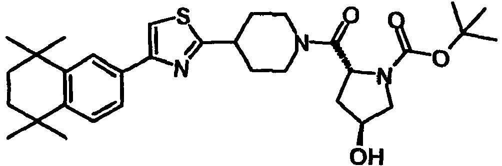 | 568 | |
| "A61" | 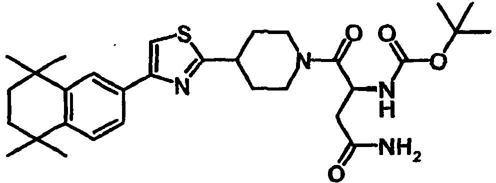 | 569 | |
| "A62" | 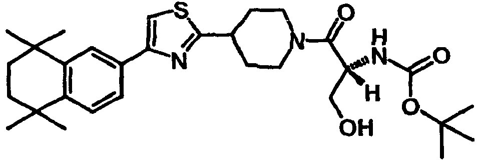 | 542 | |
| "A80" | 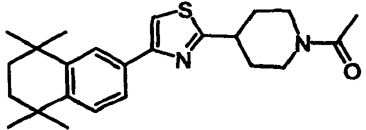 | 397 | 3.71 |

### Beispiel 7

### Herstellung von (S)-2-Amino-3-(3H-imidazol-4-yl)-1-{4-[4-(5,5,8,8-tetramethyl-5,6,7,8-tetrahydro-naphthalin-2-yl)-thiazol-2-yl]-piperidin-1-yl}-propan-1-on ("A63")

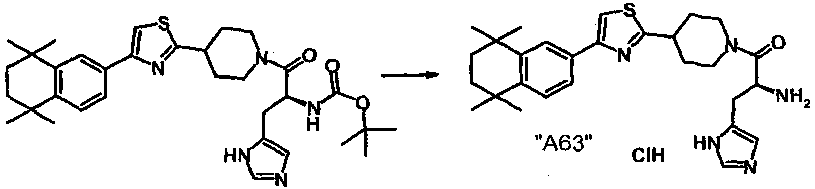

250 mg (0.42 mmol) ((S)-1-(3H-Imidazol-4-ylmethyl)-2-oxo-2-{4-[4-(5,5,8,8-tetramethyl-5,6,7,8-tetrahydro-naphthalin-2-yl)-thiazo]-2-y)]-piperidin-1-yl}-ethyl)-carbaminsäure-*tert*.-butylester werden in 20 ml Dichlormethan und 1 ml Trifluoressigsäure gelöst und 12 h bei Raumtemperatur gerührt. Das Reaktionsgemisch wird mit 0.1 N NaOH gewaschen, die organische Phase über Natriumsulfat getrocknet und mittels Säulenchromatographie an Kieselgel aufgereinigt. Das Rohprodukt wird in Aceton gelöst und mit 1 N HCl wird ein pH-Wert von 4 eingestellt. Dabei bildet sich ein weißer Niederschlag, der abgesaugt, mit Ether gewaschen und im Vakuum getrocknet wird. Ausbeute: 200 mg "A63" Hydrochlorid, weißer Feststoff; ESI: 492 (M+H).

Analog der oben genannten Vorschrift werden folgende Verbindungen hergestellt:

| Verbindung Nr. | Struktur | ESI (M+H) | HPLC Rt. in min. |
|---|---|---|---|
| "A64" | 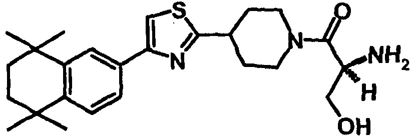 | 442 | |
| | Hydrochlorid | | |
| "A65" | 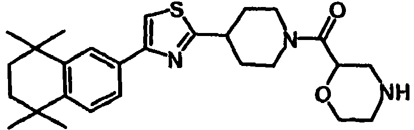 | 468 | |
| | Hydrochlorid | | |
| "A66" | 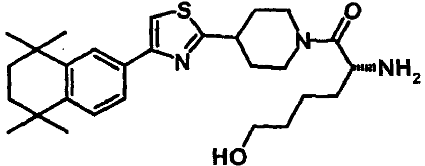 | 484 | |
| | Hydrochlorid | | |
| "A67" | 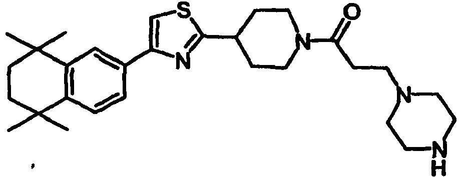 | 495 | |
| "A68" | 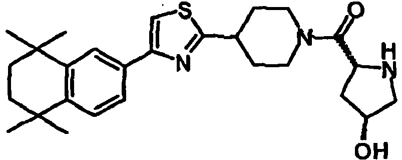 | 468 | |
| "A69" | 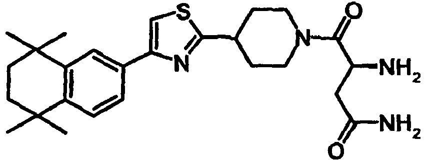 | 469 | |
| "A70" | 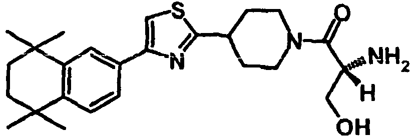 | 442 | |
| "A81" | 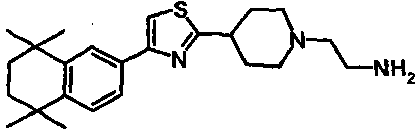 | 398 | 2.78 |
| | Trifluoracetat | | |
| "B16" | 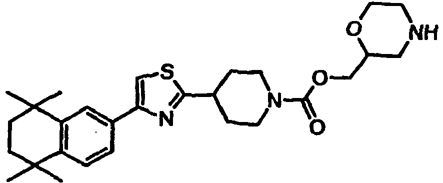 | 498 (M+H) | 3.21 |
| | Trifluoracetat | | |

### Beispiel 8

### Herstellung von (S)-2-Amino-3-(3H-imidazol-4-yl)-1-{4-[4-(5,5,8,8-tetramethyl-5,6,7,8-tetrahydro-naphthalin-2-yl)-thiazol-2-yl]-piperidin-1-yl}-propan-1-on ("A82")

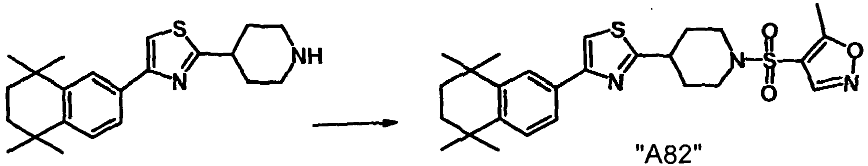

200 mg (0.46 mmol) 4-[4-(5,5,8,8-Tetramethyl-5,6,7,8-tetrahydro-naphthalin-2-yl)-thiazol-2-yl]-piperidin Hydrobromid werden in 5 ml Dichlormethan mit 255 µl (1.84 mmol) Triethylamin und 97 mg (0.51 mmol) 5-Methyl-4-isoxazolsulfonyl-chlorid versetzt. Das Reaktionsgemisch wird 24 h bei Raumtemperatur gerührt. Das Reaktionsgemisch wird mit gesättigter Natriumhydrogencarbonatlösung gewaschen, die organische Phase über Natriumsulfat getrocknet und mittels Säulenchromatographie an Kieselgel aufgereinigt. Das Produkt wird aus Ether kristallisiert.
Ausbeute: 98 mg "A82", weißer Feststoff; ESI: 500 (M+H), Rt. = 3.83 min.

Analog erhält man

| Verbindung Nr. | Struktur | ESI (M+H) | HPLC Rt. in min. |
|---|---|---|---|
| "A83" | 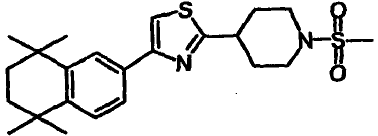 | 433 | 3.84 |

### Pharmakologische Daten

### Met-Kinase-Inhibierung (Enzym Assay)

**Tabelle 1**

| Verbindung Nr. | IC₅₀ | Verbindung Nr. | IC₅₀ |
|---|---|---|---|
| "A1" | C | "A31" | B |
| "A2" | B | "A32" | A |
| "A3" | C | "A33" | A |
| "A4" | B | "A34" | A |
| "A5" | B | "A35" | C |
| "A6" | C | "A36" | B |
| "A7" | C | "A37" | C |
| "A8" | B | "A38" | A |
| "A9" | A | "A40" | A |
| "A10" | | "A41" | B |
| "A11" | B | "A42" | B |
| "A12" | B | "A43" | B |
| "A13" | B | "A44" | C |
| "A14" | B | "A45" | C |
| "A15" | B | "A46" | B |
| "A16" | B | "A47" | B |
| "A17" | A | "A48" | B |
| "A18" | B | "A49" | C |
| "A19" | B | "A50" | C |
| "A20" | A | "A51" | B |
| "A21" | B | "A52" | B |
| "A22" | A | "A55" | C |
| "A23" | B | "A57" | B |
| "A24" | B | "A63" | B |
| "A25" | B | "A64" | B |
| "A26" | B | "A65" | A |
| "A27" | B | "A66" | A |
| "A28" | A | "A67" | C |
| "A29" | B | "A68" | B |
| "A30" | A | "A69" | B |
| | | | |

| Verbindung Nr. | IC₅₀ | Verbindung Nr. | IC₅₀ |
|---|---|---|---|
| "A70" | B | "A81" | A |
| "A71" | B | "A82" | B |
| "A72" | A | "A83" | C |
| "A73" | A | "B1" | B |
| "A74" | B | "B2" | A |
| "A75" | A | "B3" | A |
| "A76" | C | "B4" | A |
| "A77" | B | "B5" | A |
| "A78" | B | "B6" | A |
| "A79" | B | "B7" | A |
| "A80" | B | "B8" | B |
| | | "B9" | B |
| "C1" | A | "B10" | A |
| "C2" | A | "B11" | B |
| "C3" | A | "B12" | B |
| "C5" | B | "B13" | A |
| "C6" | A | "B14" | B |
| "C7" | A | "B15" | |
| "C10" | B | "B16" | B |
| "C11" | C | | |

| | | | |
|---|---|---|---|
| IC₅₀: 10 nM - 1 µM = A 1 µM-10 µM=B > 10 µM = C | | | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verwendung von Verbindungen der Formel I 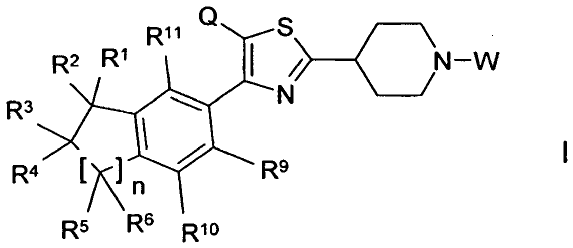 worin
R¹, R², R³, R⁴, R⁵, R⁶ jeweils unabhängig voneinander H oder A',
Q H, A, Hal, COOR⁷ oder CON(R^{7'}R^{7'}),
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, SO₂-[C(R⁸R^{8'})]ₚZ, CO[C(R⁸R^{8'})]ₚNHCOOA oder [C(R⁸R^{8'})]ₚNHCOOA,
R⁷, R^{7'} jeweils unabhängig voneinander H oder A',
R⁸, R^{8'} jeweils unabhängig voneinander H, A, OA, NAA' oder Het,
R⁹ H oder A',
R¹⁰, R¹¹ jeweils unabhängig voneinander H, A' oder OH,
Z Het, Ar oder A,
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, S, SO, SO₂, CO, COO, NR⁷, NR⁷CO, CONR⁷, CH=CH und/oder CH≡CH-Gruppen ersetzt sein können
oder
cyclisches Alkyl mit 3-7 C-Atomen,
Ar unsubstituiertes oder ein-, zwei-, drei-, vier- oder fünffach durch Hal, A, [C(R⁷R^{7'})]ₚOR⁷, [C(R⁷R^{7'})]ₚN(R⁷)₂, SR⁷, NO₂, CN, CHO, COOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R^{7'}), NHCOO[C(R⁷R^{7'})]ₚN(R⁷)₂, NHCOO[C(R⁷R^{7'})]ₚHet, NHCONH[C(R⁷R^{7'})]ₚN(R⁷)₂, NHCONH[C(R⁷R^{7'})]ₚHet, OCONH[C(R⁷R^{7'})]ₚN(R⁷)₂, OCONH[C(R⁷R^{7'})]ₚHet, CONR⁷[C(R⁷R^{7'})]ₚN(R⁷)₂, CONR⁷[C(R⁷R⁷)]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet und/oder S(O)ₘ[C(R⁷R^{7'})]ₚAr' substituiertes Phenyl, Naphthyl oder Biphenyl,
Ar' unsubstituiertes oder ein-, zwei-, drei-, vier- oder fünffach durch Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA und/oder COA substituiertes Phenyl,
A' unverzweigtes oder verzweigtes Alkyl mit 1-8 C-Atomen, worin 1-7 H-Atome durch F, Cl und/oder Br ersetzt sein können,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR⁷, [C(R⁷R^{7'})]ₚAr, [C(R⁷R^{7'})]ₚHet', COA, CO[C(R⁷R^{7'})]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R^{7'}), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR⁷SO₂A, NR⁷SO₂Ar, NR⁷SO₂Het', SO₂N(R⁷)A, SO₂N(R⁷)Ar, SO₂N(R⁷)Het', CHO, =S, =NH, =NA, Oxy (-O⁻) und/oder =O (Carbonylsauerstoff) substituiert sein kann,
Het' einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
m 0, 1 oder 2,
n 1, 2 oder 3,
p 0, 1, 2, 3 oder 4,
sowie ihrer pharmazeutisch verwendbaren Salze, Tautomeren und Stereoisomeren, einschließlich deren Mischungen in allen Verhältnissen,
zur Herstellung eines Arzneimittels zur Behandlung von Krankheiten,
wobei die zu behandelnden Krankheiten ausgewählt sind aus der Gruppe
hyperproliferative Erkrankung, inflammatorische Erkrankung, angiogene Erkrankung, fibrotische Erkrankung der Lunge, Niere, Leber und des Herzens.

2. Verwendung nach Anspruch 1, wobei die hyperproliferative Erkrankung ausgewählt ist aus der Gruppe
Krebs (Tumorerkrankung), Atherosclerose, Restenose, proliferative Erkrankung der Mesangiumzellen, Psoriasis.

3. Verwendung nach Anspruch 2, wobei die Tumorerkrankung ausgewählt ist aus der Gruppe
Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

4. Verwendung nach Anspruch 2, wobei die proliferative Erkrankung der Mesangiumzellen ausgewählt ist aus der Gruppe
Glomerulonephritis, diabetische Nephropathie, maligne Nephrosclerose, thrombotisches Mikroangiopathie-Syndrom, Transplantatabstossung, Glomerulopathie.

5. Verwendung nach Anspruch 1, wobei die inflammatorische Erkrankung ausgewählt ist aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis, durch T-Zellen - vermittelte Immunerkrankung.

6. Verwendung nach Anspruch 5, wobei die entzündliche Darmerkrankung ausgewählt ist aus der Gruppe
ulcerative Colitis, Morbus Crohn, unbestimmte Colitis.

7. Verwendung nach Anspruch 5, wobei die durch T-Zellen - vermittelte Immunerkrankung ausgewählt ist aus der Gruppe
allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

8. Verwendung nach Anspruch 5, wobei die Arthritis-Erkrankung ausgewählt ist aus der Gruppe
rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

9. Verwendung nach Anspruch 5, wobei die entzündliche Nierenerkrankung ausgewählt ist aus der Gruppe
Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

10. Verwendung nach Anspruch 5, wobei die entzündliche Hauterkrankung ausgewählt ist aus der Gruppe
Psoriasis, atopische Dermatitis, Kontaktempfindlichkeit, Akne.

11. Verwendung nach Anspruch 1, wobei die angiogene Erkrankung ausgewählt ist aus der Gruppe
diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

12. Verwendung nach einem oder mehreren der Ansprüche 1-11,
von Verbindungen der Formel I,
worin
R¹, R² jeweils unabhängig voneinander H oder A',
R³, R⁴ H,
R⁵, R⁶ jeweils unabhängig voneinander H oder A',
R⁷, R^{7'} jeweils unabhängig voneinander H oder A',
R⁸, R^{8'} jeweils unabhängig voneinander H, A oder Het,
R⁹ H oder A',
R¹⁰, R¹¹ jeweils unabhängig voneinander H, A' oder OH,
Q H,
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA oder [C(R⁸R^{8'})]ₚNHCOOA,
Z Het oder A,
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, NR⁷, SO₂, NR⁷CO und/oder CONR⁷-Gruppen ersetzt sein können,
A' unverzweigtes oder verzweigtes Alkyl mit 1-8 C-Atomen, worin 1-7 H-Atome durch F, Cl und/oder Br ersetzt sein können,
Ar unsubstituiertes oder ein-, zwei-, drei-, vier- oder fünffach durch Hal und/oder [C(R⁷R^{7'})]ₚOR⁷ substituiertes Phenyl,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann,
n 1 oder 2,
p 0, 1, 2, 3 oder 4,
bedeuten,
sowie ihrer pharmazeutisch verwendbaren Salze, Tautomeren und
Stereoisomeren, einschließlich deren Mischungen in allen Verhältnissen.

13. Verwendung nach einem oder mehreren der Ansprüche 1-12,
von Verbindungen der Formel I ausgewählt aus der Gruppe
| Nr. | Struktur und/oder Name |
|---|---|
| "A1" | 4-(2-Methyl-3-{4-[4-(5,5,8,8-tetramethyl-5,6,7,8-tetrahydro-naphthalin-2-yl)-thiazol-2-yl]-piperidin-1-yl}-propyl)-morpholin |
| "A2" | 1'-Ethyl-4-[4-(5,5,8,8-tetramethyl-5,6,7,8-tetrahydro-naphthalin-2-yl)-thiazol-2-yl]-[1,3']bipiperidinyl |
| "A3" | 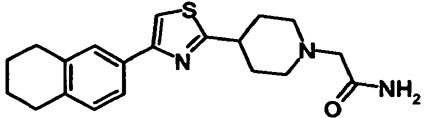 |
| "A4" | 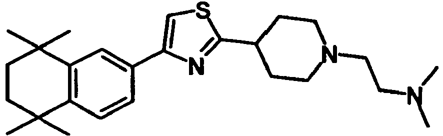 |
| "A5" | 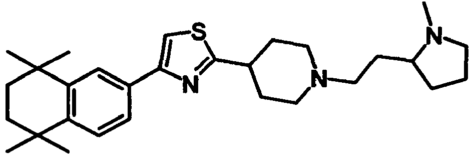 |
| "A6" | 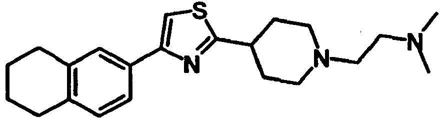 |
| "A7" | 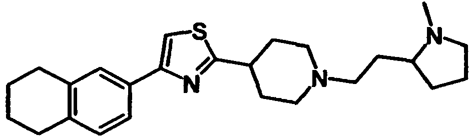 |
| "A8" | 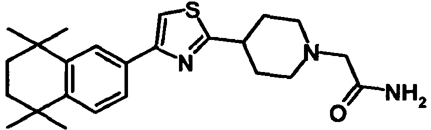 |
| "A9" | 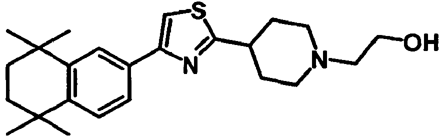 |
| "A10" | 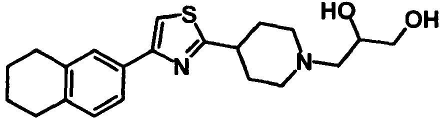 |
| "A11" | 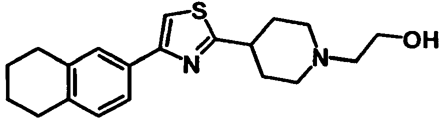 |
| "A12" | 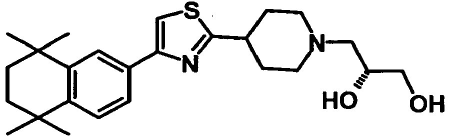 |
| "A13" | 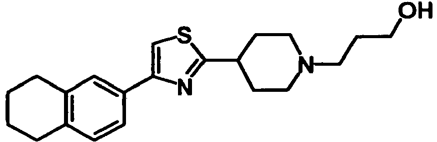 |
| "A 14" | 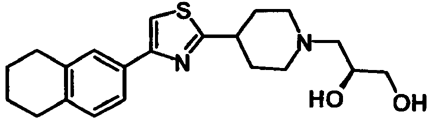 |
| "A15" | 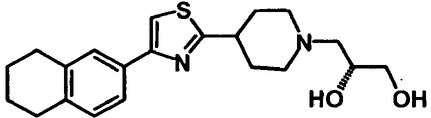 |
| "A16" | 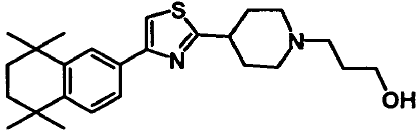 |
| "A17" | 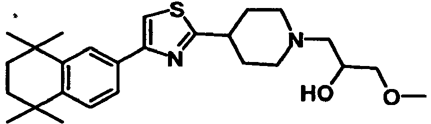 |
| "A18" | 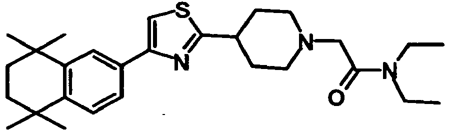 |
| "A19" | 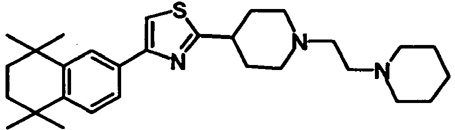 |
| "A20" | 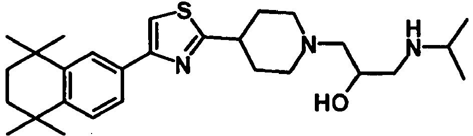 |
| "A21" | 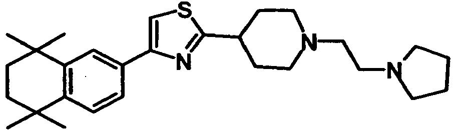 |
| "A22" | 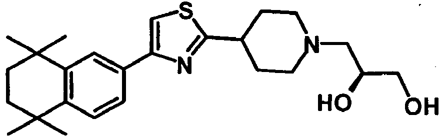 |
| "A23" | 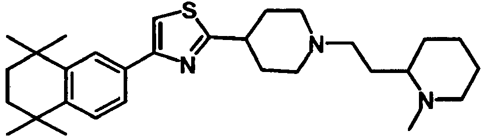 |
| "A24" | 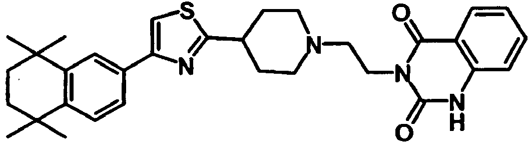 |
| "A25" | 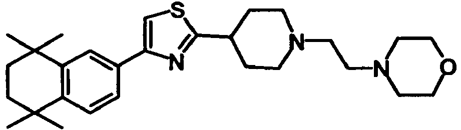 |
| "A26" | 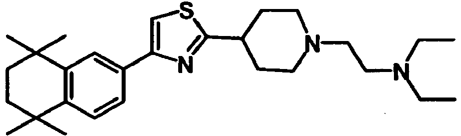 |
| "A27" | 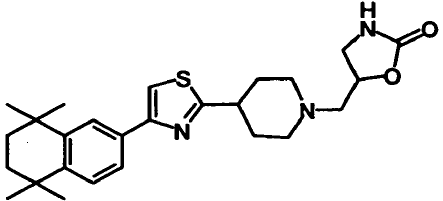 |
| "A28" | 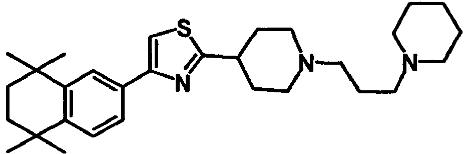 |
| "A29" | 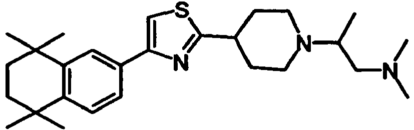 |
| "A30" | 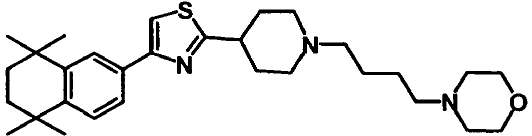 |
| "A31" | 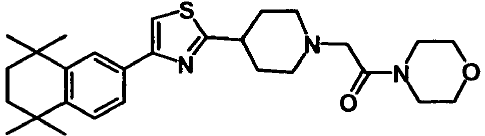 |
| "A32" | 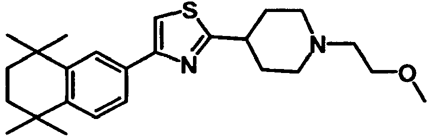 |
| "A33" | 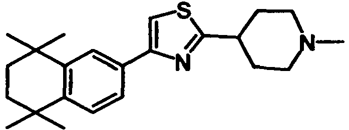 |
| "A34" | 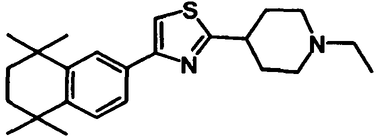 |
| "A35" | 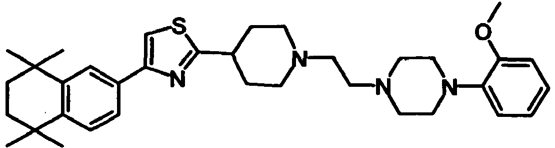 |
| "A36" | 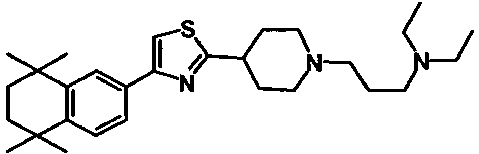 |
| "A37" | (2-{4-[4-(5,5,8,8-Tetramethyl-5,6,7,8-tetrahydro-naphthalin-2-yl)-thiazol-2-yl]-piperidin-1-yl}-ethyl)-carbaminsäure-*tert*.-butylester |
| "A38" | 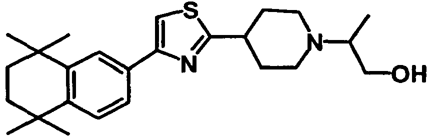 |
| "A39" | 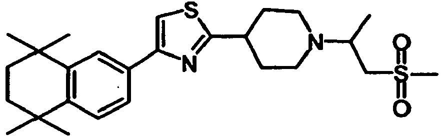 |
| "A40" | 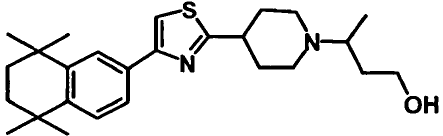 |
| "A41" | (2-{4-[4-(5,5,8,8-Tetramethyl-5,6,7,8-tetrahydro-naphthalin-2-yl)-thiazol-2-yl]-piperidin-1-yl}-ethyl)-carbaminsäure-*tert*.-butylester |
| "A42" | 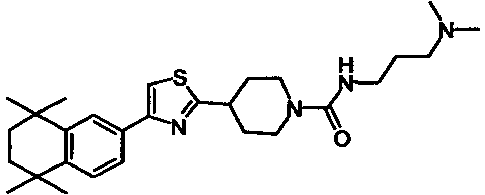 |
| "A43" | 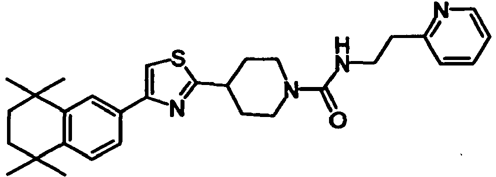 |
| "A44" | 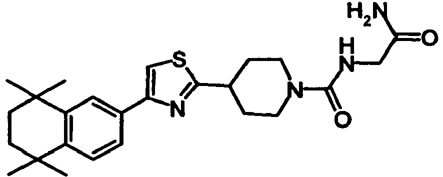 |
| "A45" | 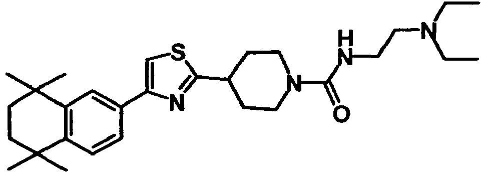 |
| "A46" | 4-[4-(5,5,8,8-Tetramethyl-5,6,7,8-tetrahydro-naphthalin-2-yl)-thiazol-2-yl]-piperidin-1-carbonsäure-3-methoxy-propylester |
| "A47" | 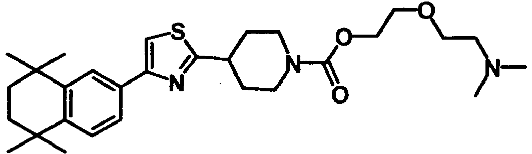 |
| "A48" | 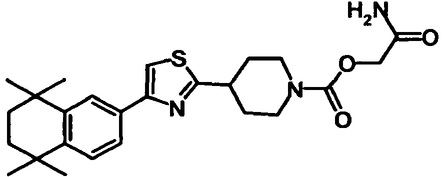 |
| "A49" | 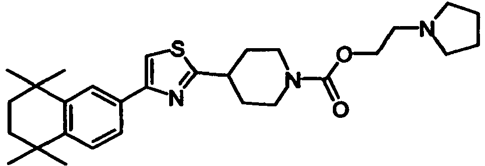 |
| "A50" | 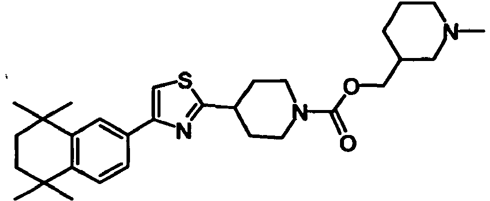 |
| "A51" | 2-Pyridin-4-yl-1-{4-[4-(5,5,8,8-tetramethyl-5,6,7,8-tetrahydro-naphthalin-2-yl)-thiazol-2-yl]-piperidin-1-yl}-ethanon |
| "A52" | 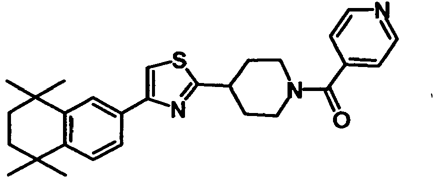 |
| "A53" | 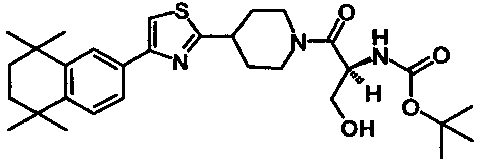 |
| "A54" | 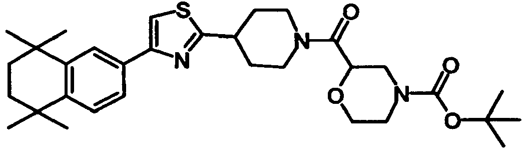 |
| "A55" | 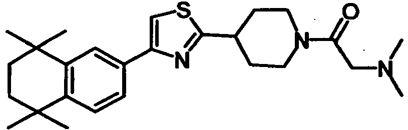 |
| "A56" | 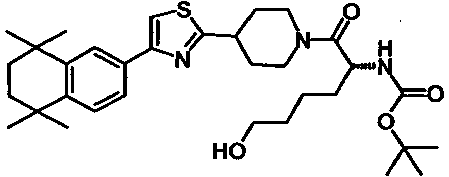 |
| "A57" | 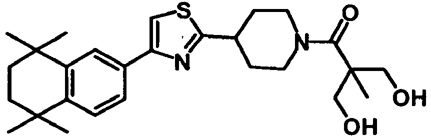 |
| "A58" | 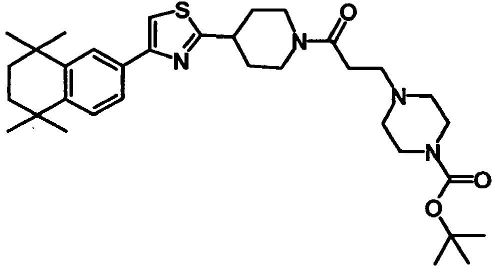 |
| "A59" | 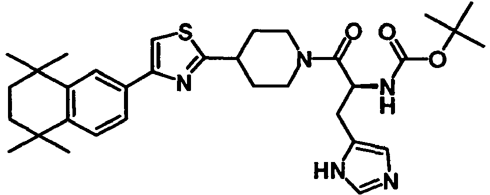 |
| "A60" | 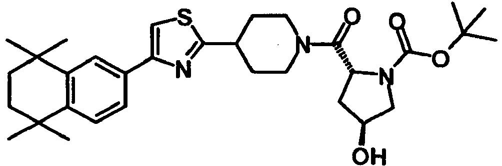 |
| "A61" | 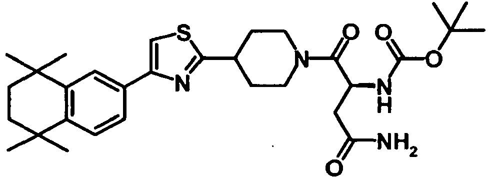 |
| "A62" | 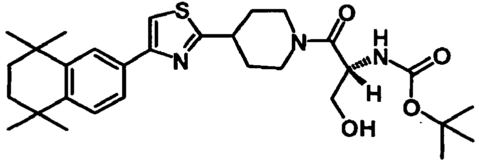 |
| "A63" | (S)-2-Amino-3-(3H-imidazol-4-yl)-1-{4-[4-(5,5,8,8-tetramethyl-5,6,7,8-tetrahydro-naphthalin-2-yl)-thiazol-2-yl]-piperidin-1-yl}-propan-1-on |
| "A64" | 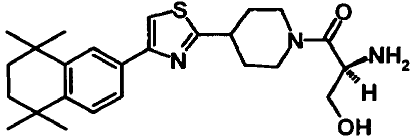 |
| "A65" | 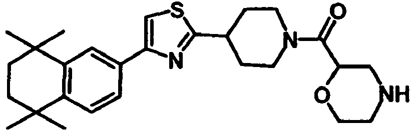 |
| "A66" | 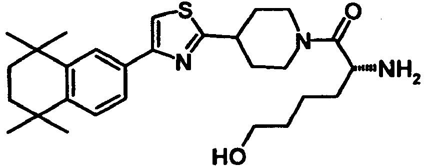 |
| "A67" | 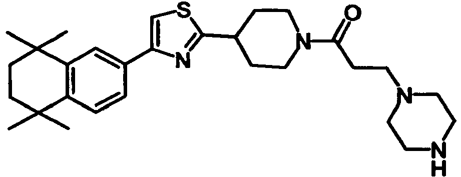 |
| "A68" | 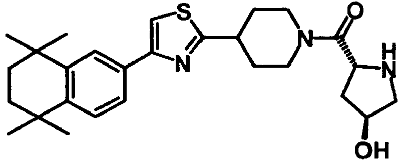 |
| "A69" | 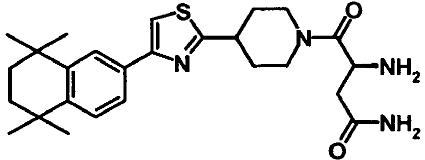 |
| "A70" | 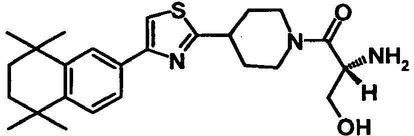 |
| "A71" | 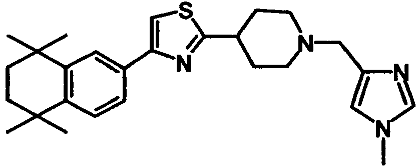 |
| "A72" | 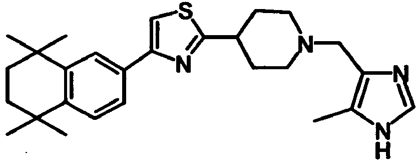 |
| "A73" | 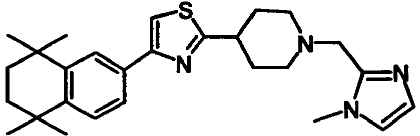 |
| "A74" | 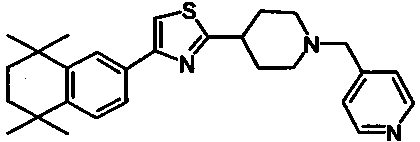 |
| "A75" | 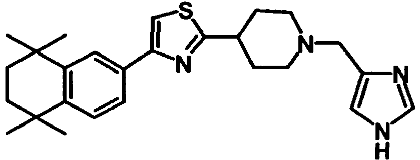 |
| "A76" | 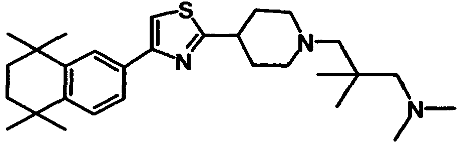 |
| "A77" | 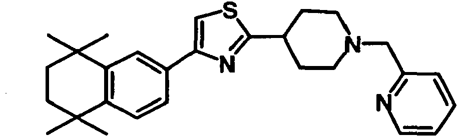 |
| "A78" | 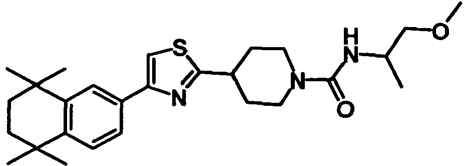 |
| "A79" | 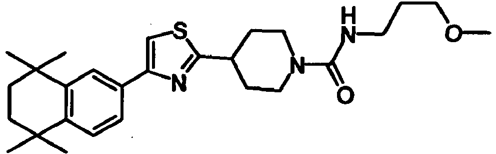 |
| "A80" | 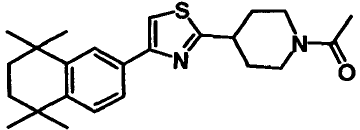 |
| "A81" | 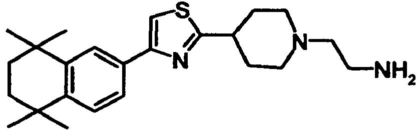 |
| "A82" | 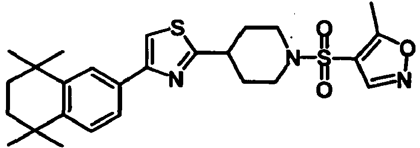 |
| "A83" | 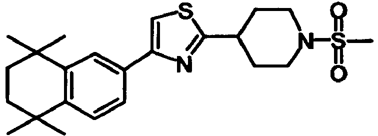 |
| "B1" | 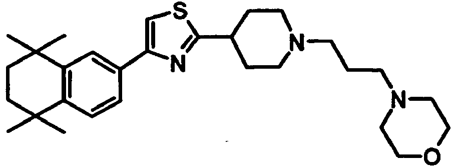 |
| "B2" | 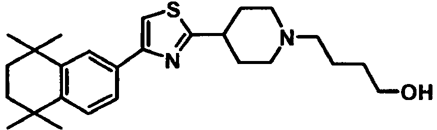 |
| "B3" | 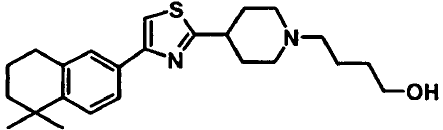 |
| "B4" | 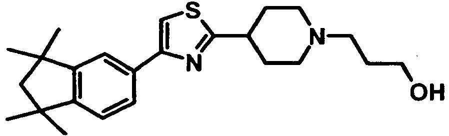 |
| "B5" | 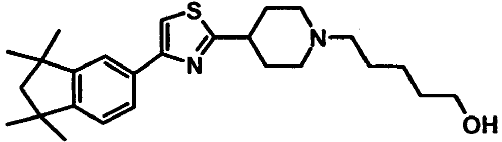 |
| "B6" | 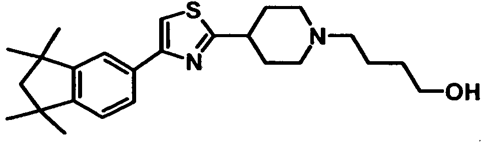 |
| "B7" | 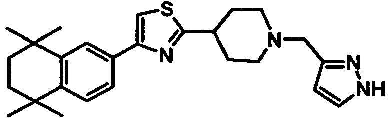 |
| "B8" | 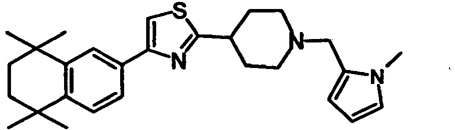 |
| "B9" | 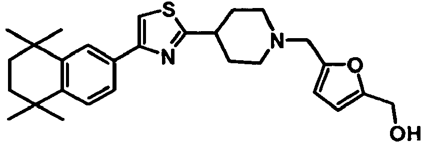 |
| "B10" | 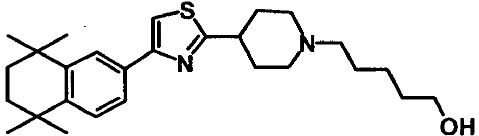 |
| "B11" | 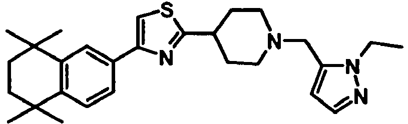 |
| "B12" | 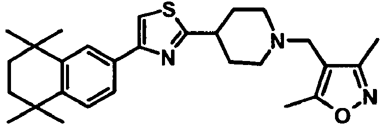 |
| "B13" | 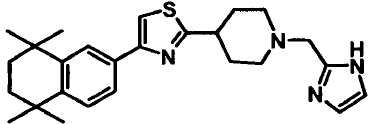 |
| "B14" | 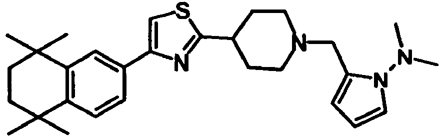 |
| "B15" | 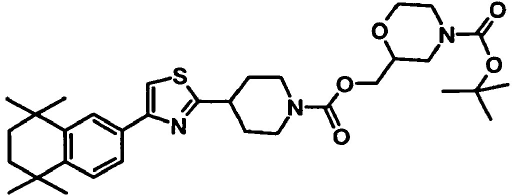 |
| "B16" | 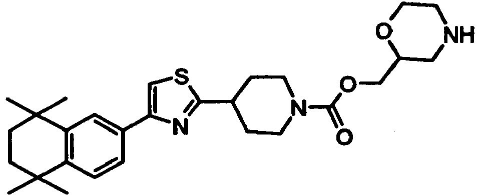 |
sowie ihre pharmazeutisch verwendbaren Salze, Tautomere und Stereoisomere, einschließlich deren Mischungen in allen Verhältnissen.

14. Verbindungen der Formel I 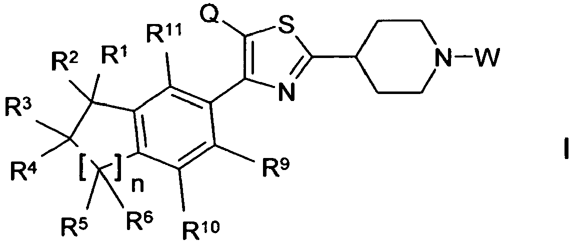 worin
R¹, R², R³, R⁴, R⁵, R⁶ jeweils unabhängig voneinander H oder A',
Q H, A, Hal, COOR⁷ oder CON(R⁷R^{7'}),
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, SO₂-[C(R⁸R^{8'})]ₚZ, CO[C(R⁸R^{8'})]ₚNHCOOA oder [C(R⁸R^{8'})]ₚNHCOOA,
R⁷, R^{7'} jeweils unabhängig voneinander H oder A',
R⁸, R^{8'} jeweils unabhängig voneinander H, A, OA, NAA' oder Het,
R⁹ H oder A',
R¹⁰, R¹¹ jeweils unabhängig voneinander H, A' oder OH,
Z Het, Ar oder A,
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, S, SO, SO₂, CO, COO, NR⁷, NR⁷CO, CONR⁷, CH=CH und/oder CH≡CH-Gruppen ersetzt sein können
oder
cyclisches Alkyl mit 3-7 C-Atomen,
Ar unsubstituiertes oder ein-, zwei-, drei-, vier- oder fünffach durch Hal, A, [C(R⁷R^{7'})]ₚOR⁷, [C(R⁷R^{7'})]ₚN(R⁷)₂, SR⁷, NO₂, CN, CHO, COOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R^{7'}), NHCOO[C(R⁷R^{7'})]ₚN(R⁷)₂, NHCOO[C(R⁷R^{7'})]ₚHet, NHCONH[C(R⁷R^{7'})]ₚN(R⁷)₂, NHCONH[C(R⁷R^{7'})]ₚHet, OCONH[C(R⁷R^{7'})]ₚN(R⁷)₂, OCONH[C(R⁷R⁷)]pHet, CONR⁷[C(R⁷R^{7'})]ₚN(R⁷)₂, CONR⁷[C(R⁷R⁷)]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet und/oder S(O)ₘ[C(R⁷R^{7'})]ₚAr' substituiertes Phenyl, Naphthyl oder Biphenyl,
Ar' unsubstituiertes oder ein-, zwei-, drei-, vier- oder fünffach durch Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA und/oder COA substituiertes Phenyl,
A' unverzweigtes oder verzweigtes Alkyl mit 1-8 C-Atomen, worin 1-7 H-Atome durch F, Cl und/oder Br ersetzt sein können,
Het einen ein-, zwei- oder dreikernigen gesättigten, ungesättigten oder aromatischen Heterocyclus mit 1 bis 4 N-, O- und/oder S-Atomen, der unsubstituiert oder
ein-, zwei- oder dreifach durch Hal, A, OR⁷, [C(R⁷R^{7'})]ₚAr, [C(R⁷R^{7'})]ₚHet', COA, CO[C(R⁷R^{7'})]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R^{7'}), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR⁷SO₂A, NR⁷SO₂Ar, NR⁷SO₂Het', SO₂N(R⁷)A, SO₂N(R⁷)Ar, SO₂N(R⁷)Het', CHO, =S, =NH, =NA, Oxy (-O⁻) und/oder =O (Carbonylsauerstoff) substituiert sein kann,
Het' einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
m 0, 1 oder 2,
n 1, 2 oder 3,
p 0, 1, 2, 3 oder 4,
bedeuten,
sowie ihre pharmazeutisch verwendbaren Salze, Tautomere und Stereoisomere, einschließlich deren Mischungen in allen Verhältnissen;
wobei Verbindungen der Formel I ausgeschlossen sind, worin
a) W = COCH₂Het bedeutet;
b) n = 2 und W = unsubstituiertes Alkyl mit 1-10 C-Atomen oder Cycloalkyl mit 3-7 C-Atomen bedeuten.

15. Verbindungen nach Anspruch 14, worin
R¹, R² jeweils unabhängig voneinander H oder A',
R³, R⁴ H,
R⁵, R⁶ jeweils unabhängig voneinander H oder A',
bedeuten,
sowie ihre pharmazeutisch verwendbaren Salze, Tautomere und Stereoisomere, einschließlich deren Mischungen in allen Verhältnissen.

16. Verbindungen nach Anspruch 14 oder 15, worin
Q H bedeutet,
sowie ihre pharmazeutisch verwendbaren Salze, Tautomere und Stereoisomere, einschließlich deren Mischungen in allen Verhältnissen.

17. Verbindungen nach einem oder mehreren der Ansprüche 14-16, worin
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA oder [C(R⁸R^{8'})]ₚNHCOOA,
bedeutet,
sowie ihre pharmazeutisch verwendbaren Salze, Tautomere und Stereoisomere, einschließlich deren Mischungen in allen Verhältnissen.

18. Verbindungen nach einem oder mehreren der Ansprüche 14-16, worin
R⁷, R^{7'} jeweils unabhängig voneinander H oder A',
R⁸, R^{8'} jeweils unabhängig voneinander H, A oder Het,
bedeuten,
sowie ihre pharmazeutisch verwendbaren Salze, Tautomere und Stereoisomere, einschließlich deren Mischungen in allen Verhältnissen.

19. Verbindungen nach einem oder mehreren der Ansprüche 14-18, worin
Z Het oder A bedeutet,
sowie ihre pharmazeutisch verwendbaren Salze, Tautomere und Stereoisomere, einschließlich deren Mischungen in allen Verhältnissen.

20. Verbindungen nach einem oder mehreren der Ansprüche 16-21, worin
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, SO₂, NR⁷, NR⁷CO und/oder CONR⁷-Gruppen ersetzt sein können,
bedeutet,
sowie ihre pharmazeutisch verwendbaren Salze, Tautomere und Stereoisomere, einschließlich deren Mischungen in allen Verhältnissen.

21. Verbindungen nach einem oder mehreren der Ansprüche 14-20, worin
Ar unsubstituiertes oder ein-, zwei-, drei-, vier- oder fünffach durch Hal und/oder [C(R⁷R^{7'})]ₚOR⁷ substituiertes Phenyl,
bedeutet,
sowie ihre pharmazeutisch verwendbaren Salze, Tautomere und Stereoisomere, einschließlich deren Mischungen in allen Verhältnissen.

22. Verbindungen nach einem oder mehreren der Ansprüche 14-21, worin
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann,
bedeutet,
sowie ihre pharmazeutisch verwendbaren Salze, Tautomere und Stereoisomere, einschließlich deren Mischungen in allen Verhältnissen.

23. Verbindungen nach einem oder mehreren der Ansprüche 14-22, worin
Het unsubstituiertes oder ein-, zwei- oder dreifach durch A, OR⁷, [C(R⁷R^{7'})]ₚAr, COOR⁷ und/oder =O
(Carbonylsauerstoff) substituiertes Pyrrolidinyl, Piperidinyl, Morpholinyl, Oxazolidinyl, Tetrahydrochinazolinyl, Piperazinyl, Thiazolyl, Furyl, Thienyl, Pyrrolyl, Imidazolyl, Pyrazolyl, Oxazolyl, Isoxazolyl, Isothiazolyl, Pyridyl, Pyrimidinyl, Triazolyl, Tetrazolyl, Oxadiazolyl oder Thiadiazolyl,
bedeutet, sowie ihre pharmazeutisch verwendbaren Salze, Tautomere und Stereoisomere, einschließlich deren Mischungen in allen Verhältnissen.

24. Verbindungen nach einem oder mehreren der Ansprüche 14-23, worin
R¹, R² jeweils unabhängig voneinander H oder A',
R³, R⁴ H,
R⁵, R⁶ jeweils unabhängig voneinander H oder A',
R⁷, R^{7'} jeweils unabhängig voneinander H oder A',
R⁸, R^{8'} jeweils unabhängig voneinander H, A oder Het,
R⁹ H oder A',
R¹⁰, R¹¹ jeweils unabhängig voneinander H, A' oder OH,
Q H,
W [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA oder [C(R⁸R^{8'})]ₚNHCOOA,
Z Het oder A,
A unverzweigtes oder verzweigtes Alkyl mit 1-10 C-Atomen, worin 1-7 H-Atome durch F, Cl, Br, OH und/oder OCH₃ ersetzt sein können,
und/oder worin eine oder zwei nicht-benachbarte CH₂-Gruppen durch O, NR⁷, SO₂, NR⁷CO und/oder CONR⁷-Gruppen ersetzt sein können,
A' unverzweigtes oder verzweigtes Alkyl mit 1-8 C-Atomen, worin 1-7 H-Atome durch F, Cl und/oder Br ersetzt sein können,
Ar unsubstituiertes oder ein-, zwei-, drei-, vier- oder fünffach durch Hal und/oder [C(R⁷R^{7'})]ₚOR⁷ substituiertes Phenyl,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OR⁷, [C(R⁷R^{7'})]ₚAr, COOR⁷ und/oder =O (Carbonylsauerstoff) substituiert sein kann,
n 1 oder 2,
p 0, 1, 2, 3 oder 4,
bedeuten,
sowie ihre pharmazeutisch verwendbaren Salze, Tautomere und Stereoisomere, einschließlich deren Mischungen in allen Verhältnissen;
wobei Verbindungen der Formel I ausgeschlossen sind, worin
a) W = COCH₂Het bedeutet;
b) n = 2 und W = unsubstituiertes Alkyl mit 1-10 C-Atomen oder Cycloalkyl mit 3-7 C-Atomen bedeuten.

25. Verbindungen nach Anspruch 14, ausgewählt aus der Gruppe
| Nr. | Struktur und/oder Name |
|---|---|
| "A1" | 4-(2-Methyl-3-{4-[4-(5,5,8,8-tetramethyl-5,6,7,8-tetrahydro-naphthalin-2-yl)-thiazol-2-yl]-piperidin-1-yl}-propyl)-morpholin |
| "A2" | 1'-Ethyl-4-[4-(5,5,8,8-tetramethyl-5,6,7,8-tetrahydro-naphthalin-2-yl)-thiazol-2-yl]-[1,3']bipiperidinyl |
| "A3" | 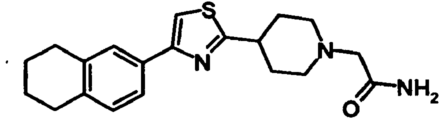 |
| "A4" | 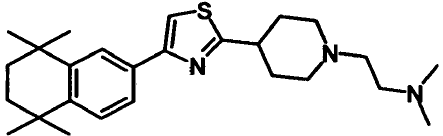 |
| "A5" | 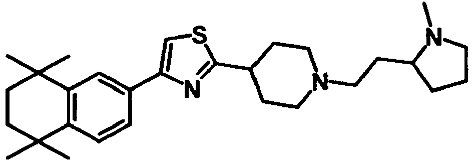 |
| "A6" | 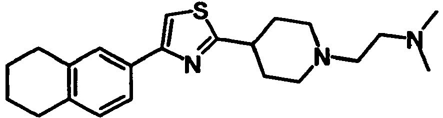 |
| "A7" | 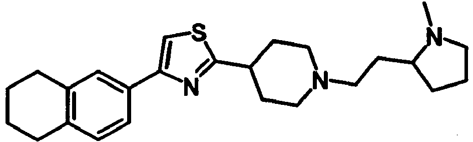 |
| "A8" | 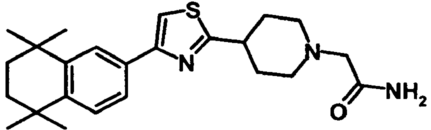 |
| "A9" | 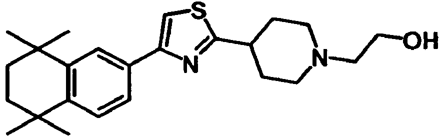 |
| "A10" | 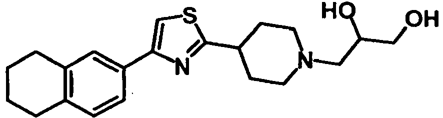 |
| "A11" | 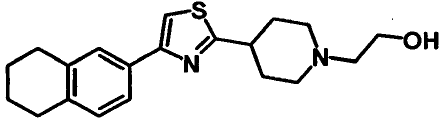 |
| "A12" | 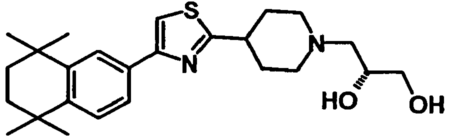 |
| "A13" | 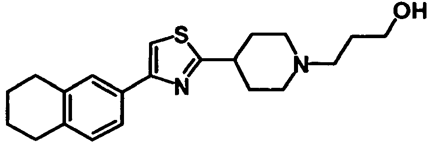 |
| "A14" | 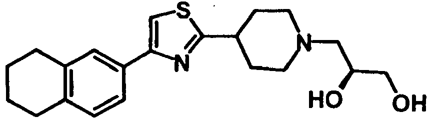 |
| "A15" | 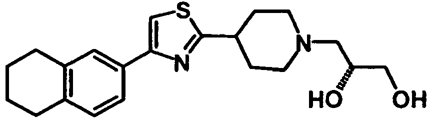 |
| "A16" | 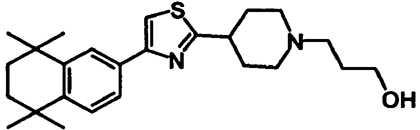 |
| "A17" | 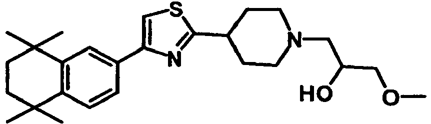 |
| "A18" | 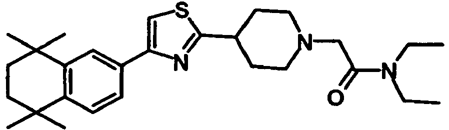 |
| "A19" | 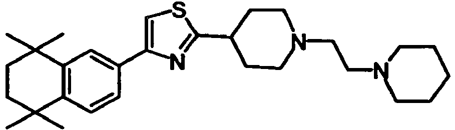 |
| "A20" | 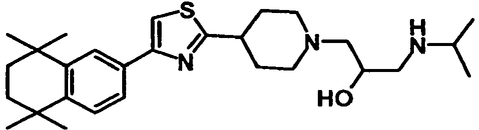 |
| "A21" | 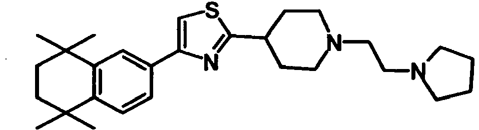 |
| "A22" | 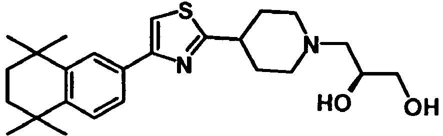 |
| "A23" | 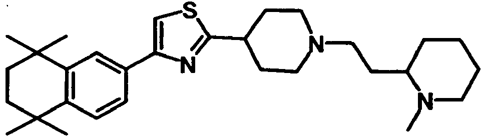 |
| "A24" | 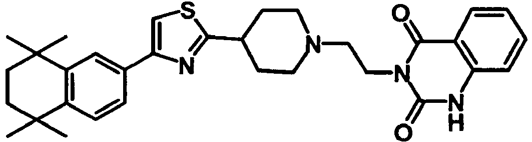 |
| "A25" | 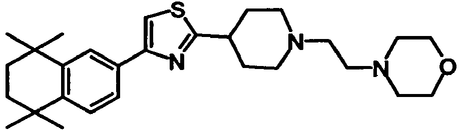 |
| "A26" | 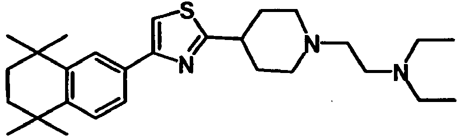 |
| "A27" | 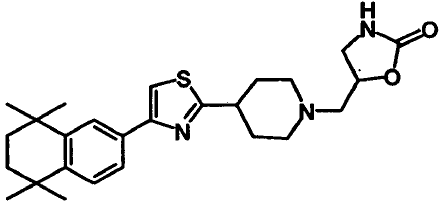 |
| "A28" | 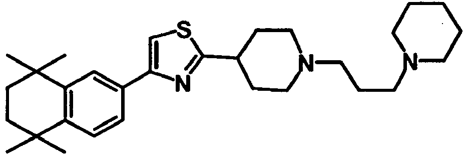 |
| "A29" | 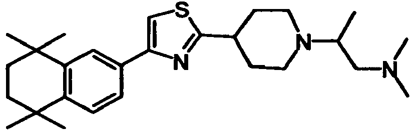 |
| "A30" | 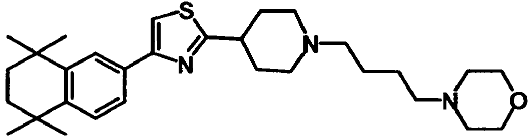 |
| "A31" | 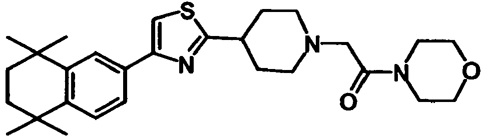 |
| "A32" | 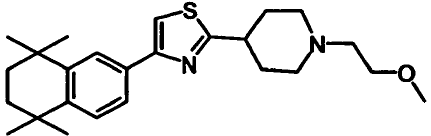 |
| "A33" | 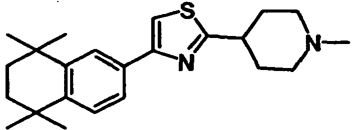 |
| "A34" | 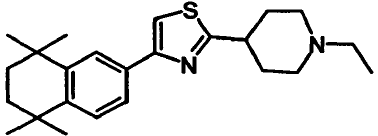 |
| "A35" | 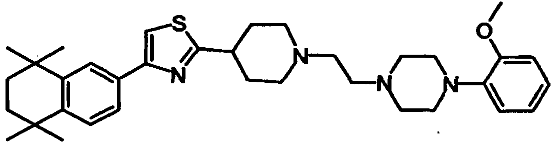 |
| "A36" | 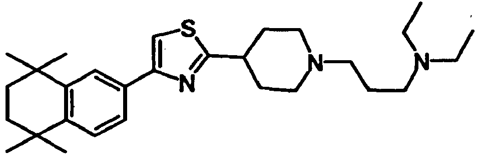 |
| "A37" | (2-{4-[4-(5,5,8,8-Tetramethyl-5,6,7,8-tetrahydro-naphthalin-2-yl)-thiazol-2-yl]-piperidin-1-yl}-ethyl)-carbaminsäure-*tert*.-butylester |
| "A38" | 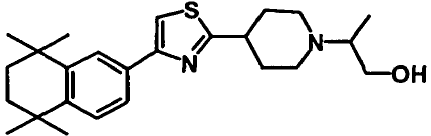 |
| "A39" | 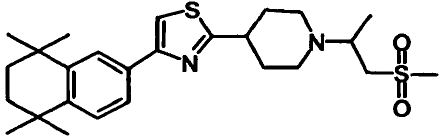 |
| "A40" | 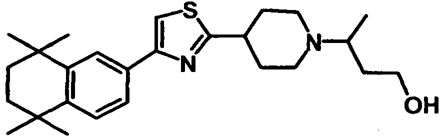 |
| "A41" | (2-{4-[4-(5,5,8,8-Tetramethyl-5,6,7,8-tetrahydro-naphthalin-2-yl)-thiazol-2-yl]-piperidin-1-yl}-ethyl)-carbaminsäure-*tert*.-butylester |
| "A42" | 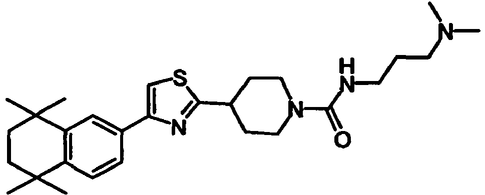 |
| "A43" | 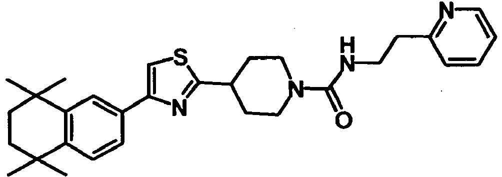 |
| "A44" | 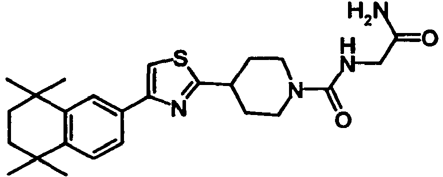 |
| "A45" | 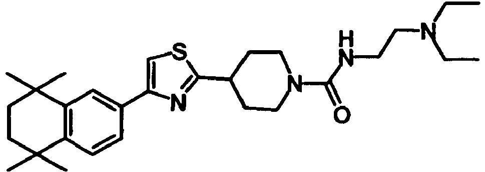 |
| "A46" | 4-[4-(5,5,8,8-Tetramethyl-5,6,7,8-tetrahydro-naphthalin-2-yl)-thiazol-2-yl]-piperidin-1-carbonsäure-3-methoxy-propylester |
| "A47" | 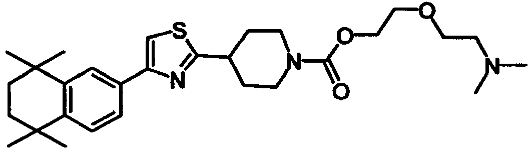 |
| "A48" | 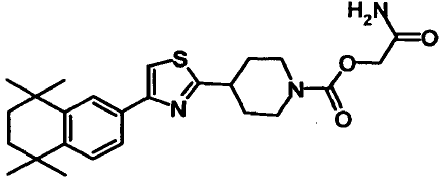 |
| "A49" | 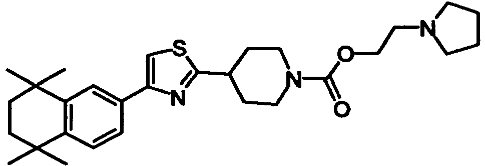 |
| "A50" | 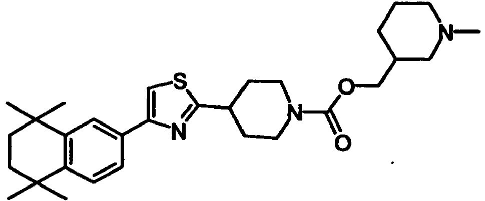 |
| "A51" | 2-Pyridin-4-yl-1-{4-[4-(5,5,8,8-tetramethyl-5,6,7,8-tetrahydro-naphthalin-2-yl)-thiazol-2-yl]-piperidin-1-yl}-ethanon |
| "A52" | 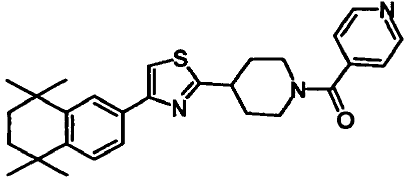 |
| "A53" | 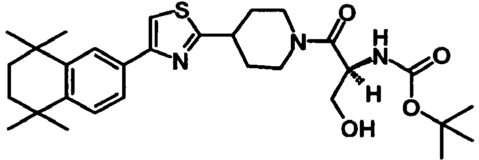 |
| "A54" | 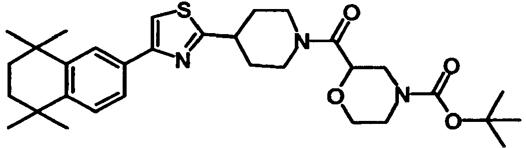 |
| "A55" | 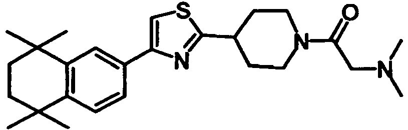 |
| "A56" | 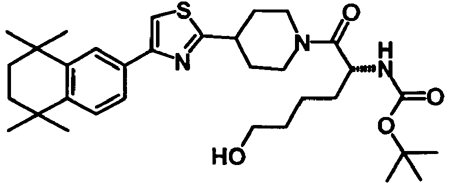 |
| "A57" | 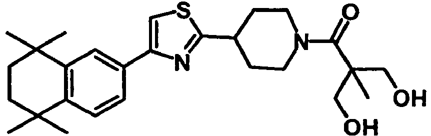 |
| "A58" | 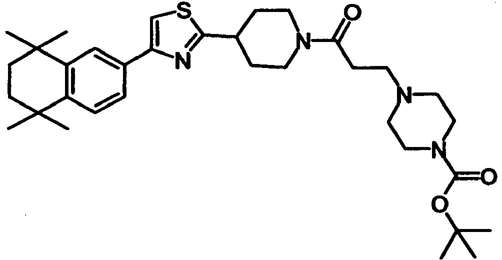 |
| "A59" | 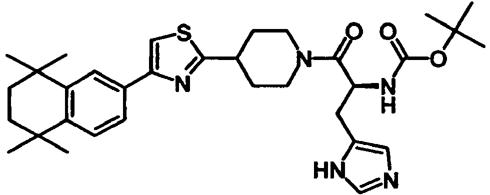 |
| "A60" | 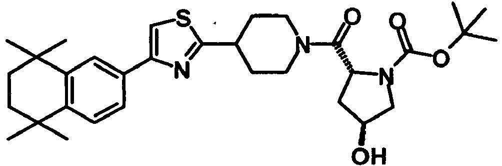 |
| "A61" | 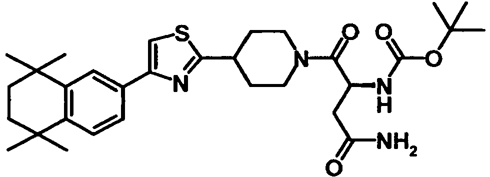 |
| "A62" | 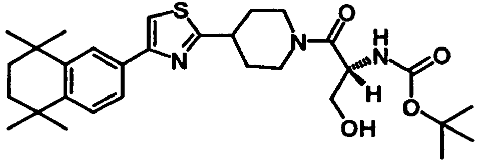 |
| "A63" | (S)-2-Amino-3-(3H-imidazol-4-yl)-1-{4-[4-(5,5,8,8-tetramethyl-5,6,7,8-tetrahydro-naphthalin-2-yl)-thiazol-2-yl]-piperidin-1-yl}-propan-1-on |
| "A64" | 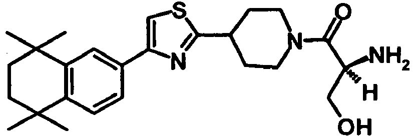 |
| "A65" | 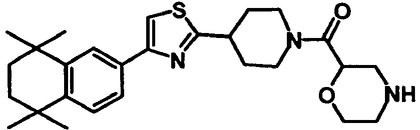 |
| "A66" | 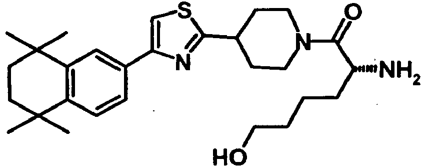 |
| "A67" | 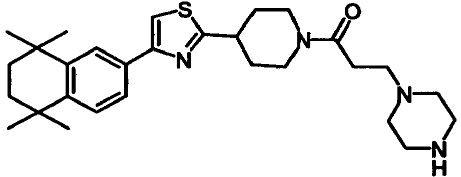 |
| "A68" | 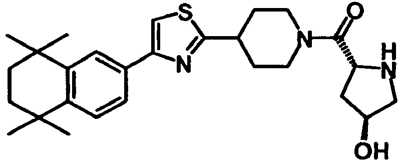 |
| "A69" | 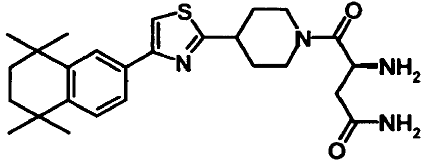 |
| "A70" | 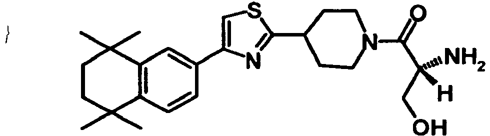 |
| "A71" | 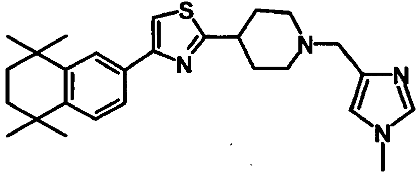 |
| "A72" | 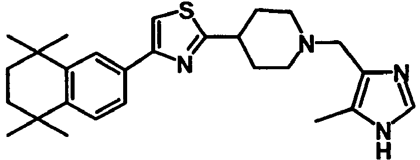 |
| "A73" | 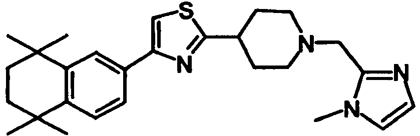 |
| "A74" | 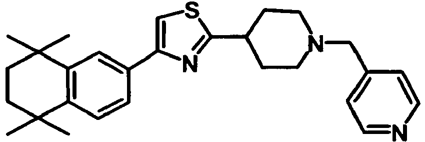 |
| "A75" | 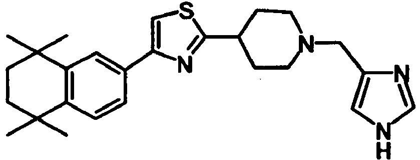 |
| "A76" | 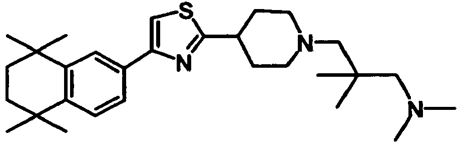 |
| "A77" | 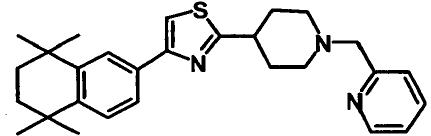 |
| "A78" | 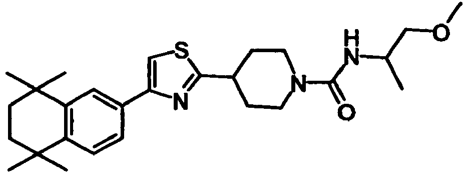 |
| "A79" | 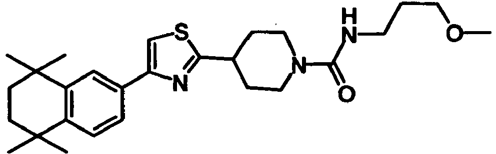 |
| "A80" | 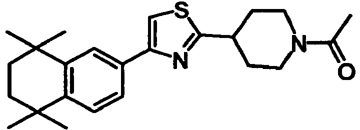 |
| "A81" | 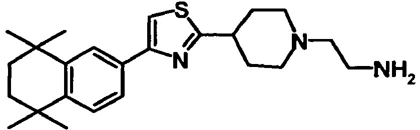 |
| "A82" | 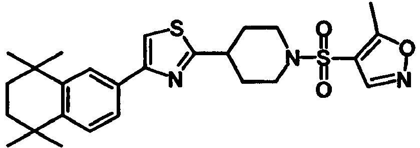 |
| "A83" | 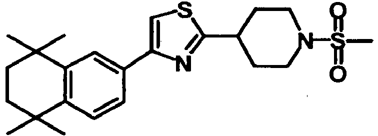 |
| "B1" | 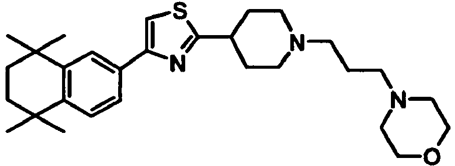 |
| "B2" | 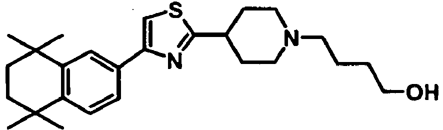 |
| "B3" | 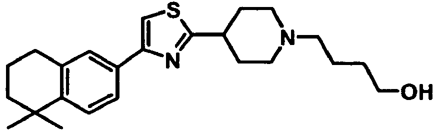 |
| "B4" | 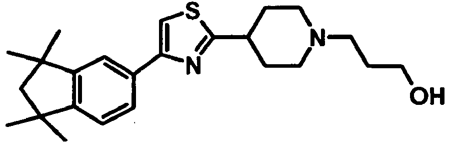 |
| "B5" | 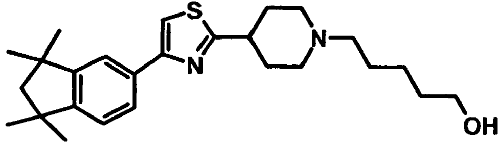 |
| "B6" | 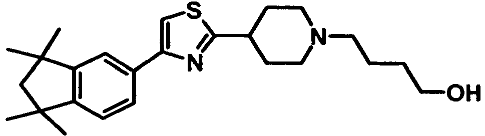 |
| "B7" | 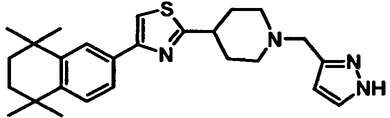 |
| "B8" | 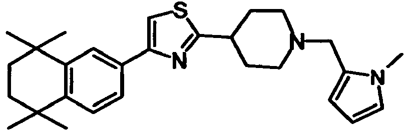 |
| "B9" | 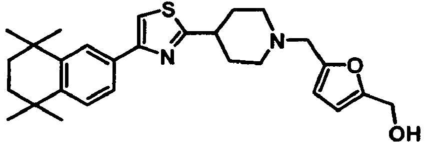 |
| "B10" | 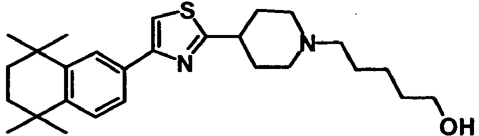 |
| "B11" | 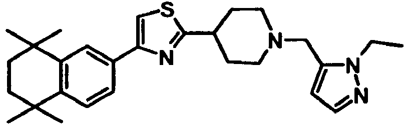 |
| "B12" | 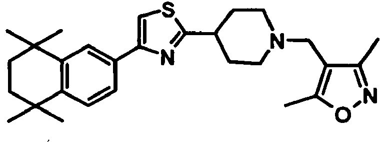 |
| "B13" | 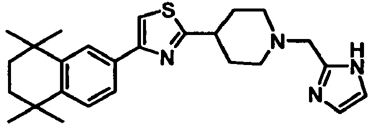 |
| "B14" | 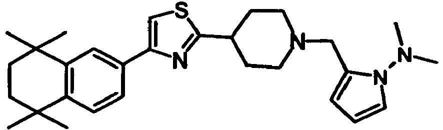 |
| "B15" | 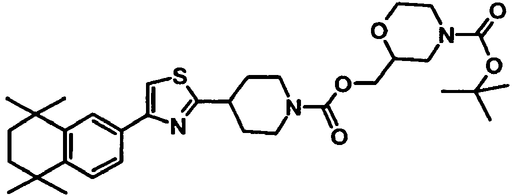 |
| "B16" | 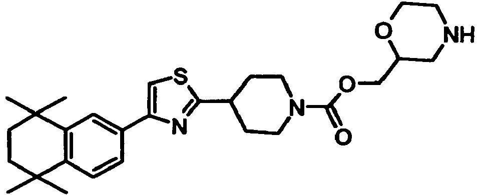 |
sowie ihre pharmazeutisch verwendbaren Salze, Tautomere und Stereoisomere, einschließlich deren Mischungen in allen Verhältnissen.

26. Verfahren zur Herstellung von Verbindungen der Formel I nach den Ansprüchen 14-25 sowie ihrer pharmazeutisch verwendbaren Salze, Tautomeren und Stereoisomeren, **dadurch gekennzeichnet, daß** man
a) eine Verbindung der Formel II 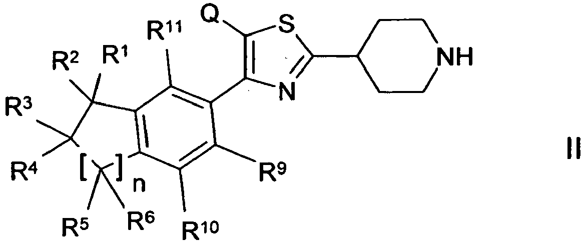 worin R¹, R², R³, R⁴, R⁵, R⁶, R⁹, R¹⁰, R¹¹, Q und n die in Anspruch 1 angegebenen Bedeutungen haben,
mit einer Verbindung der Formel III
L-W III,
worin W die in Anspruch 1 angegebene Bedeutung hat und
L Cl, Br, I oder eine freie oder reaktionsfähig funktionell abgewandelte OH-Gruppe bedeutet,
umsetzt,
oder
b) zur Herstellung von Verbindungen der Formel I, worin
W [C(R⁸R^{8'})]ₚZ oder [C(R⁸R^{8'})]ₚNHCOOA,
R⁸ H,
p 1, 2, 3 oder 4
bedeuten,
R^{8'}, Z, A die in Anspruch 1 angegebenen Bedeutungen haben,
eine Verbindung der Formel II
i) mit einer Verbindung der Formel IVa
R^{8'}-CO-[C(R⁸R^{8'})]ₚ₋₁Z IVa
worin
p 1, 2, 3 oder 4 bedeutet und
R^{8'}, Z die in Anspruch 1 angegebenen Bedeutungen haben,
in einer reduktiven Aminierung umsetzt,
oder
ii) mit einer Verbindung der Formel IVb
R^{8'}-CO-[C(R⁸R^{8'})]ₚ₋₁NHCOOA IVb
worin
p 1, 2, 3 oder 4 bedeutet und
R⁸, A die in Anspruch 1 angegebenen Bedeutungen haben,
in einer reduktiven Aminierung umsetzt,
oder
c) daß man sie aus einem ihrer funktionellen Derivate durch Behandeln mit einem solvolysierenden oder hydrogenolysierenden Mittel in Freiheit setzt,
und/oder
eine Base oder Säure der Formel I in eines ihrer Salze umwandelt.

27. Arzneimittel, enthaltend mindestens eine Verbindung der Formel I nach Anspruch 14-25 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

28. Arzneimittel enthaltend mindestens eine Verbindung der Formel I gemäß einem oder mehreren der Ansprüche 14 bis 25, und/oder ihre pharmazeutisch verwendbaren Salze und Stereoisomere, einschließlich deren Mischungen in allen Verhältnissen, und mindestens einen weiteren Arzneimittelwirkstoff.

29. Set (Kit), bestehend aus getrennten Packungen von
(a) einer wirksamen Menge an einer Verbindung der Formel I gemäß einem oder mehreren der Ansprüche 14 bis 25, und/oder ihrer pharmazeutisch verwendbaren Salze und Stereoisomere, einschließlich deren Mischungen in allen Verhältnissen,
und
(b) einer wirksamen Menge eines weiteren Arzneimittelswirkstoffs.

30. Verbindungen ausgewählt aus der Gruppe
| Nr. | Struktur und/oder Name |
|---|---|
| "C2" | 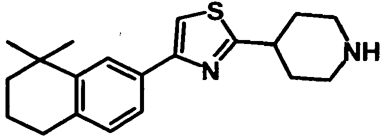 |
| "C3" | 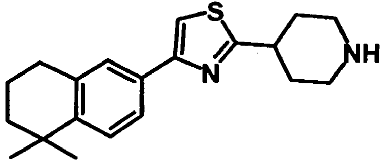 |
| "C4" | 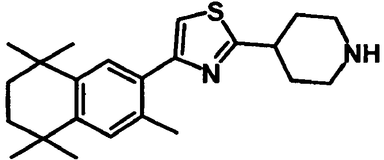 |
| "C6" | 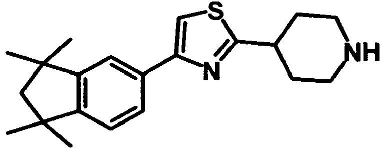 |
| "C7" | 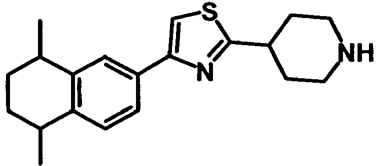 |
| "C8" | 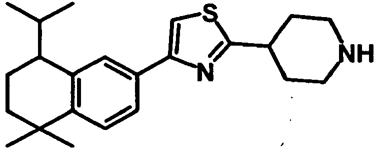 |
| "C9" | 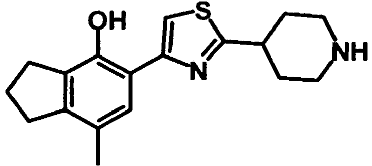 |
| "C10" | 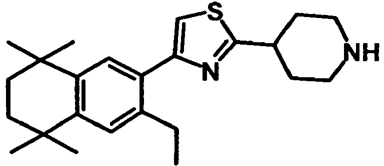 |
| "C11" | 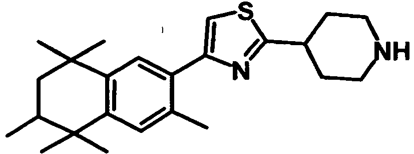 |
| "C12" | 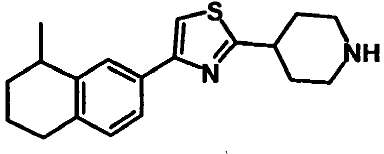 |
sowie ihre pharmazeutisch verwendbaren Salze, Tautomere und Stereoisomere, einschließlich deren Mischungen in allen Verhältnissen.

31. Verwendung von Verbindungen ausgewählt aus der Gruppe
| Nr. | Struktur und/oder Name |
|---|---|
| "C1" | 4-[4-(5,5,8,8-Tetramethyl-5,6,7,8-tetrahydronaphthalin-2-yl)-thiazol-2-yl]-piperidin |
| "C2" | 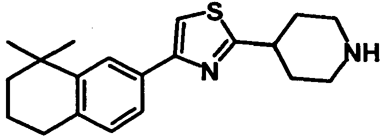 |
| "C3" | 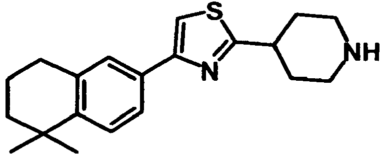 |
| "C4" | 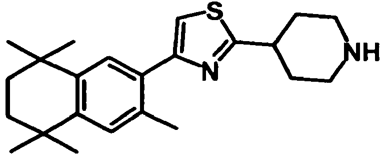 |
| "C5" | 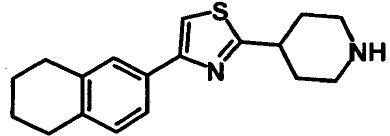 |
| "C6" | 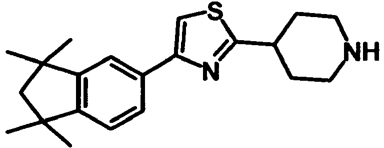 |
| "C7" | 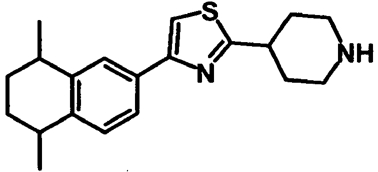 |
| "C8" | 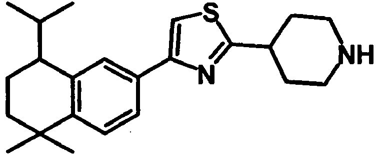 |
| "C9" | 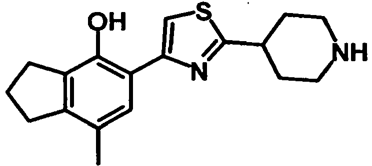 |
| "C10" | 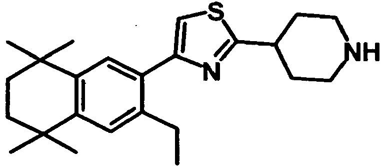 |
| "C11" | 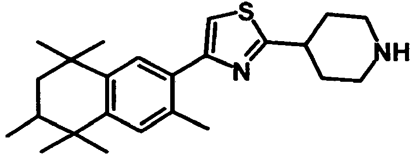 |
| "C12" | 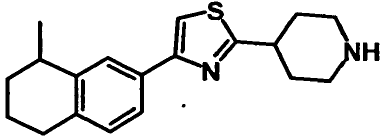 |
sowie ihre pharmazeutisch verwendbaren Salze, Tautomere und Stereoisomere, einschließlich deren Mischungen in allen Verhältnissen, zur Herstellung eines Arzneimittels zur Behandlung von Krankheiten,
wobei die zu behandelnden Krankheiten ausgewählt sind aus der Gruppe
hyperproliferative Erkrankung, inflammatorische Erkrankung, angiogene Erkrankung, fibrotische Erkrankung der Lunge, Niere, Leber und des Herzens.

32. Verwendung nach Anspruch 31, wobei die hyperproliferative Erkrankung ausgewählt ist aus der Gruppe
Krebs (Tumorerkrankung), Atherosclerose, Restenose, proliferative Erkrankung der Mesangiumzellen, Psoriasis.

33. Verwendung nach Anspruch 32, wobei die Tumorerkrankung ausgewählt ist aus der Gruppe
Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

34. Verwendung nach Anspruch 32, wobei die proliferative Erkrankung der Mesangiumzellen ausgewählt ist aus der Gruppe
Glomerulonephritis, diabetische Nephropathie, maligne Nephrosclerose, thrombotisches Mikroangiopathie-Syndrom, Transplantatabstossung, Glomerulopathie.

35. Verwendung nach Anspruch 31, wobei die inflammatorische Erkrankung ausgewählt ist aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis, durch T-Zellen - vermittelte Immunerkrankung.

36. Verwendung nach Anspruch 35, wobei die entzündliche Darmerkrankung ausgewählt ist aus der Gruppe
ulcerative Colitis, Morbus Crohn, unbestimmte Colitis.

37. Verwendung nach Anspruch 35, wobei die durch T-Zellen - vermittelte Immunerkrankung ausgewählt ist aus der Gruppe
allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

38. Verwendung nach Anspruch 35, wobei die Arthritis-Erkrankung ausgewählt ist aus der Gruppe
rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

39. Verwendung nach Anspruch 35, wobei die entzündliche Nierenerkrankung ausgewählt ist aus der Gruppe
Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

40. Verwendung nach Anspruch 35, wobei die entzündliche Hauterkrankung ausgewählt ist aus der Gruppe
Psoriasis, atopische Dermatitis, Kontaktempfindlichkeit, Akne.

41. Verwendung nach Anspruch 31, wobei die angiogene Erkrankung ausgewählt ist aus der Gruppe
diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

42. Arzneimittel, enthaltend mindestens eine Verbindung ausgewählt aus der Gruppe
| Nr. | Struktur und/oder Name |
|---|---|
| "C2" | 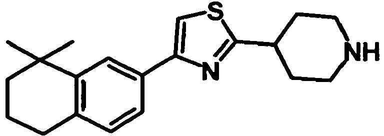 |
| "C3" | 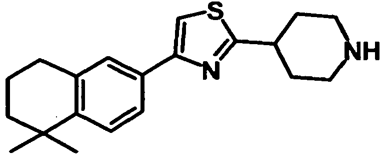 |
| "C4" | 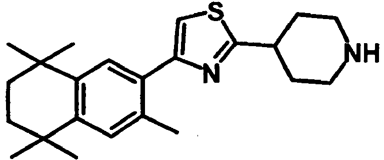 |
| "C6" | 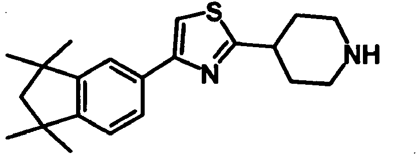 |
| "C7" | 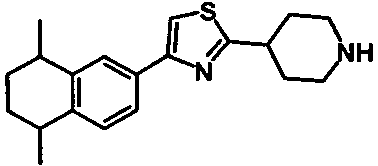 |
| "C8" | 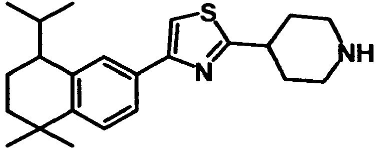 |
| "C9" | 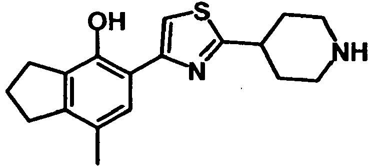 |
| "C10" | 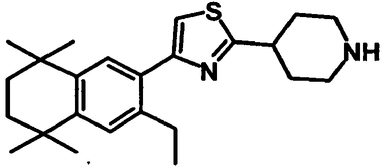 |
| "C11" | 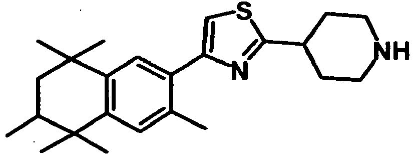 |
| "C12" | 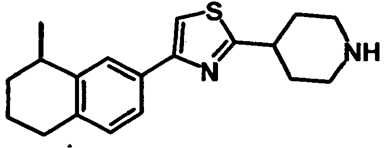 |
und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

## Claims

1. Use of compounds of the formula I 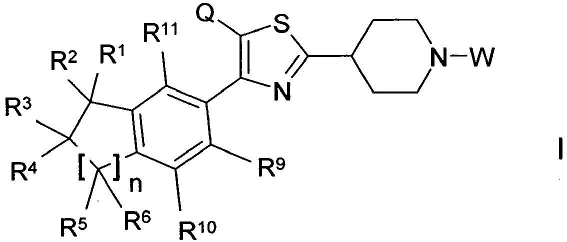 in which
R¹, R², R³, R⁴, R⁵, R⁶ each, independently of one another, denote H or A',
Q denotes H, A, Hal, COOR⁷ or CON(R⁷R⁷'),
W denotes [C(R⁸R⁸')]ₚZ, CO-[C(R⁸R8^{'})]ₚZ, CO-N(R⁸)-[C(R⁸R⁸)]ₚZ, CO-O-[C(R⁸R^{8'})]ₚZ, SO₂-[C(R⁸R^{8'})]ₚZ, CO[C(R⁸R^{8'})]ₚNHCOOA or [C(R⁸R^{8'})]ₚNHCOOA,
R⁷, R^{7'} each, independently of one another, denote H or A',
R⁸, R^{8'} each, independently of one another, denote H, A, OA, NAA' or Het,
R⁹ denotes H or A',
R¹⁰, R¹¹ each, independently of one another, denote H, A' or OH,
Z denotes Het, Ar or A,
A denotes unbranched or branched alkyl having 1-10 C atoms, in which 1-7 H atoms may be replaced by F, Cl, Br, OH and/or OCH₃,
and/or in which one or two non-adjacent CH₂ groups may be replaced by O, S, SO, SO₂, CO, COO, NR⁷, NR⁷CO, CONR⁷, CH=CH and/or CH≡CH groups
or
cyclic alkyl having 3-7 C atoms,
Ar denotes phenyl, naphthyl or biphenyl, each of which is unsubstituted or mono-, di-, tri-, tetra- or pentasubstituted by Hal, A, [C(R⁷R⁷')]ₚOR⁷, [C(R⁷R^{7'})]ₚN(R⁷)₂, SR⁷, NO₂, CN, CHO, COOR⁷, CON(R⁷R⁷'), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R⁷), NHCOO[C(R⁷R^{7'})]ₚN(R⁷)₂, NHCOO[C(R⁷R^{7'})]ₚHet, NHCONH[C(R⁷R^{7'})]ₚN(R⁷)₂, NHCONH[C(R⁷R^{7'})]ₚHet, OCONH[C(R⁷R^{7'})]ₚN(R⁷)₂, OCONH[C(R⁷R^{7'})]ₚHet, CONR⁷[C(R⁷R^{7'})]ₚN(R⁷)₂, CONR⁷[C(R⁷R^{7'})]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet and/or S(O)ₘ[C(R⁷R^{7'})]ₚAr',
Ar' denotes phenyl which is unsubstituted or mono-, di-, tri-, tetra- or pentasubstituted by Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA and/or COA,
A' denotes unbranched or branched alkyl having 1-8 C atoms, in which 1-7 H atoms may be replaced by F, Cl and/or Br,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OR⁷, [C(R⁷R^{7'})]ₚAr, [C(R⁷R⁷)]ₚHet', COA, CO[C(R⁷R⁷)]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R⁷'), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR⁷SO₂A, NR⁷SO₂Ar, NR⁷SO₂Het', SO₂N(R⁷)A, SO₂N(R⁷)Ar, SO₂N(R⁷)Het', CHO, =S, =NH, =NA, oxy (-O-) and/or =O (carbonyl oxygen),
Het' denotes a monocyclic saturated heterocycle having 1 to 2 N and/or O atoms, which may be mono- or disubstituted by A, OA, OH, Hal and/or =O (carbonyl oxygen),
m denotes 0, 1 or 2,
n denotes 1, 2 or 3,
p denotes 0, 1, 2, 3 or 4,
and pharmaceutically usable salts, tautomers and stereoisomers thereof, including mixtures thereof in all ratios,
for the preparation of a medicament for the treatment of diseases, where the diseases to be treated are selected from the group hyperproliferative disease, inflammatory disease, angiogenic disease, fibrotic disease of the lung, kidney, liver and heart.

2. Use according to Claim 1, where the hyperproliferative disease is selected from the group
cancer (tumour disease), atherosclerosis, restenosis, proliferative disease of the mesangial cells, psoriasis.

3. Use according to Claim 2, where the tumour disease is selected from the group
tumour of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

4. Use according to Claim 2, where the proliferative disease of the mesangial cells is selected from the group
glomerulonephritis, diabetic nephropathy, malignant nephrosclerosis, thrombotic microangiopathy syndrome, transplant rejection, glomerulopathy.

5. Use according to Claim 1, where the inflammatory disease is selected from the group
inflammatory bowel disease, arthritis, atherosclersosis, asthma, allergies, inflammatory kidney diseases, multiple sclerosis, chronic obstructive pulmonary disease, inflammatory skin diseases, pardontal diseases, psoriasis, T-cell-promoted immune disease.

6. Use according to Claim 5, where the inflammatory bowel disease is selected from the group
ulcerative colitis, Crohn's disease, non-specific colitis.

7. Use according to Claim 5, where the T-cell-promoted immune disease is selected from the group
allergic encephalomyelitis, allergic neuritis, transplant rejection, graft-versus-host reaction, myocarditis, thyroiditis, nephritis, systemic lupus erythematosus, insulin-dependent diabetes mellitus.

8. Use according to Claim 5, where the arthritis disease is selected from the group
rheumatoid arthritis, osteoarthritis, Caplan's syndrome, Felty's syndrome, Sjogren's syndrome, spondylitis ankylosans, Still's disease, chondrocalcinosis, metabolic arthritis, rheumatic fever, Reiter's disease, Wissler`s syndrome.

9. Use according to Claim 5, where the inflammatory kidney disease is selected from the group
glomerulonephritis, glomerular injury, nephrotic syndrome, interstitial nephritis, lupus nephritis, Goodpasture's syndrome, Wegener's granulo-matosis, renal vasculitis, IgA nephropathy, idiopathic glomerular disease.

10. Use according to Claim 5, where the inflammatory skin disease is selected from the group
psoriasis, atopic dermatitis, contact sensitivity, acne.

11. Use according to Claim 1, where the angiogenic disease is selected from the group
diabetic retinopathy, arthritis, cancer, psoriasis, Kaposi's sarcoma, haemangioma, myocardial angiogenesis, atherosclerotic plaque neovascularisation, angiogenic eye diseases, choroidal neovascularisation, retrolental fibroplasia, macular degeneration, corneal transplant rejection, rubeosis iridis, neuroscular glaucoma, Oster Webber syndrome.

12. Use according to one or more of Claims 1-11
of compounds of the formula I
in which
R¹, R² each, independently of one another, denote H or A',
R³, R⁴ denote H,
R⁵, R⁶ each, independently of one another, denote H or A',
R⁷, R^{7'} each, independently of one another, denote H or A',
R⁸, R^{8'} each, independently of one another, denote H, A or Het,
R⁹ denotes H or A',
R¹⁰, R¹¹ each, independently of one another, denote H, A' or OH,
Q denotes H,
W denotes [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA or [C(R⁸R^{8'})]ₚNHCOOA,
Z denotes Het or A,
A denotes unbranched or branched alkyl having 1-10 C atoms, in which 1-7 H atoms may be replaced by F, Cl, Br, OH and/or OCH₃,
and/or in which one or two non-adjacent CH₂ groups may be replaced by O, NR⁷, SO₂, NR⁷CO and/or CONR⁷ groups,
A' denotes unbranched or branched alkyl having 1-8 C atoms, in which 1-7 H atoms may be replaced by F, Cl and/or Br,
Ar denotes phenyl which is unsubstituted or mono-, di-, tri-, tetra- or pentasubstituted by Hal and/or [C(R⁷R^{7'})]ₚOR⁷,
Het denotes a mono- or bicyclic saturated, unsaturated or aromatic heterocycle having 1 to 4 N, O and/or S atoms, which may be unsubstituted or mono-, di- or trisubstituted by A, OR⁷, [C(R⁷R^{7'})]ₚAr, COOR⁷ and/or =O (carbonyl oxygen),
n denotes 1 or 2,
p denotes 0, 1, 2, 3 or 4,
and pharmaceutically usable salts, tautomers and stereoisomers thereof, including mixtures thereof in all ratios.

13. Use according to one or more of Claims 1-12
of compounds of the formula I selected from the group
| No. | Structure and/or name |
|---|---|
| "A1" | 4-(2-Methyl-3-{4-[4-(5,5,8,8-tetramethyl-5,6,7,8-tetrahydronaphthalen-2-yl)thiazol-2-yl]piperidin-1-yl}propyl)-morpholine |
| "A2" | 1'-Ethyl-4-[4-(5,5,8,8-tetramethyl-5,6,7,8-tetrahydronaphthalen-2-yl)thiazol-2-yl]-[1,3']bipiperidinyl |
| "A3" | 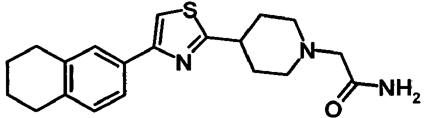 |
| "A4" | 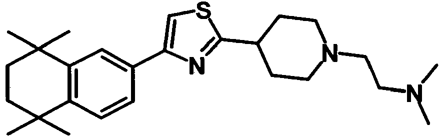 |
| "A5" | 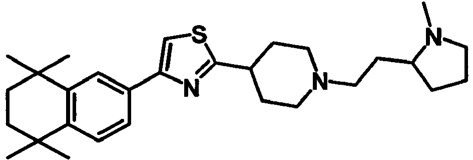 |
| "A6" | 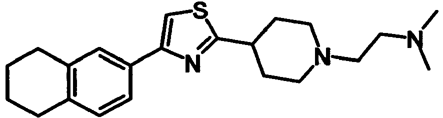 |
| "A7" | 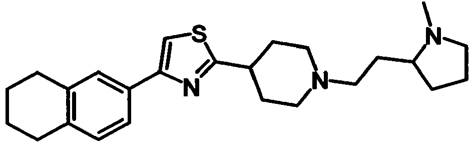 |
| "A8" | 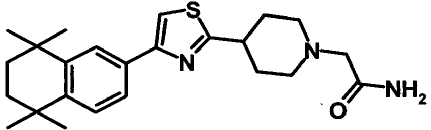 |
| "A9" | 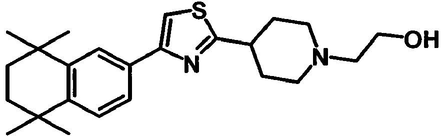 |
| "A10" | 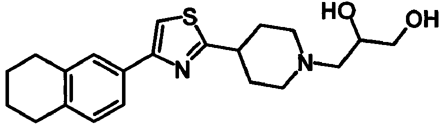 |
| "A11" | 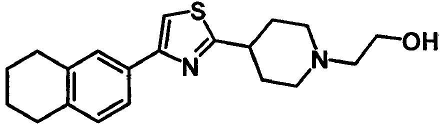 |
| "A12" | 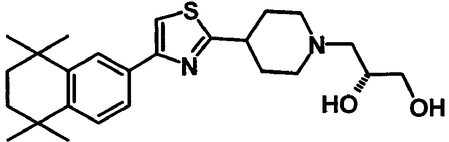 |
| "A13" | 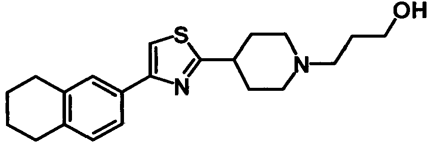 |
| "A14" | 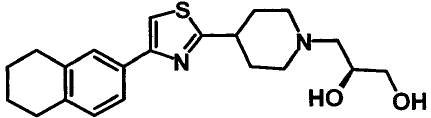 |
| "A15" | 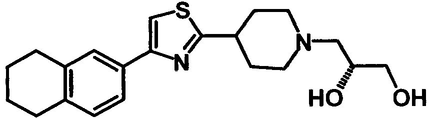 |
| "A16" | 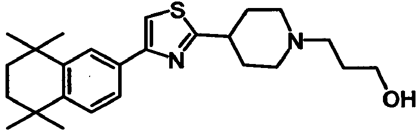 |
| "A17" | 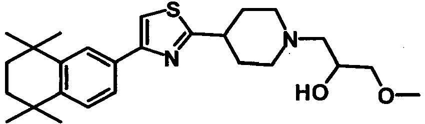 |
| "A18" | 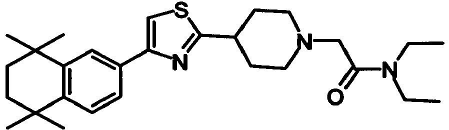 |
| "A19" | 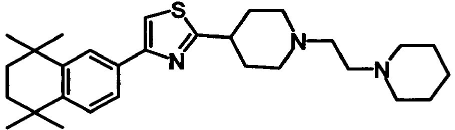 |
| "A20" | 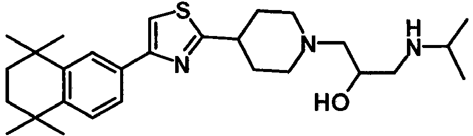 |
| "A21" | 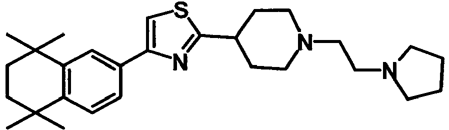 |
| "A22" | 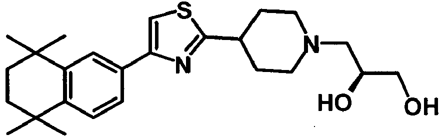 |
| "A23" | 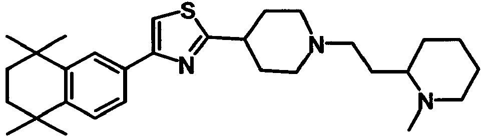 |
| "A24" | 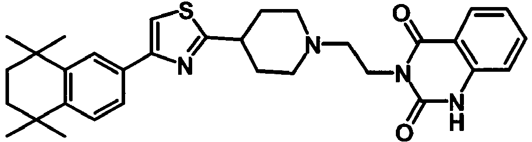 |
| "A25" | 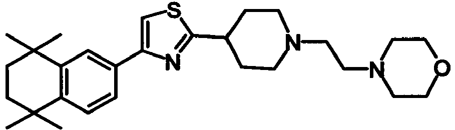 |
| "A26" | 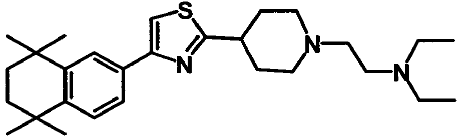 |
| "A27" | 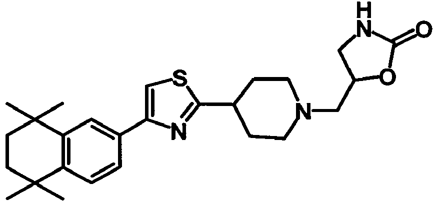 |
| "A28" | 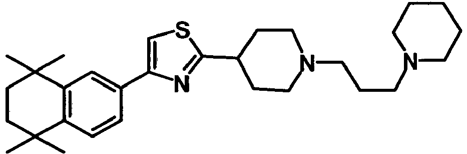 |
| "A29" | 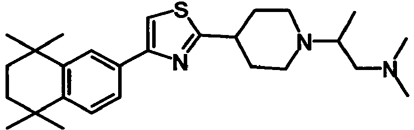 |
| "A30" | 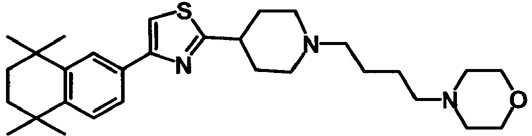 |
| "A31" | 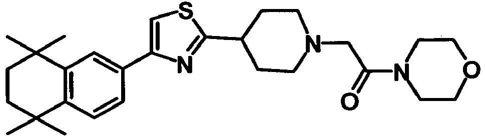 |
| "A32" | 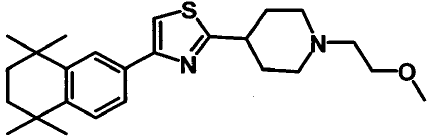 |
| "A33" | 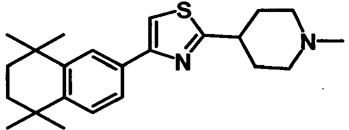 |
| "A34" | 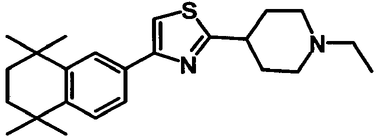 |
| "A35" | 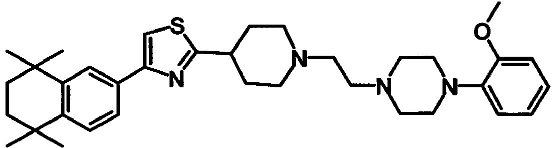 |
| "A36" | 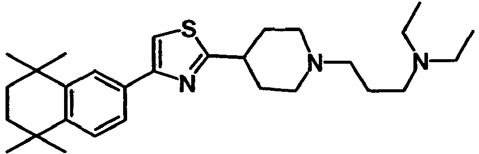 |
| "A37" | *tert-Butyl* (2-{4-[4-(5,5,8,8-tetramethyl-5,6,7,8-tetrahydronaphthalen-2-yl)thiazol-2-yl]piperidin-1-yl}ethyl)carbamate |
| "A38" | 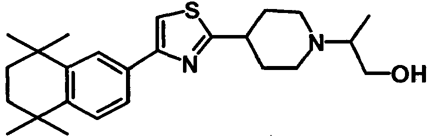 |
| "A39" | 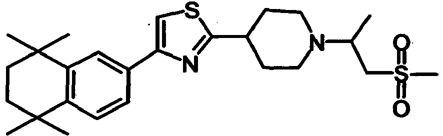 |
| "A40" | 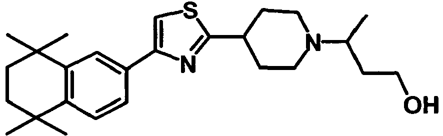 |
| "A41" | *tert*-Butyl (2-{4-[4-(5,5,8,8-tetramethyl-5,6,7,8-tetrahydronaphthalen-2-yl)thiazol-2-yl]piperidin-1-yl}ethyl)carbamate |
| "A42" | 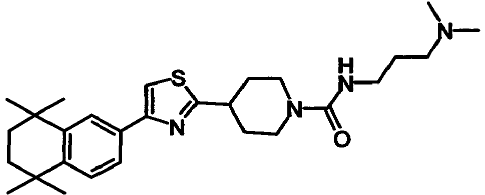 |
| "A43" | 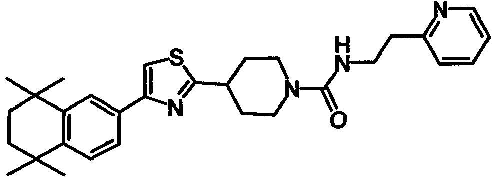 |
| "A44" | 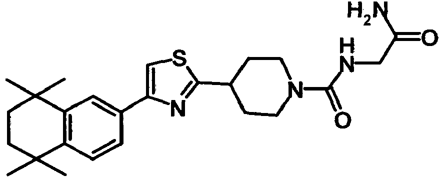 |
| "A45" | 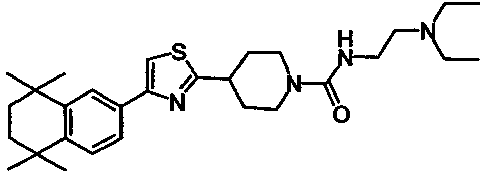 |
| "A46" | 3-Methoxypropyl 4-[4-(5,5,8,8-tetramethyl-5,6,7,8-tetrahydronaphthalen-2-yl)thiazol-2-yl]piperidine-1-carboxylate |
| "A47" | 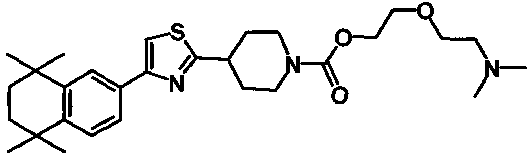 |
| "A48" | 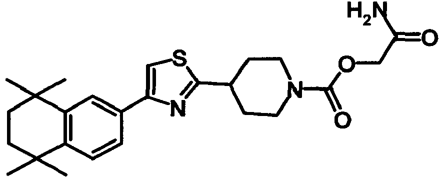 |
| "A49" | 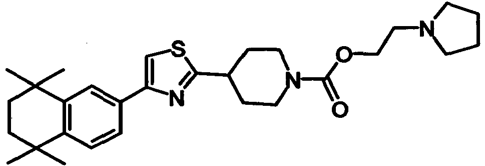 |
| "A50" | 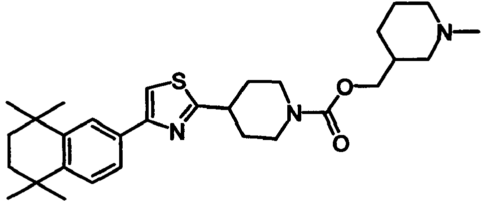 |
| "A51" | 2-Pyridin-4-yl-1-{4-[4-(5,5,8,8-tetramethyl-5,6,7,8-tetrahydronaphthalen-2-yl)thiazol-2-yl]piperidin-1-yl}-ethanone |
| "A52" | 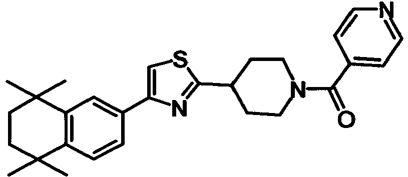 |
| "A53" | 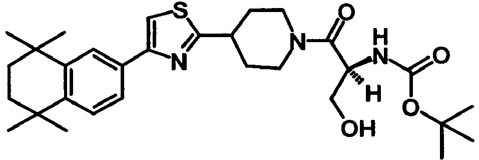 |
| "A54" | 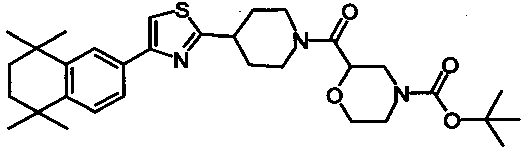 |
| "A55" | 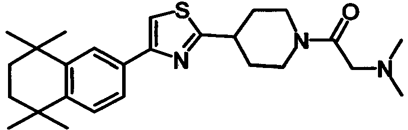 |
| "A56" | 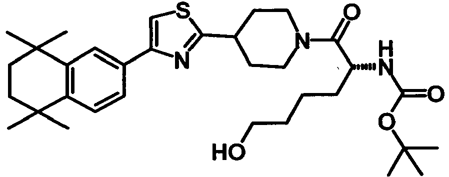 |
| "A57" | 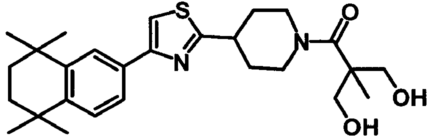 |
| "A58" | 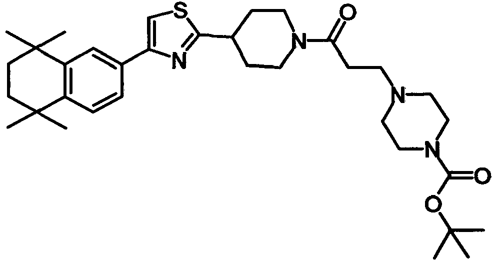 |
| "A59" | 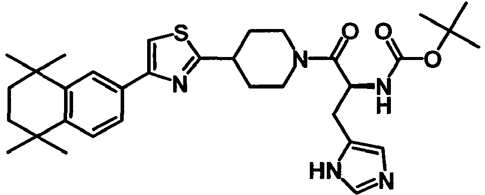 |
| "A60" | 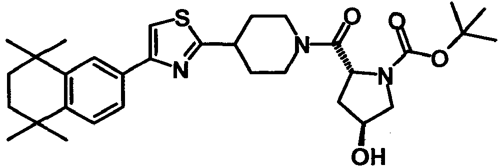 |
| "A61" | 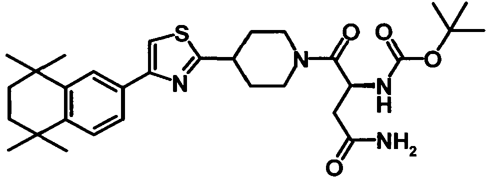 |
| "A62" | 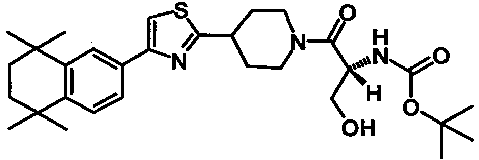 |
| "A63" | (S)-2-Amino-3-(3H-imidazol-4-yl)-1-{4-[4-(5,5,8,8-tetramethyl-5,6,7,8-tetrahydronaphthalen-2-yl)thiazol-2-yl]-piperidin-1-yl}propan-1-one |
| "A64" | 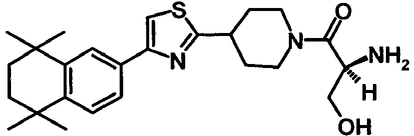 |
| "A65" | 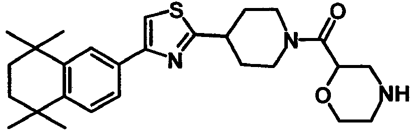 |
| "A66" | 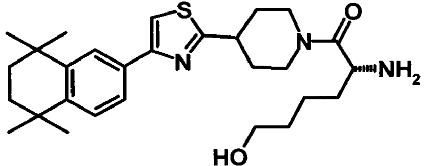 |
| "A67" | 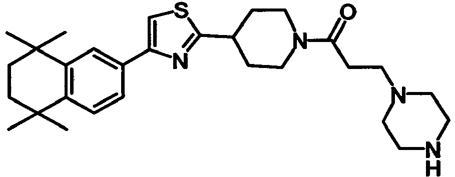 |
| "A68" | 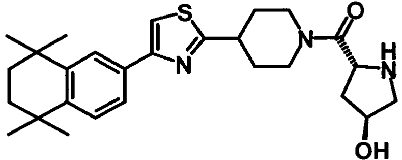 |
| "A69" | 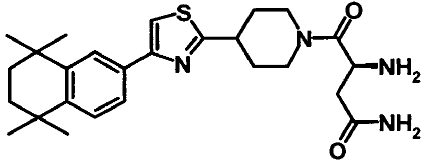 |
| "A70" | 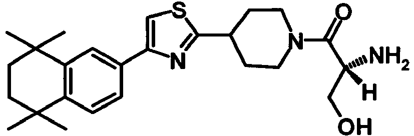 |
| "A71" | 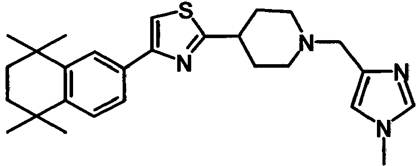 |
| "A72" | 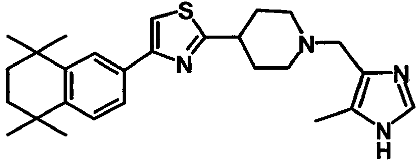 |
| "A73" | 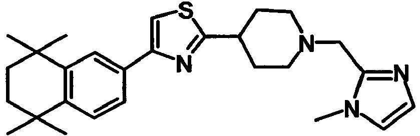 |
| "A74" | 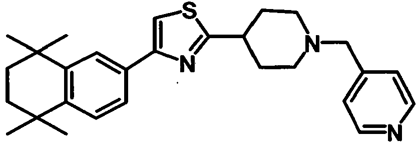 |
| "A75" | 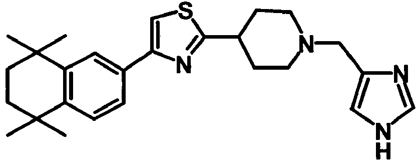 |
| "A76" | 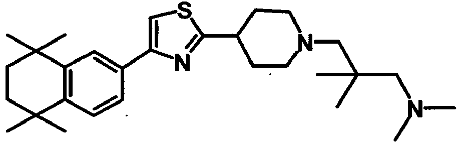 |
| "A77" | 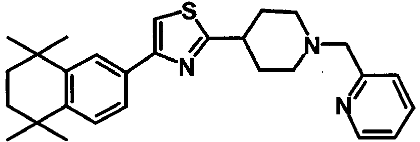 |
| "A78" | 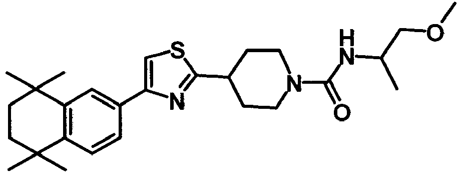 |
| "A79" | 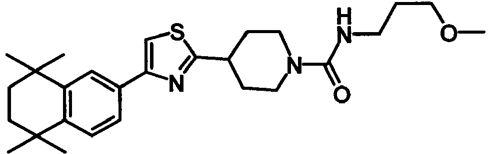 |
| "A80" | 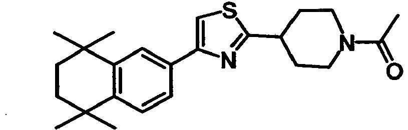 |
| "A81" | 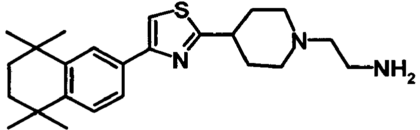 |
| "A82" | 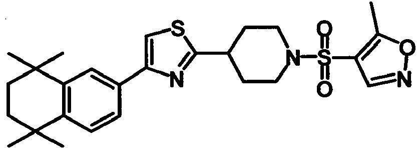 |
| "A83" | 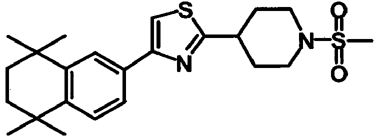 |
| "B1" | 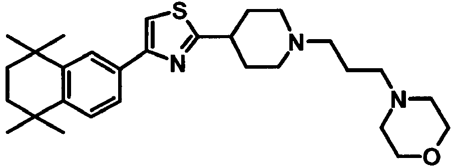 |
| "B2" | 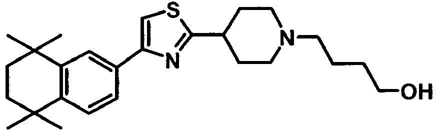 |
| "B3" | 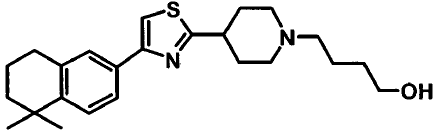 |
| "B4" | 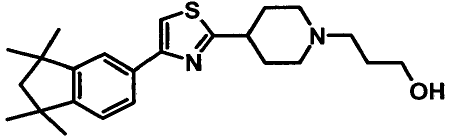 |
| "B5" | 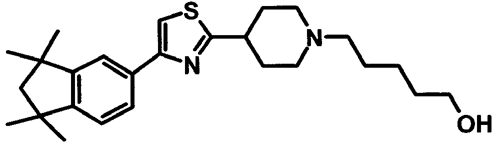 |
| "B6" | 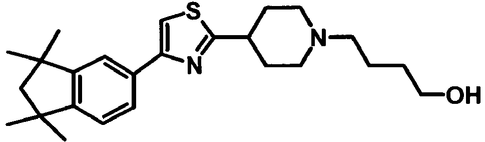 |
| "B7" | 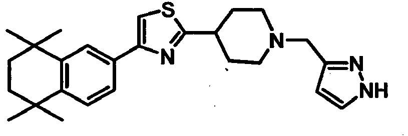 |
| "B8" | 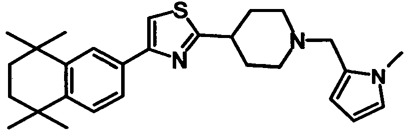 |
| "B9" | 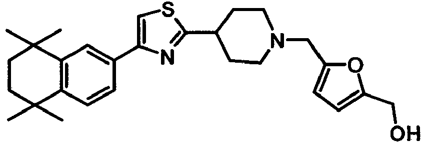 |
| "B10" | 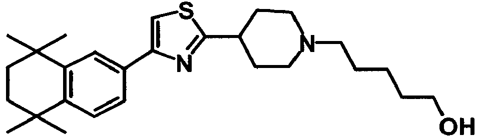 |
| "B11" | 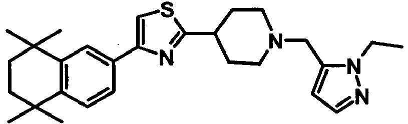 |
| "B12" | 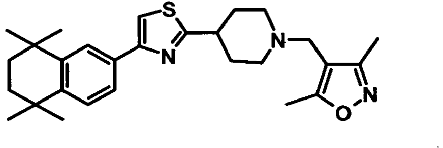 |
| "B13" | 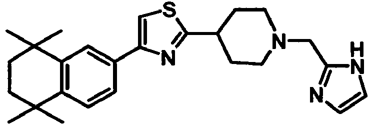 |
| "B14" | 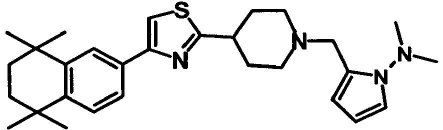 |
| "B15" | 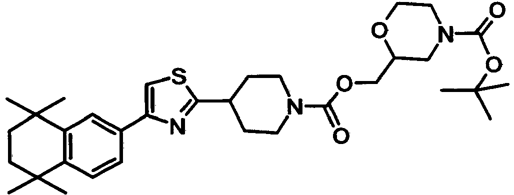 |
| "B16" | 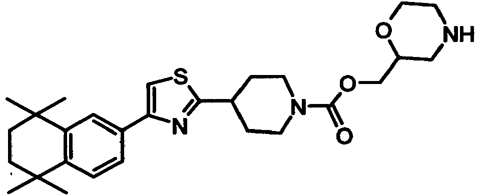 |
and pharmaceutically usable salts, tautomers and stereoisomers thereof, including mixtures thereof in all ratios.

14. Compounds of the formula I 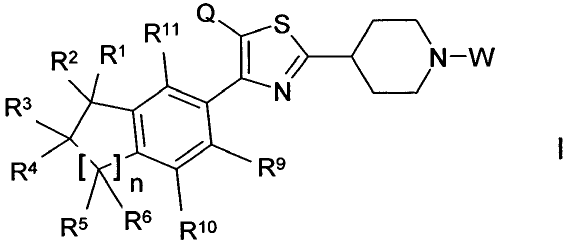 in which
R¹, R², R³, R⁴, R⁵, R⁶ each, independently of one another, denote H or A',
Q denotes H, A, Hal, COOR⁷ or CON(R⁷R^{7'}),
W denotes [C(R⁸R^{8'})]ₚZ, CO-[C(R⁸R^{8'})]ₚZ, CO-N(R⁸)-[C(R⁸R⁸)]ₚZ, CO-O-[C(R⁸R^{8'})]ₚZ, S02-[C(R⁸R^{8'})]ₚZ, CO[C(R⁸R^{8'})]ₚNHCOOA or [C(R⁸R^{8'})]ₚNHCOOA,
R⁷, R^{7'} each, independently of one another, denote H or A',
R⁸, R^{8'} each, independently of one another, denote H, A, OA, NAA' or Het,
R⁹ denotes H or A',
R¹⁰, R¹¹ each, independently of one another, denote H, A' or OH,
Z denotes Het, Ar or A,
A denotes unbranched or branched alkyl having 1-10 C atoms, in which 1-7 H atoms may be replaced by F, Cl, Br, OH and/or OCH₃,
and/or in which one or two non-adjacent CH₂ groups may be replaced by O, S, SO, SO₂, CO, COO, NR⁷, NR⁷CO, CONR⁷, CH=CH and/or CH≡CH groups
or
cyclic alkyl having 3-7 C atoms,
Ar denotes phenyl, naphthyl or biphenyl, each of which is unsubstituted or mono-, di-, tri-, tetra- or pentasubstituted by Hal, A, [C(R⁷R^{7'})]ₚOR⁷, [C(R⁷R^{7'})]ₚN(R⁷)₂, SR⁷, NO₂, CN, CHO, COOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Het, NHCOOA, NHCON(R⁷R^{7'}), NHCOO[C(R⁷R^{7'})]ₚN(R⁷)₂, NHCOO[C(R⁷R^{7'})]ₚHet, NHCONH[C(R⁷R^{7'})]ₚN(R⁷)₂, NHCONH[C(R⁷R^{7'})]ₚHet, OCONH[C(R⁷R^{7'})]ₚN(R⁷)₂, OCONH[C(R⁷R⁷)]ₚHet, CONR⁷[C(R⁷R^{7'})]ₚN(R⁷)₂, CONR⁷[C(R⁷R^{7'})]ₚHet, COA, [C(R⁷R^{7'})]ₚHet, [C(R⁷R^{7'})]ₚAr', CO[C(R⁷R^{7'})]ₚHet, CO[C(R⁷R^{7'})]ₚAr', CONR⁷[C(R⁷R^{7'})]ₚAr', COO[C(R⁷R^{7'})]ₚHet, COO[C(R⁷R^{7'})]ₚAr', S(O)ₘ[C(R⁷R^{7'})]ₚHet and/or S(O)ₘ[C(R⁷R^{7'})]ₚAr',
Ar' denotes phenyl which is unsubstituted or mono-, di-, tri-, tetra- or pentasubstituted by Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA and/or COA,
A' denotes unbranched or branched alkyl having 1-8 C atoms, in which 1-7 H atoms may be replaced by F, Cl and/or Br,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OR⁷, [C(R⁷R^{7'})]ₚAr, [C(R⁷R^{7'})]ₚHet', COA, CO[C(R⁷R^{7'})]ₚAr, CO[C(R⁷R^{7'})]ₚHet', CON(R⁸)[C(R⁷R^{7'})]ₚAr, CON(R⁸)[C(R⁷R^{7'})]ₚHet', CON(R⁷R^{7'}), COOR⁷, COO[C(R⁷R^{7'})]ₚAr, COO[C(R⁷R^{7'})]ₚHet', S(O)ₘA, S(O)ₘAr, S(O)ₘHet', NO₂, CN, NR⁷COOA, NR⁷COOAr, NR⁷COOHet', OCONHA', OCONHAr, OCONHHet', NR⁷SO₂A, NR⁷SO₂Ar, NR⁷SO₂Het', SO₂N(R⁷)A, SO₂N(R⁷)Ar, SO₂N(R⁷)Het', CHO, =S, =NH, =NA, oxy (-O⁻) and/or =O (carbonyl oxygen),
Het' denotes a monocyclic saturated heterocycle having 1 to 2 N and/or O atoms, which may be mono- or disubstituted by A, OA, OH, Hal and/or =O (carbonyl oxygen),
m denotes 0, 1 or 2,
n denotes 1, 2 or 3,
p denotes 0, 1, 2, 3 or 4,
and pharmaceutically usable salts, tautomers and stereoisomers thereof, including mixtures thereof in all ratios;
where compounds of the formula I in which
a) W = COCH₂Het;
b) n = 2 and W = unsubstituted alkyl having 1-10 C atoms or cycloalkyl having 3-7 C atoms,
are excluded.

15. Compounds according to Claim 14 in which
R¹, R² each, independently of one another, denote H or A',
R³, R⁴ denote H,
R⁵, R⁶ each, independently of one another, denote H or A',
and pharmaceutically usable salts, tautomers and stereoisomers thereof, including mixtures thereof in all ratios.

16. Compounds according to Claim 14 or 15 in which
Q denotes H,
and pharmaceutically usable salts, tautomers and stereoisomers thereof,
including mixtures thereof in all ratios.

17. Compounds according to one or more of Claims 14-16 in which
W denotes [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA or [C(R⁸R^{8'})]ₚNHCOOA,
and pharmaceutically usable salts, tautomers and stereoisomers thereof, including mixtures thereof in all ratios.

18. Compounds according to one or more of Claims 14-16 in which
R⁷, R^{7'} each, independently of one another, denote H or A',
R⁸, R^{8'} each, independently of one another, denote H, A or Het,
and pharmaceutically usable salts, tautomers and stereoisomers thereof, including mixtures thereof in all ratios.

19. Compounds according to one or more of Claims 14-18 in which
Z denotes Het or A,
and pharmaceutically usable salts, tautomers and stereoisomers thereof, including mixtures thereof in all ratios.

20. Compounds according to one or more of Claims 16-21 in which
A denotes unbranched or branched alkyl having 1-10 C atoms, in which 1-7 H atoms may be replaced by F, Cl, Br, OH and/or OCH₃,
and/or in which one or two non-adjacent CH₂ groups may be replaced by O, SO₂, NR⁷, NR⁷CO and/or CONR⁷ groups,
and pharmaceutically usable salts, tautomers and stereoisomers thereof, including mixtures thereof in all ratios.

21. Compounds according to one or more of Claims 14-20 in which
Ar denotes phenyl which is unsubstituted or mono-, di-, tri-, tetra- or pentasubstituted by Hal and/or [C(R⁷R^{7'})]ₚOR⁷,
and pharmaceutically usable salts, tautomers and stereoisomers thereof, including mixtures thereof in all ratios.

22. Compounds according to one or more of Claims 14-21 in which
Het denotes a mono- or bicyclic saturated, unsaturated or aromatic heterocycle having 1 to 4 N, O and/or S atoms, which may be unsubstituted or mono-, di- or trisubstituted by A, OR⁷, [C(R⁷R^{7'})]ₚAr, COOR⁷ and/or =O (carbonyl oxygen),
and pharmaceutically usable salts, tautomers and stereoisomers thereof, including mixtures thereof in all ratios.

23. Compounds according to one or more of Claims 14-22 in which
Het denotes pyrrolidinyl, piperidinyl, morpholinyl, oxazolidinyl, tetrahydroquinazolinyl, piperazinyl, thiazolyl, furyl, thienyl, pyrrolyl, imidazolyl, pyrazolyl, oxazolyl, isoxazolyl, isothiazolyl, pyridyl, pyrimidinyl, triazolyl, tetrazolyl, oxadiazolyl or thiadiazolyl, each of which is unsubstituted or mono-, di- or trisubstituted by A, OR⁷, [C(R⁷R^{7'})]ₚAr, COOR⁷ and/or =O (carbonyl oxygen),
and pharmaceutically usable salts, tautomers and stereoisomers thereof, including mixtures thereof in all ratios.

24. Compounds according to one or more of Claims 14-23 in which
R¹, R² each, independently of one another, denote H or A',
R³, R⁴ denote H,
R⁵, R⁶ each, independently of one another, denote H or A',
R⁷, R^{7'} each, independently of one another, denote H or A',
R⁸, R^{8'} each, independently of one another, denote H, A or Het,
R⁹ denotes H or A',
R¹⁰, R¹¹ each, independently of one another, denote H, A' or OH,
Q denotes H,
W denotes [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA or [C(R⁸R^{8'})]ₚNHCOOA,
Z denotes Het or A,
A denotes unbranched or branched alkyl having 1-10 C atoms, in which 1-7 H atoms may be replaced by F, Cl, Br, OH and/or OCH₃,
and/or in which one or two non-adjacent CH₂ groups may be replaced by O, NR⁷, SO₂, NR⁷CO and/or CONR⁷ groups,
A' denotes unbranched or branched alkyl having 1-8 C atoms, in which 1-7 H atoms may be replaced by F, Cl and/or Br,
Ar denotes phenyl which is unsubstituted or mono-, di-, tri-, tetra- or pentasubstituted by Hal and/or [C(R⁷R^{7'})]ₚOR⁷,
Het denotes a mono- or bicyclic saturated, unsaturated or aromatic heterocycle having 1 to 4 N, O and/or S atoms, which may be unsubstituted or mono-, di- or trisubstituted by A, OR⁷, [C(R⁷R^{7'})]ₚAr, COOR⁷ and/or =O (carbonyl oxygen),
n denotes 1 or 2,
p denotes 0, 1, 2, 3 or 4,
and pharmaceutically usable salts, tautomers and stereoisomers thereof, including mixtures thereof in all ratios;
where compounds of the formula I in which
a) W = COCH₂Het;
b) n = 2 and W = unsubstituted alkyl having 1-10 C atoms or cycloalkyl having 3-7 C atoms,
are excluded.

25. Compounds according to Claim 14, selected from the group
| No. | Structure and/or name |
|---|---|
| "A1" | 4-(2-Methyl-3-{4-[4-(5,5,8,8-tetramethyl-5,6,7,8-tetrahydronaphthalen-2-yl)thiazol-2-yl]piperidin-1-yl}-propyl)morpholine |
| "A2" | 1'-Ethyl-4-[4-(5,5,8,8-tetramethyl-5,6,7,8-tetrahydronaphthalen-2-yl)thiazol-2-yl]-[1,3']bipiperidinyl |
| "A3" | 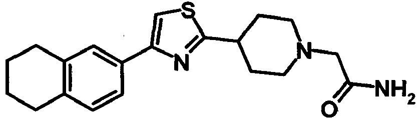 |
| "A4" | 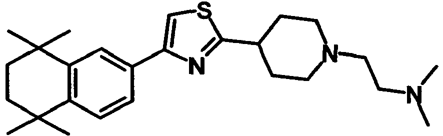 |
| "A5" | 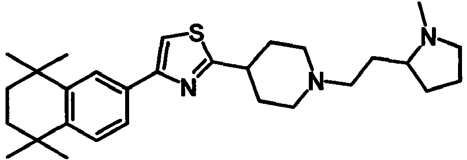 |
| "A6" | 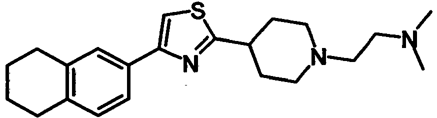 |
| "A7" | 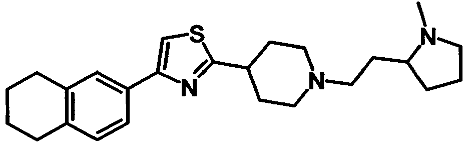 |
| "A8" | 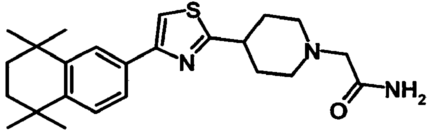 |
| "A9" | 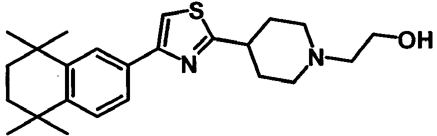 |
| "A10" | 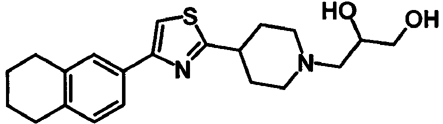 |
| "A11" | 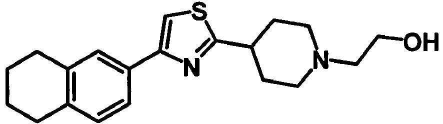 |
| "A12" | 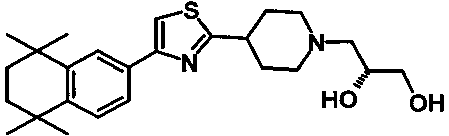 |
| "A13" | 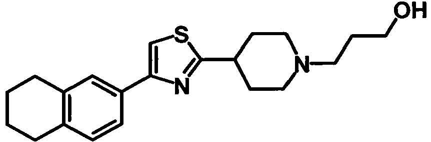 |
| "A14" | 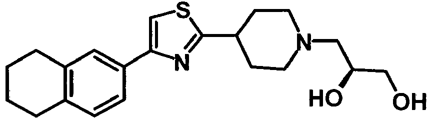 |
| "A15" | 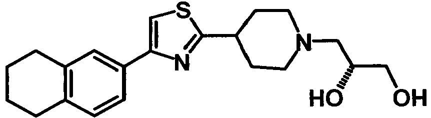 |
| "A16" | 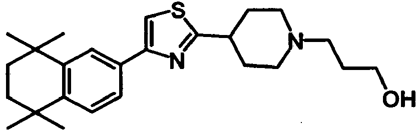 |
| "A17" | 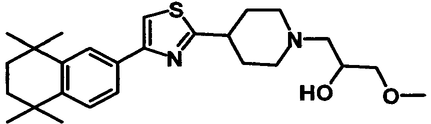 |
| "A18" | 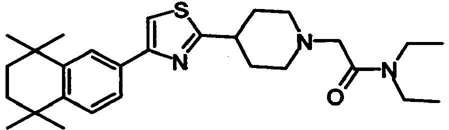 |
| "A19" | 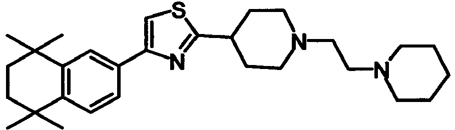 |
| "A20" | 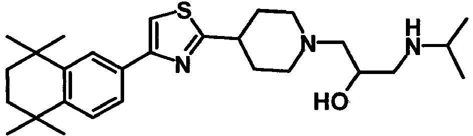 |
| "A21" | 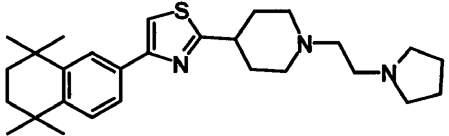 |
| "A22" | 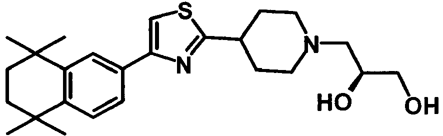 |
| "A23" | 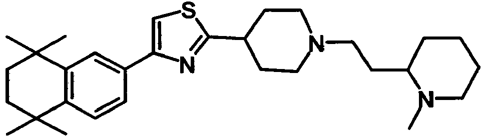 |
| "A24" | 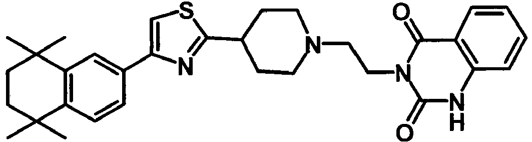 |
| "A25" | 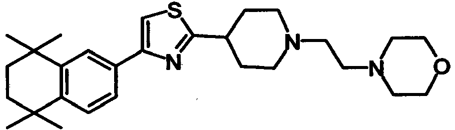 |
| "A26" | 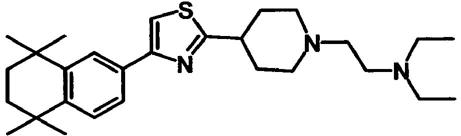 |
| "A27" | 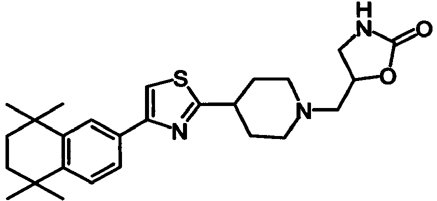 |
| "A28" | 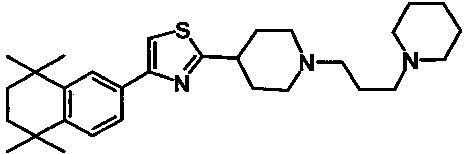 |
| "A29" | 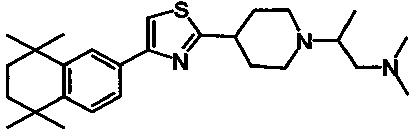 |
| "A30" | 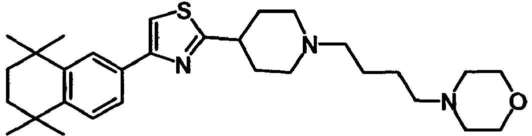 |
| "A31" | 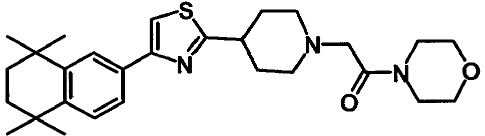 |
| "A32" | 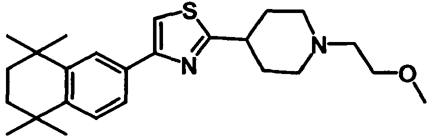 |
| "A33" | 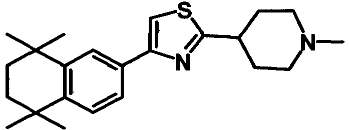 |
| "A34" | 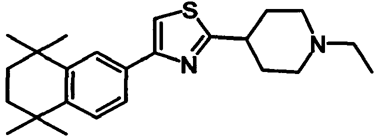 |
| "A35" | 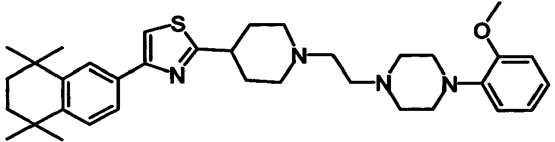 |
| "A36" | 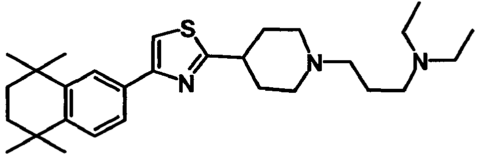 |
| "A37" | *tert-*Butyl (2-{4-[4-(5,5,8,8-tetramethyl-5,6,7,8-tetrahydronaphthalen-2-yl)thiazol-2-yl]piperidin-1-yl}ethyl)carbamate |
| "A38" | 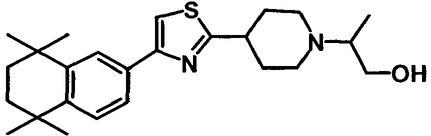 |
| "A39" | 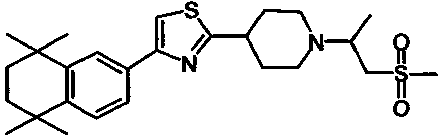 |
| "A40" | 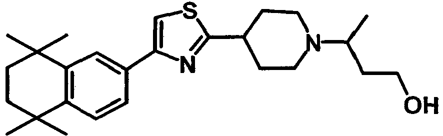 |
| "A41" | *tert*-Butyl (2-{4-[4-(5,5,8,8-tetramethyl-5,6,7,8-tetrahydronaphthalen-2-yl)thiazol-2-yl]piperidin-1-yl}ethyl)carbamate |
| "A42" | 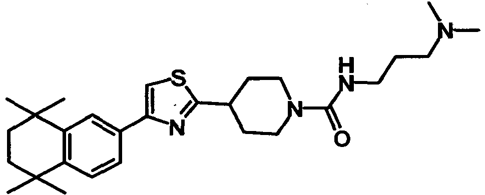 |
| "A43" | 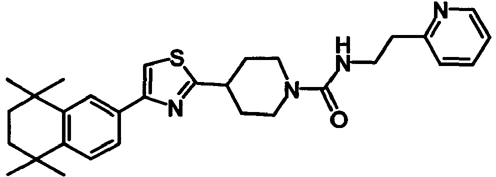 |
| "A44" | 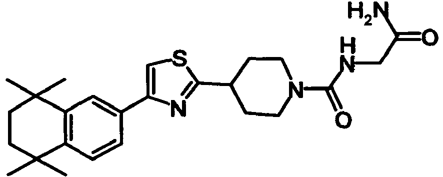 |
| "A45" | 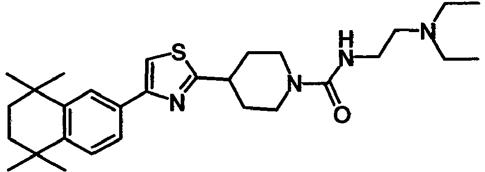 |
| "A46" | 3-Methoxypropyl 4-[4-(5,5,8,8-tetramethyl-5,6,7,8-tetrahydronaphthalen-2-yl)thiazol-2-yl]piperidine-1-carboxylate |
| "A47" | 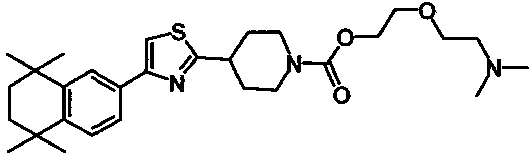 |
| "A48" | 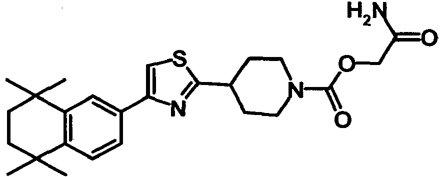 |
| "A49" | 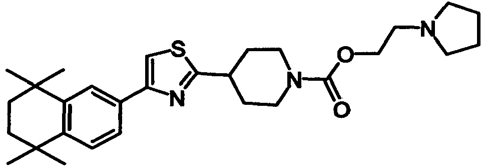 |
| "A50" | 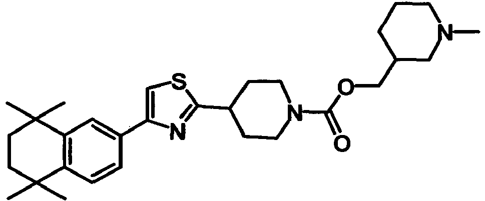 |
| "A51" | 2-Pyridin-4-yl-1-{4-[4-(5,5,8,8-tetramethyl-5,6,7,8-tetrahydronaphthalen-2-yl)thiazol-2-yl]piperidin-1-yl}-ethanone |
| "A52" | 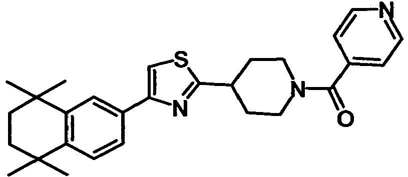 |
| "A53" | 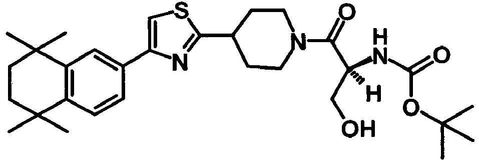 |
| "A54" | 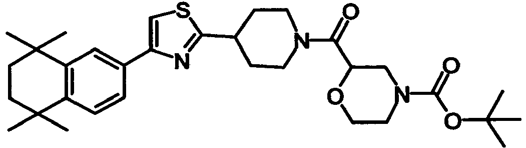 |
| "A55" | 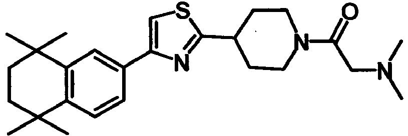 |
| "A56" | 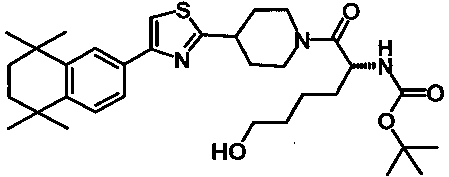 |
| "A57" | 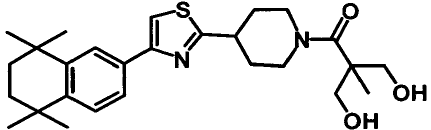 |
| "A58" | 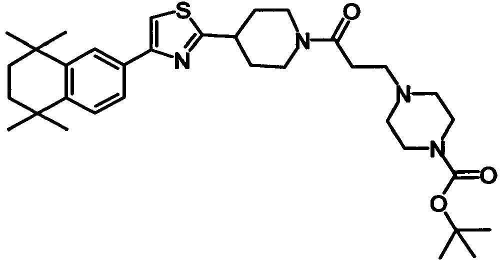 |
| "A59" | 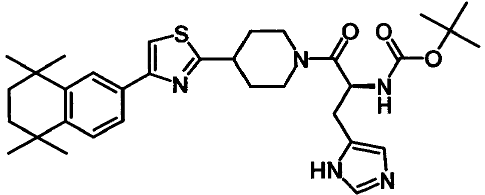 |
| "A60" | 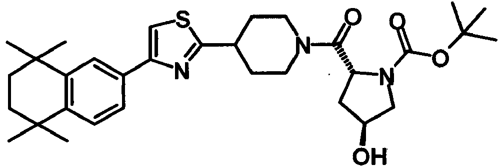 |
| "A61" | 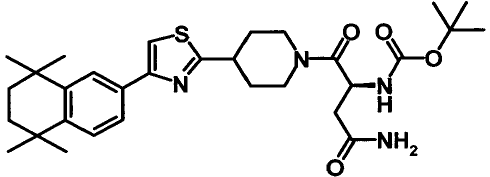 |
| "A62" | 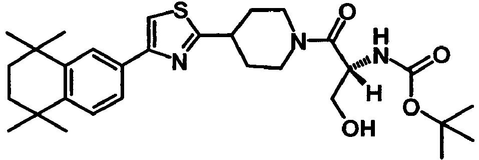 |
| "A63" | (S)-2-Amino-3-(3H-imidazol-4-yl)-1-{4-[4-(5,5,8,8-tetramethyl-5,6,7,8-tetrahydronaphthalen-2-yl)thiazol-2-yl]piperidin-1-yl}propan-1-one |
| "A64" | 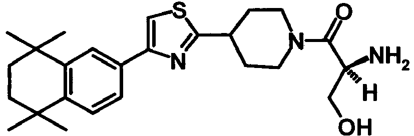 |
| "A65" | 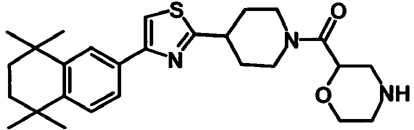 |
| "A66" | 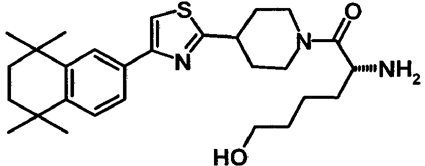 |
| "A67" | 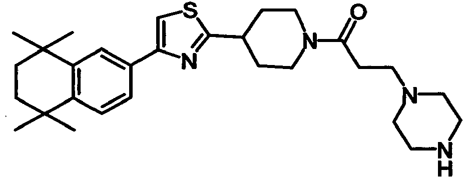 |
| "A68" | 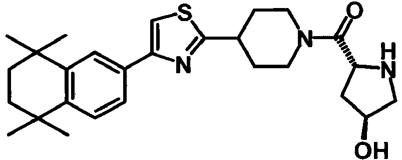 |
| "A69" | 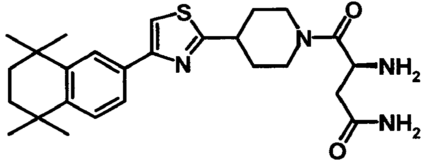 |
| "A70" | 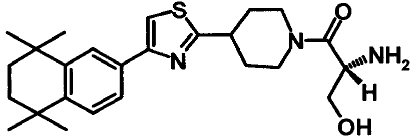 |
| "A71" | 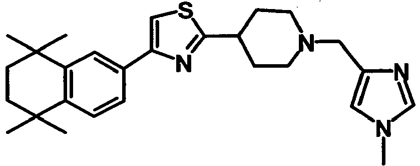 |
| "A72" | 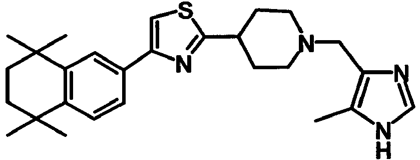 |
| "A73" | 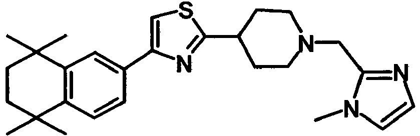 |
| "A74" | 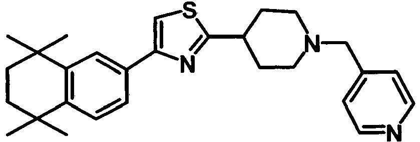 |
| "A75" | 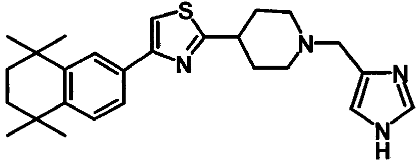 |
| "A76" | 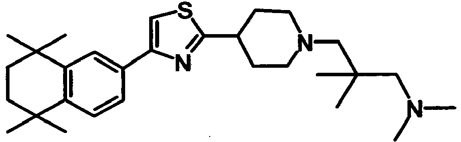 |
| "A77" | 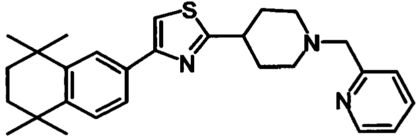 |
| "A78" | 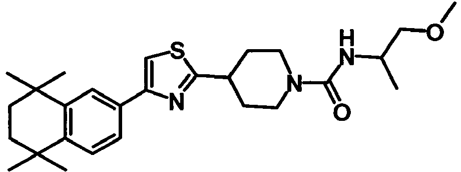 |
| "A79" | 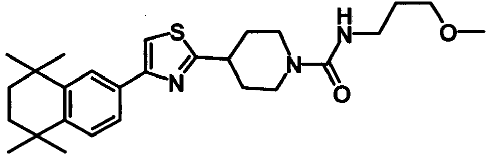 |
| "A80" | 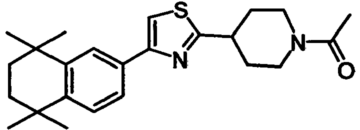 |
| "A81" | 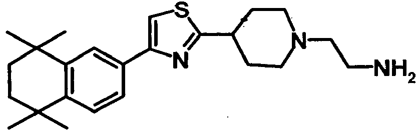 |
| "A82" | 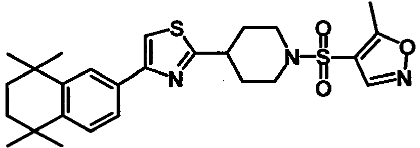 |
| "A83" | 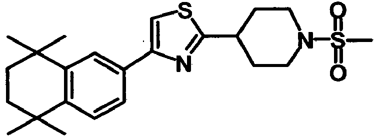 |
| "B1" | 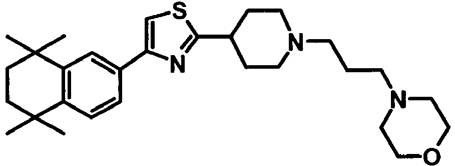 |
| "B2" | 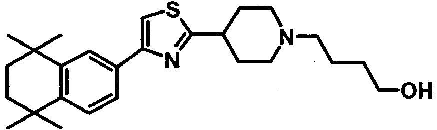 |
| "B3" | 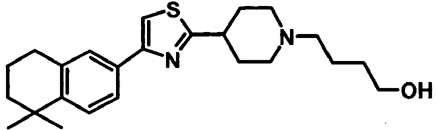 |
| "B4" | 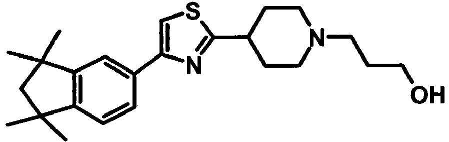 |
| "B5" | 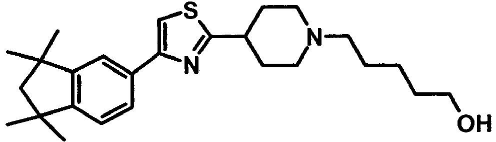 |
| "B6" | 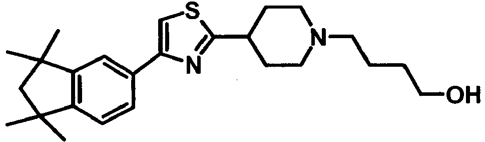 |
| "B7" | 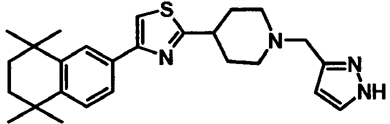 |
| "B8" | 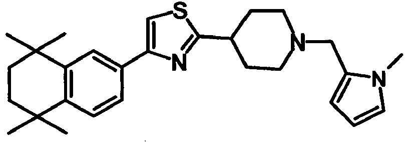 |
| "B9" | 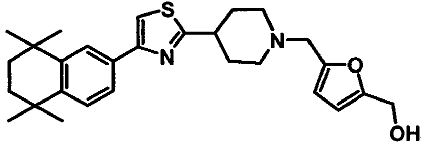 |
| "B10" | 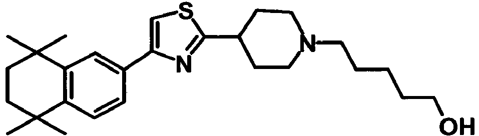 |
| "B11" | 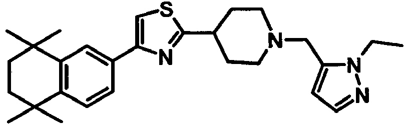 |
| "B12" | 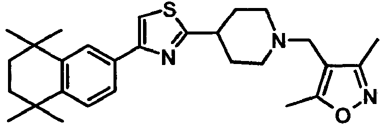 |
| "B13" | 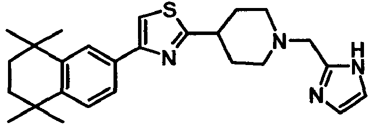 |
| "B14" | 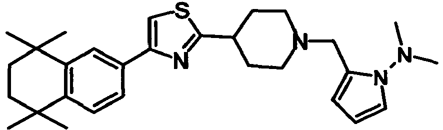 |
| "B15" | 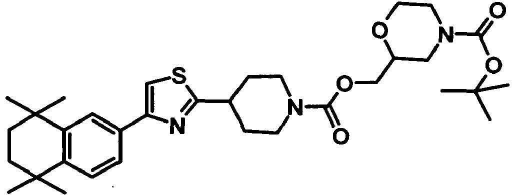 |
| "B16" | 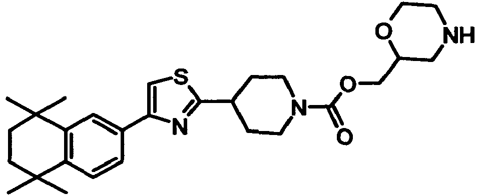 |
and pharmaceutically usable salts, tautomers and stereoisomers thereof, including mixtures thereof in all ratios.

26. Process for the preparation of compounds of the formula I according to Claims 14-25 and pharmaceutically usable salts, tautomers and stereoisomers thereof, **characterised in that**
a) a compound of the formula II 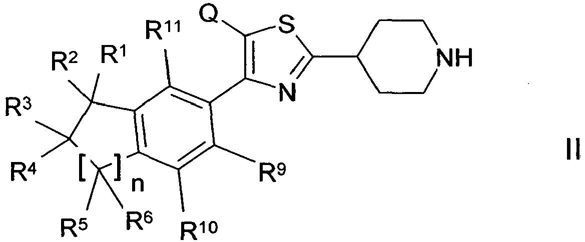 in which R¹, R², R³, R⁴, R⁵, R⁶, R⁹, R¹⁰, R¹¹, Q and n have the meanings indicated in Claim 1,
is reacted with a compound of the formula III
L-W III,
in which W has the meaning indicated in Claim 1 and
L denotes Cl, Br, I or a free or reactively functionally modified OH group,
or
b) for the preparation of compounds of the formula I in which
W denotes [C(R⁸R^{8'})]ₚZ or [C(R⁸R^{8'})]ₚNHCOOA,
R⁸ denotes H,
p denotes 1, 2, 3 or 4,
R^{8'}, Z, A have the meanings indicated in Claim 1,
a compound of the formula II is reacted
i) with a compound of the formula IVa
R^{8'}-CO-[C(R⁸R^{8'})]ₚ₋₁Z IVa,
in which
p denotes 1, 2, 3 or 4 and
R^{8'}, Z have the meanings indicated in Claim 1,
in a reductive amination,
or
ii) with a compound of the formula IVb
R^{8'}-CO-[C(R⁸R^{8'})]ₚ₋₁NHCOOA IVb,
in which
p denotes 1, 2, 3 or 4 and
R⁸, A have the meanings indicated in Claim 1,
in a reductive amination,
or
c) in that they are liberated from one of their functional derivatives by treatment with a solvolysing or hydrogenolysing agent,
and/or
a base or acid of the formula I is converted into one of its salts.

27. Medicaments comprising at least one compound of the formula I according to Claims 14-25 and/or pharmaceutically usable salts, tautomers and stereoisomers thereof, including mixtures thereof in all ratios, and optionally excipients and/or adjuvants.

28. Medicaments comprising at least one compound of the formula I according to one or more of Claims 14 to 25, and/or pharmaceutically usable salts and stereoisomers thereof, including mixtures thereof in all ratios, and at least one further medicament active compound.

29. Set (kit) consisting of separate packs of
(a) an effective amount of a compound of the formula I according to one or more of Claims 14 to 25, and/or pharmaceutically usable salts and stereoisomers thereof, including mixtures thereof in all ratios,
and
(b) an effective amount of a further medicament active compound.

30. Compounds selected from the group
| No. | Structure and/or name |
|---|---|
| "C2" | 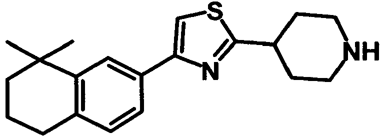 |
| "C3" | 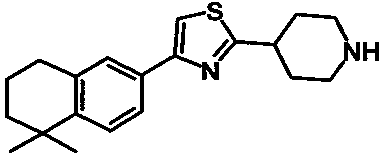 |
| "C4" | 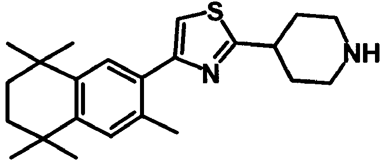 |
| "C6" | 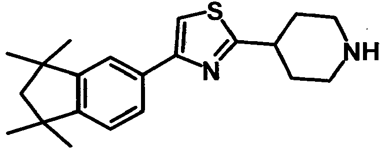 |
| "C7" | 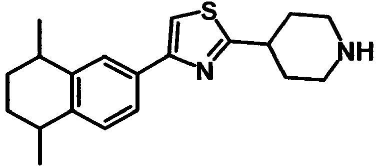 |
| "C8" | 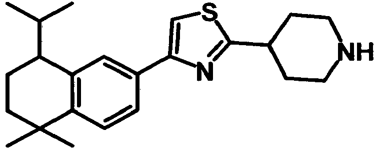 |
| "C9" | 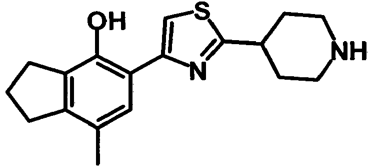 |
| "C10" | 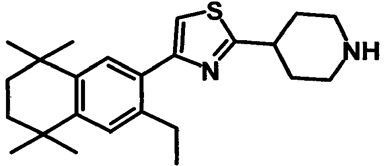 |
| "C11" | 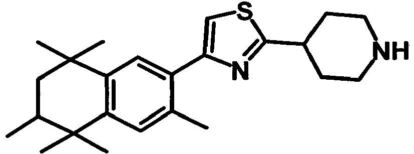 |
| "C12" | 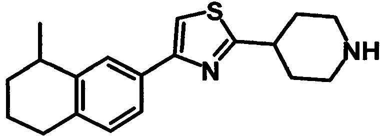 |
and pharmaceutically usable salts, tautomers and stereoisomers thereof, including mixtures thereof in all ratios.

31. Use of compounds selected from the group
| No. | Structure and/or name |
|---|---|
| "C1" | 4-[4-(5,5,8,8-Tetramethyl-5,6,7,8-tetrahydronaphthalen-2-yl)thiazol-2-yl]piperidine |
| "C2" | 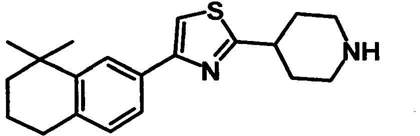 |
| "C3" | 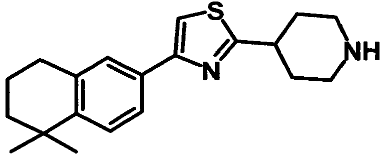 |
| "C4" | 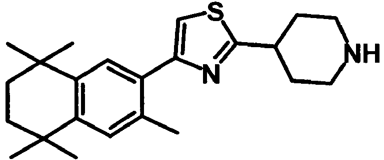 |
| "C5" | 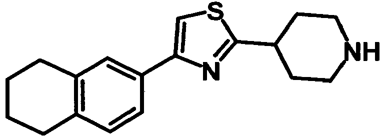 |
| "C6" | 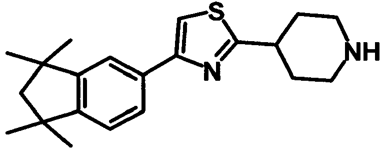 |
| "C7" | 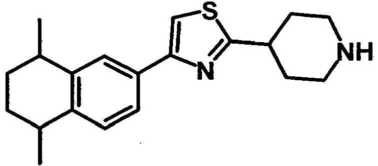 |
| "C8" | 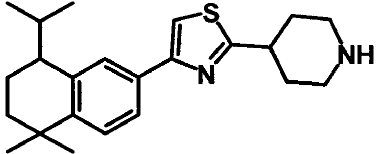 |
| "C9" | 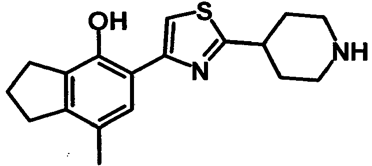 |
| "C10" | 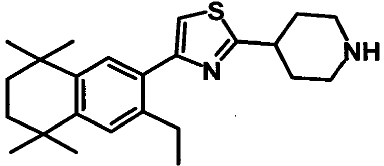 |
| "C11" | 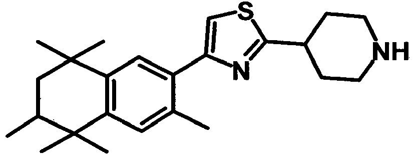 |
| "C12" | 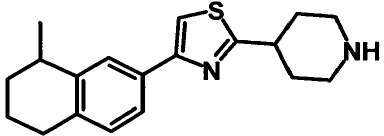 |
and pharmaceutically usable salts, tautomers and stereoisomers thereof, including mixtures thereof in all ratios, for the preparation of a medicament for the treatment of diseases, where the diseases to be treated are selected from the group
hyperproliferative disease, inflammatory disease, angiogenic disease, fibrotic disease of the lung, kidney, liver and heart

32. Use according to Claim 31, where the hyperproliferative disease is selected from the group
cancer (tumour disease), atherosclerosis, restenosis, proliferative disease of the mesangial cells, psoriasis.

33. Use according to Claim 32, where the tumour disease is selected from the group
tumour of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

34. Use according to Claim 32, where the proliferative disease of the mesangial cells is selected from the group
glomerulonephritis, diabetic nephropathy, malignant nephrosclerosis, thrombotic microangiopathy syndrome, transplant rejection, glomerulopathy.

35. Use according to Claim 31, where the inflammatory disease is selected from the group
inflammatory bowel disease, arthritis, atherosclersosis, asthma, allergies, inflammatory kidney diseases, multiple sclerosis, chronic obstructive pulmonary disease, inflammatory skin diseases, pardontal diseases, psoriasis, T-cell-promoted immune disease.

36. Use according to Claim 35, where the inflammatory bowel disease is selected from the group
ulcerative colitis, Crohn's disease, non-specific colitis.

37. Use according to Claim 35, where the T-cell-promoted immune disease is selected from the group
allergic encephalomyelitis, allergic neuritis, transplant rejection, graft-versus-host reaction, myocarditis, thyroiditis, nephritis, systemic lupus erythematosus, insulin-dependent diabetes mellitus.

38. Use according to Claim 35, where the arthritis disease is selected from the group
rheumatoid arthritis, osteoarthritis, Caplan's syndrome, Felty's syndrome, Sjogren's syndrome, spondylitis ankylosans, Still's disease, chondrocalcinosis, metabolic arthritis, rheumatic fever, Reiter's disease, Wissler's syndrome.

39. Use according to Claim 35, where the inflammatory kidney disease is selected from the group
glomerulonephritis, glomerular injury, nephrotic syndrome, interstitial nephritis, lupus nephritis, Goodpasture's syndrome, Wegener's granulomatosis, renal vasculitis, IgA nephropathy, idiopathic glomerular disease.

40. Use according to Claim 35, where the inflammatory skin disease is selected from the group
psoriasis, atopic dermatitis, contact sensitivity, acne.

41. Use according to Claim 31, where the angiogenic disease is selected from the group
diabetic retinopathy, arthritis, cancer, psoriasis, Kaposi's sarcoma, haemangioma, myocardial angiogenesis, atherosclerotic plaque neovascularisation, angiogenic eye diseases, choroidal neovascularisation, retrolental fibroplasia, macular degeneration, corneal transplant rejection, rubeosis iridis, neuroscular glaucoma, Oster Webber syndrome.

42. Medicaments comprising at least one compound selected from the group
| No. | Structure and/or name |
|---|---|
| "C2" | 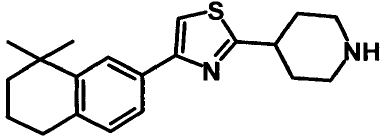 |
| "C3" | 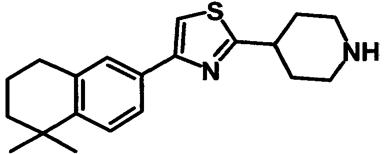 |
| "C4" | 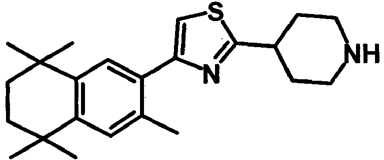 |
| "C6" | 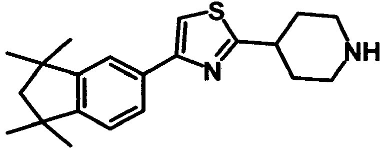 |
| "C7" | 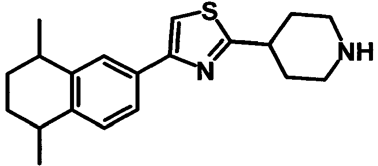 |
| "C8" | 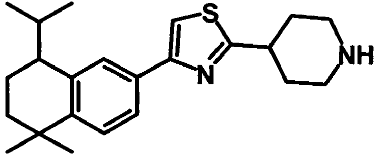 |
| "C9" | 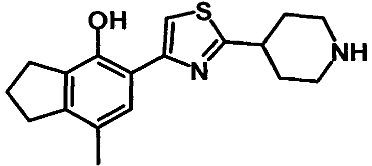 |
| "C10" | 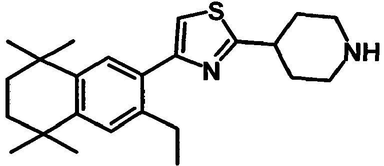 |
| "C11" | 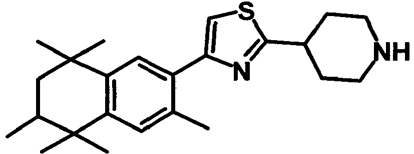 |
| "C12" | 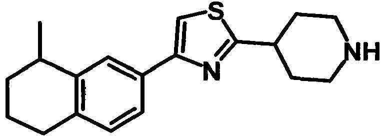 |
and/or pharmaceutically usable salts, tautomers and stereoisomers thereof, including mixtures thereof in all ratios, and optionally excipients and/or adjuvants.

## Revendications

1. Utilisation de composés de formule I 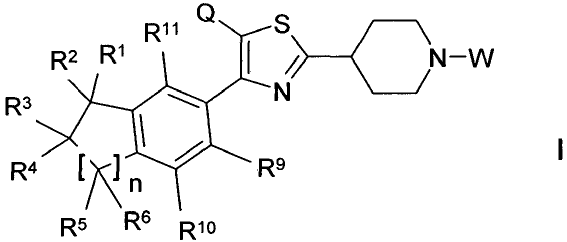 dans laquelle
R¹, R², R³, R⁴, R⁵, R⁶ désignent chacun, indépendamment les uns des autres, H ou A',
Q désigne H, A, Hal, COOR⁷ ou CON(R⁷R^{7'}),
W désigne [C(R⁸R^{8'})]ₚZ, CO-[C(R⁸R^{8'})]ₚZ, CO-N(R⁸)-[C(R⁸R⁸)]ₚZ, CO-O-[C(R⁸R^{8'})]ₚZ, SO₂-[C(R⁸R^{8'})]ₚZ, CO[C(R⁸R⁸)]ₚNHCOOA ou [C(R⁸R^{8'})]ₚNHCOOA,
R⁷, R^{7'} désignent chacun, indépendamment l'un de l'autre, H ou A',
R⁸, R^{8'} désignent chacun, indépendamment l'un de l'autre, H, A, OA, NAA' ou Hét,
R⁹ désigne H ou A',
R¹⁰, R¹¹ désignent chacun, indépendamment l'un de l'autre, H, A' ou OH,
Z désigne Hét, Ar ou A,
A désigne alkyle non ramifié ou ramifié ayant 1-10 atomes de C, où 1-7 atomes de H peuvent être remplacés par F, Cl, Br, OH et/ou OCH₃,
et/ou où un ou deux groupements CH₂ non adjacents peuvent être remplacés par des groupements O, S, SO, SO₂, CO, COO, NR⁷, NR⁷CO, CONR⁷, CH=CH et/ou CH≡CH
ou
alkyle cyclique ayant 3-7 atomes de C,
Ar désigne phényle, naphtyle ou biphényle, chacun d'entre eux étant non substitué ou mono-, di-, tri-, tétra- ou pentasubstitué par Hal, A, [C(R⁷R^{7'})]ₚOR⁷, [C(R⁷R^{7'})]ₚN(R⁷)₂, SR⁷, NO₂, CN, CHO, COR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Hét, NHCOOA, NHCON(R⁷R^{7'}), NHCOO[C(R⁷R^{7'})]ₚN(R⁷)₂, NHCOO[C(R⁷R⁷)]ₚHét, NHCONH[C(R⁷R^{7'})]ₚN(R⁷)₂, NHCONH[C(R⁷R^{7'})]ₚHét, OCONH[C(R⁷R^{7'})]ₚN(R⁷)₂, OCONH[C(R⁷R⁷)]ₚHét, CONR⁷[C(R⁷R^{7'})]ₚN(R⁷)₂, CONR⁷[C(R⁷R^{7'})]ₚHét, COA, [C(R⁷R^{7'})]ₚHét, [C(R⁷R^{7'})]ₚAr', CO[C(R⁷R⁷)]ₚHét, CO[C(R⁷R^{7'})]ₚAr', CONR⁷[C(R⁷R^{7'})]ₚAr', COO[C(R⁷R^{7'})]ₚHét, COO[C(R⁷R⁷)]ₚAr', S(O)ₘ[C(R⁷R^{7'})]ₚHét et/ou S(O)ₘ[C(R⁷R^{7'})]ₚAr',
Ar' désigne phényle qui est non substitué ou mono-, di-, tri-, tétra- ou pentasubstitué par Hal, A, OH, OA', NH₂, NHA, NAA', CN, CHO, COOR⁷, CON(R⁷R^{7'}), NR⁷COA, NR⁷SO₂A, SO₂N(R⁷R^{7'}), S(O)ₘA et/ou COA,
A' désigne alkyle non ramifié ou ramifié ayant 1-8 atomes de C, où 1-7 atomes de H peuvent être remplacés par F, Cl et/ou Br,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OR⁷, [C(R⁷R^{7'})]ₚAr, [C(R⁷R^{7'})]ₚHét', COA, CO[C(R⁷R^{7'})]ₚAr, CO[C(R⁷R^{7'})]ₚHét', CON(R⁸)[C(R⁷R^{7'})]ₚAr, CON(R⁸)[C(R⁷R^{7'})]ₚHét', CON(R⁷R^{7'}), COOR⁷, COO[C(R⁷R^{7'})]ₚAr, COO[C(R⁷R^{7'})]ₚHét', S(O)ₘA, S(O)ₘAr, S(O)ₘHét', NO₂, CN, NR⁷COOA, NR⁷COOAr, NR⁷COOHét', OCONHA', OCONHAr, OCONHHét', NR⁷SO₂A, NR⁷SO₂Ar, NR⁷SO₂Hét', SO₂N(R⁷)A, SO₂N(R⁷)Ar, SO₂N(R⁷)Hét', CHO, =S, =NH, =NA, oxy (-O⁻) et/ou =O (oxygène du carbonyle),
Hét' désigne un hétérocycle monocyclique saturé ayant de 1 à 2 atomes de N et/ou O, qui peut être mono- ou disubstitué par A, OA, OH, Hal et/ou =O (oxygène du carbonyle),
m désigne 0, 1 ou 2,
n désigne 1, 2 ou 3,
p désigne 0, 1, 2, 3 ou 4,
et les sels, tautomères et stéréoisomères pharmaceutiquement utilisables de ceux-ci, y compris des mélanges de ceux-ci en toutes proportions,
pour la préparation d'un médicament destiné au traitement de maladies, où les maladies à traiter sont choisies dans le groupe constitué par les maladies hyperprolifératives, les maladies inflammatoires, les maladies angiogènes, les maladies fibrotiques du poumon, du rein, du foie et du coeur.

2. Utilisation selon la revendication 1, dans laquelle la maladie hyper-proliférative est choisie dans le groupe constitué par le cancer (maladie tumorale), l'athérosclérose, la resténose, la maladie proliférative des cellules mésangiales, le psoriasis.

3. Utilisation selon la revendication 2, dans laquelle la maladie tumorale est choisie dans le groupe constitué par
les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

4. Utilisation selon la revendication 2, dans laquelle la maladie proliférative des cellules mésangiales est choisie dans le groupe constitué par la glomérulonéphrite, la néphropathie diabétique, la néphrosclérose maligne, le syndrome de microangiopathie thrombotique, le rejet de greffe, la glomérulopathie.

5. Utilisation selon la revendication 1, dans laquelle les maladies inflammatoires sont choisies dans le groupe constitué par
l'affection abdominale inflammatoire, l'arthrite, l'athérosclérose, l'asthme, les allergies, les maladies inflammatoires rénales, la sclérose en plaques, la broncho-pneumopathie chronique obstructive, les maladies inflammatoires cutanées, les maladies parodontales, le psoriasis, les maladies immunitaires favorisées par les lymphocytes T.

6. Utilisation selon la revendication 5, dans laquelle l'affection abdominale inflammatoire est choisie dans le groupe constitué par
la rectocolite hémorragique, la maladie de Crohn, la colite non spécifique.

7. Utilisation selon la revendication 5, dans laquelle les maladies immunitaires favorisées par les lymphocytes T sont choisies dans le groupe constitué par
l'encéphalomyélite allergique, la névrite allergique, le rejet de greffe, la réaction du greffon contre l'hôte, la myocardite, la thyroïdite, la néphrite, le lupus érythémateux disséminé, le diabète sucré insulinodépendant.

8. Utilisation selon la revendication 5, dans laquelle la maladie arthritique est choisie dans le groupe constitué par
la polyarthrite rhumatoïde, l'arthrose, le syndrome de Caplan-Colinet, le syndrome de Felty, le syndrome de Sjôgren, la spondylarthrite ankylosante, la maladie de Still, la chondrocalcinose, l'arthrite métabolique, le rhumatisme articulaire aigu, la maladie de Reiter, le syndrome de Wissler-Fanconi.

9. Utilisation selon la revendication 5, dans laquelle la maladie inflammatoire rénale est choisie dans le groupe constitué par
la glomérulonéphrite, les lésions glomérulaires, le syndrome néphrotique, la néphrite interstitielle, le lupus néphrétique, le syndrome de Goodpasture, la granulomatose de Wegener, la vascularite rénale, la néphropathie à IgA, la néphropathie glomérulaire idiopathique.

10. Utilisation selon la revendication 5, dans laquelle la maladie inflammatoire cutanée est choisie dans le groupe constitué par
le psoriasis, la dermite atopique, la sensibilité de contact, l'acné.

11. Utilisation selon la revendication 1, dans laquelle les maladies angiogènes sont choisies dans le groupe constitué par
la rétinopathie diabétique, l'arthrite, le cancer, le psoriasis, le sarcome de Kaposi, l'hémangiome, l'angiogenèse myocardique, la néovascularisation des plaques athérosclérotiques, les maladies oculaires angiogènes, la néovascularisation choroïdienne, la fibroplasie rétrolentale, la dégénérescence maculaire, le rejet de greffe cornéenne, la rubéose de l'iris, le glaucome neurovasculaire, le syndrome d'Osier-Weber.

12. Utilisation selon l'une ou plusieurs parmi les revendications 1-11 de composés de formule I
dans laquelle
R¹, R² désignent chacun, indépendamment l'un de l'autre, H ou A',
R³, R⁴ désignent H,
R⁵, R⁶ désignent chacun, indépendamment l'un de l'autre, H ou A',
R⁷, R^{7'} désignent chacun, indépendamment l'un de l'autre, H ou A',
R⁸, R^{8'} désignent chacun, indépendamment l'un de l'autre, H, A ou Hét,
R⁹ désigne H ou A',
R¹⁰ R¹¹ désignent chacun, indépendamment l'un de l'autre, H, A' ou OH,
Q désigne H,
W désigne [C(R⁸R^{8'})]ₚZ, CO-[C(R⁸R⁸)]ₚZ, CO-N(R⁸)-[C(R⁸R⁸)]ₚZ, CO-O-[C(R⁸R^{8'})]ₚZ, CO[C(R⁸R^{8'})]ₚNHCOOA ou [C(R⁸R^{8'})]ₚNHCOOA,
Z désigne Hét ou A,
A désigne alkyle non ramifié ou ramifié ayant 1-10 atomes de C, où 1-7 atomes de H peuvent être remplacés par F, Cl, Br, OH et/ou OCH₃,
et/ou où un ou deux groupements CH₂ non adjacents peuvent être remplacés par des groupements 0, NR⁷, SO₂, NR⁷CO et/ou CONR⁷,
A' désigne alkyle non ramifié ou ramifié ayant 1-8 atomes de C, où 1-7 atomes de H peuvent être remplacés par F, Cl et/ou Br,
Ar désigne phényle qui est non substitué ou mono-, di-, tri-, tétra- ou pentasubstitué par Hal et/ou [C(R⁷R^{7'})]ₚOR⁷,
Hét désigne un hétérocycle mono- ou bicyclique saturé, insaturé ou aromatique ayant de 1 à 4 atomes de N, O et/ou S, qui peut être non substitué ou mono-, di- ou trisubstitué par A, OR⁷, [C(R⁷R^{7'})]ₚAr, COOR⁷ et/ou =O (oxygène du carbonyle),
n désigne 1 ou 2,
p désigne 0, 1, 2, 3 ou 4,
et de sels, tautomères et stéréoisomères pharmaceutiquement utilisables de ceux-ci, y compris des mélanges de ceux-ci en toutes proportions.

13. Utilisation selon l'une ou plusieurs parmi les revendications 1-12 de composés de formule I choisis dans le groupe constitué par
| n° | Structure et/ou nom |
|---|---|
| "A1" | 4-(2-Méthyl-3-{4-[4-(5,5,8,8-tétraméthyl-5,6,7,8-tétrahydronaphtalén-2-yl)thiazol-2-yl]pipéridin-1-yl}-propyl)morpholine |
| "A2" | 1'-Éthyl-4-[4-(5,5,8,8-tétraméthyl-5,6,7,8-tétrahydronaphtalén-2-yl)thiazol-2-yl]-[1,3']bipipéridinyle |
| "A3" | 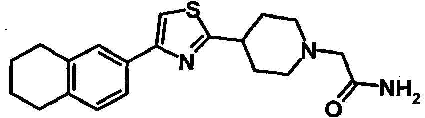 |
| "A4" | 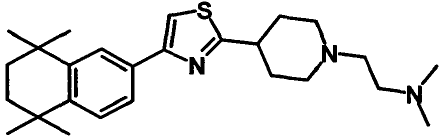 |
| "A5" | 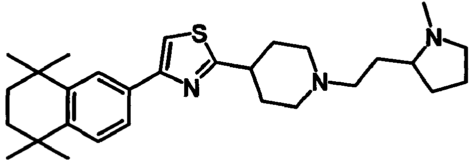 |
| "A6" | 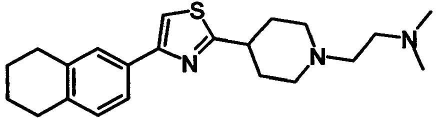 |
| "A7" | 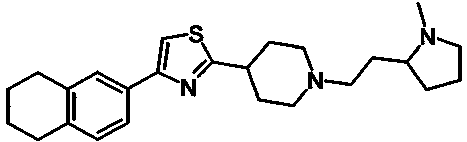 |
| "A8" | 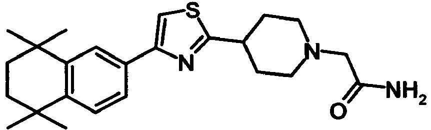 |
| "A9" | 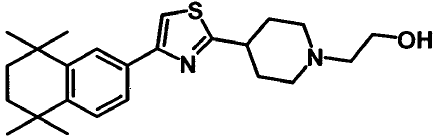 |
| "A10" | 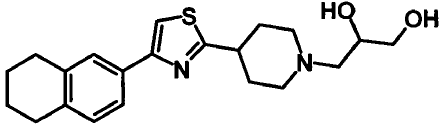 |
| "A11" | 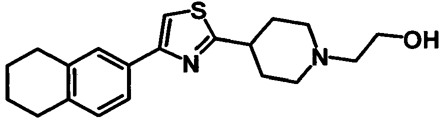 |
| "A12" | 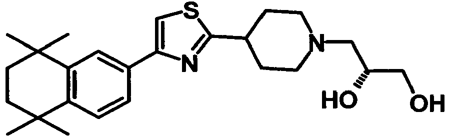 |
| "A13" | 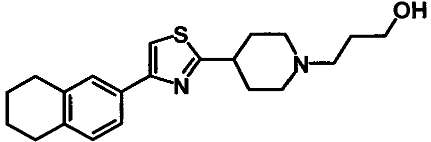 |
| "A14" | 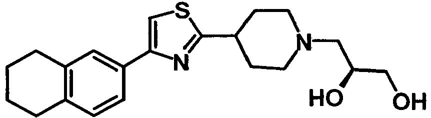 |
| "A15" | 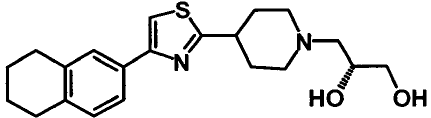 |
| "A16" | 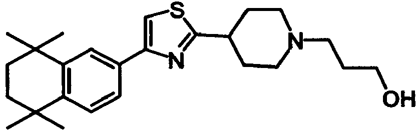 |
| "A17" | 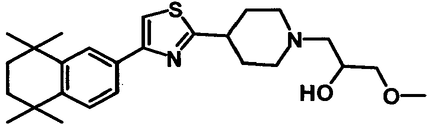 |
| "A18" | 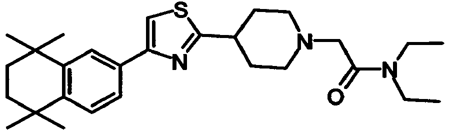 |
| "A19" | 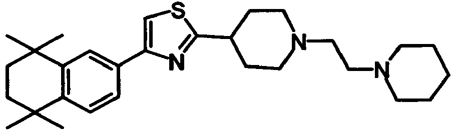 |
| "A20" | 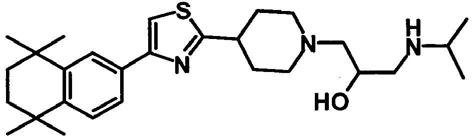 |
| "A21" | 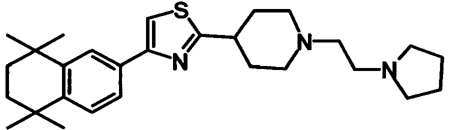 |
| "A22" | 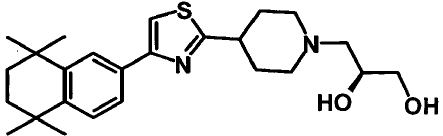 |
| "A23" | 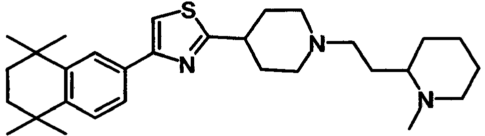 |
| "A24" | 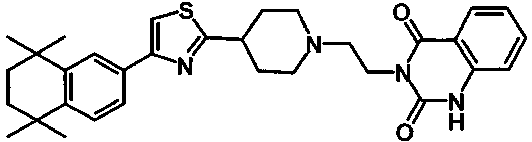 |
| "A25" | 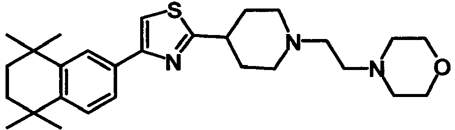 |
| "A26" | 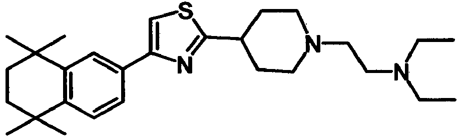 |
| "A27" | 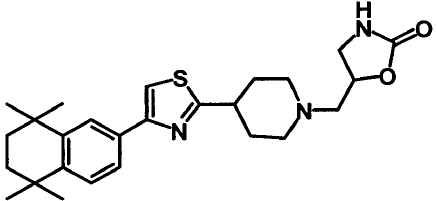 |
| "A28" | 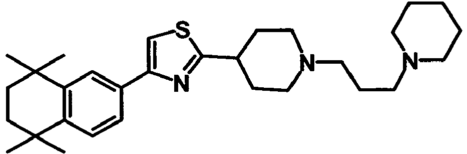 |
| "A29" | 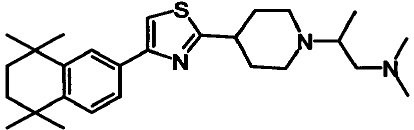 |
| "A30" | 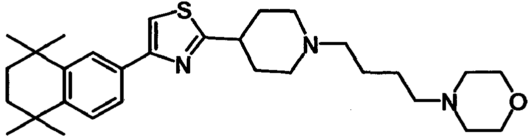 |
| "A31" | 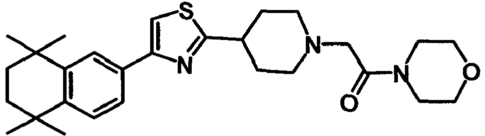 |
| "A32" | 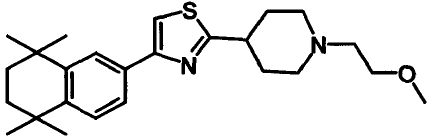 |
| "A33" | 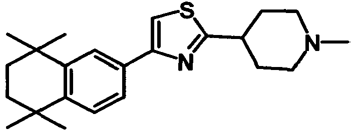 |
| "A34" | 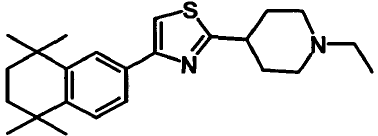 |
| "A35" | 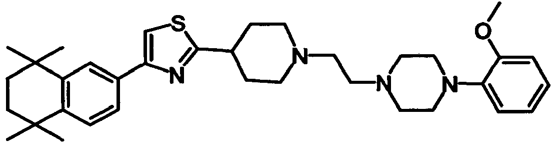 |
| "A36" | 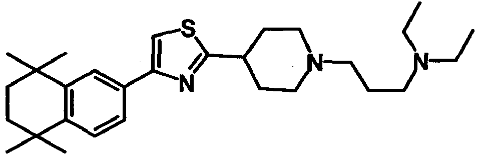 |
| "A37" | (2-{4-[4-(5,5,8,8-Tétraméthyl-5,6,7,8-tétrahydronaphtalén-2-yl)thiazol-2-yl]pipéridin-1-yl}éthyl)carbamate de tertio-butyle |
| "A38" | 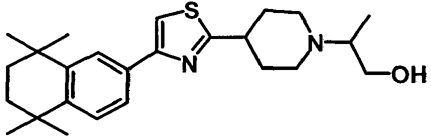 |
| "A39" | 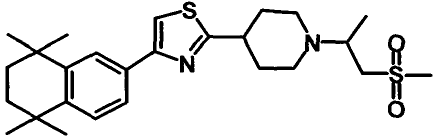 |
| "A40" | 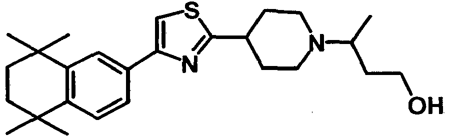 |
| "A41" | (2-{4-[4-(5,5,8,8-Tétraméthyl-5,6,7,8-tétrahydronaphtalén-2-yl)thiazol-2-yl]pipéridin-1-yl}éthyl)carbamate de tertio-butyle |
| "A42" | 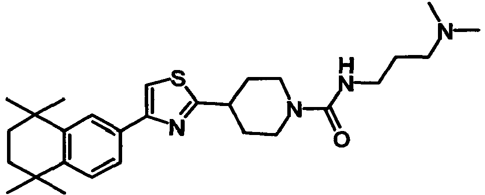 |
| "A43" | 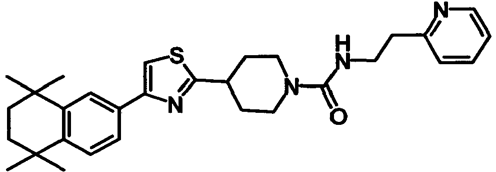 |
| "A44" | 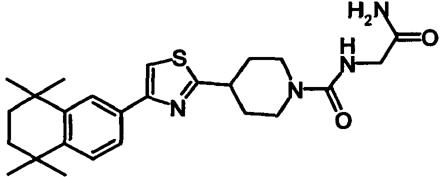 |
| "A45" | 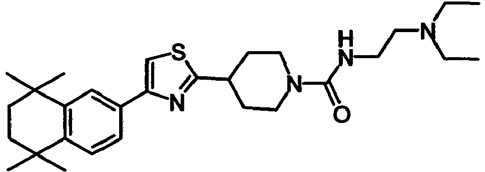 |
| "A46" | 4-[4-(5,5,8,8-Tétraméthyl-5,6,7,8-tétrahydronaphtalén-2-yl)thiazol-2-yl]pipéridine-1-carboxylate de 3-méthoxypropyle |
| "A47" | 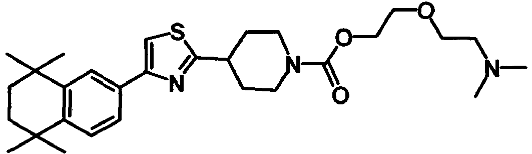 |
| "A48" | 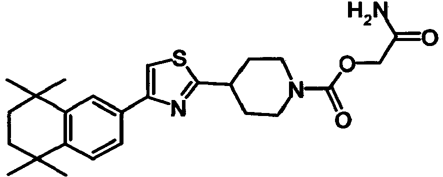 |
| "A49" | 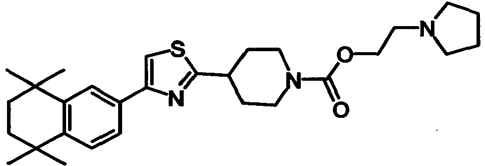 |
| "A50" | 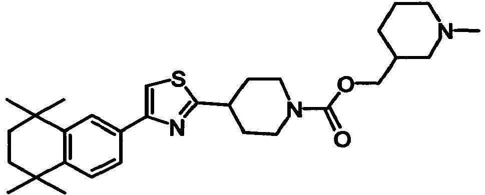 |
| "A51" | 2-Pyridin-4-yl-1-{4-[4-(5,5,8,8-tétraméthyl-5,6,7,8-tétrahydronaphtalén-2-yl)thiazol-2-yl]pipéridin-1-yl}-éthanone |
| "A52" | 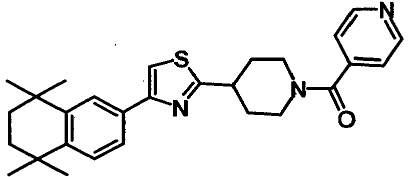 |
| "A53" | 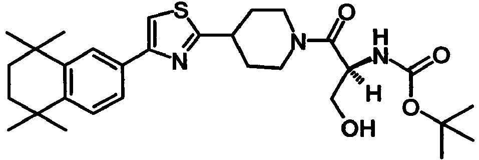 |
| "A54" | 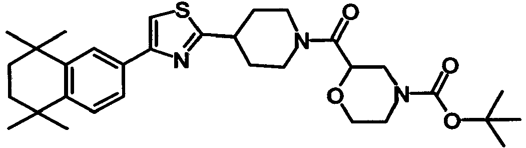 |
| "A55" | 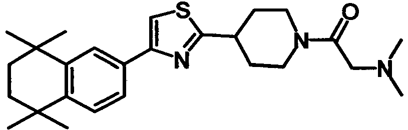 |
| "A56" | 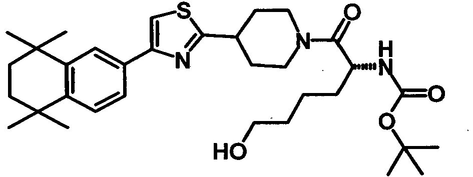 |
| "A57" | 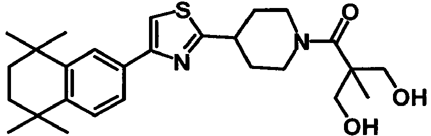 |
| "A58" | 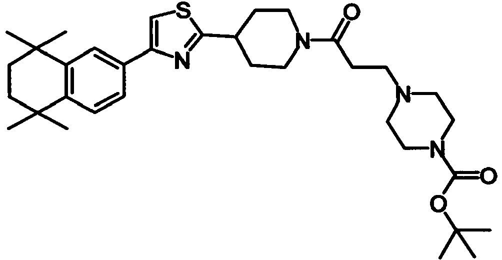 |
| "A59" | 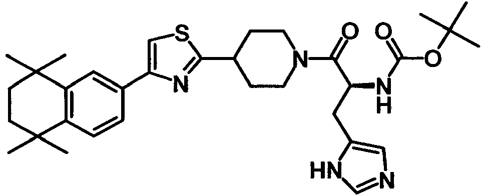 |
| "A60" | 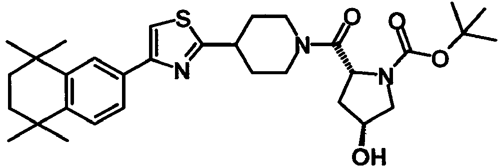 |
| "A61" | 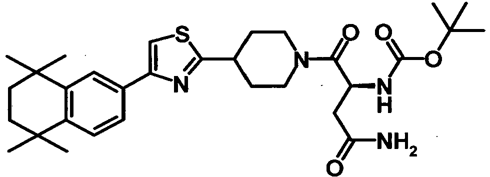 |
| "A62" | 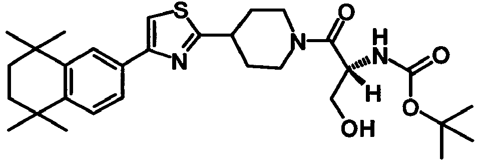 |
| "A63" | (S)-2-Amino-3-(3H-imidazol-4-yl)-1-{4-[4-(5,5,8,8-tétraméthyl-5,6,7,8-tétrahydronaphtalén-2-yl)thiazol-2-yl]-pipéridin-1-yl}propan-1-one |
| "A64" | 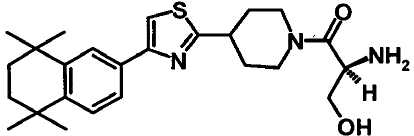 |
| "A65" | 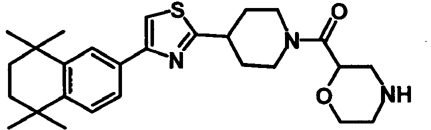 |
| "A66" | 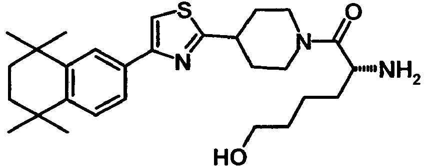 |
| "A67" | 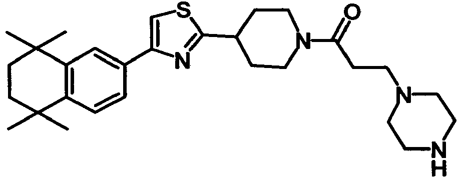 |
| "A68" | 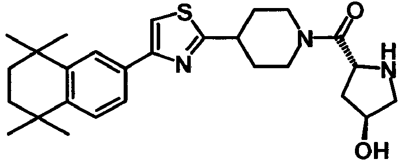 |
| "A69" | 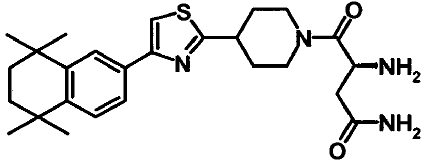 |
| "A70" | 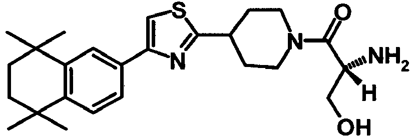 |
| "A71" | 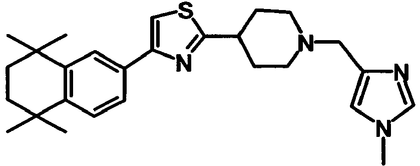 |
| "A72" | 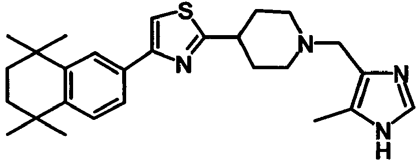 |
| "A73" | 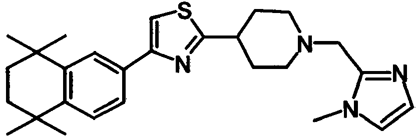 |
| "A74" | 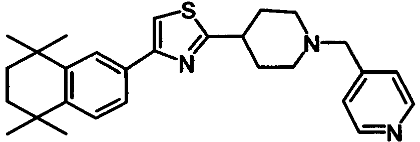 |
| "A75" | 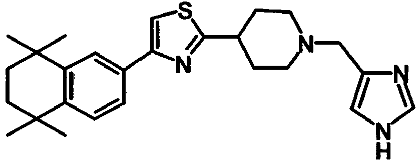 |
| "A76" | 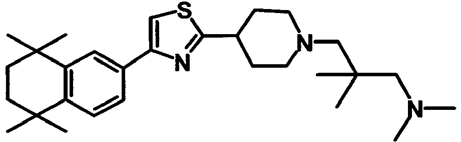 |
| "A77" | 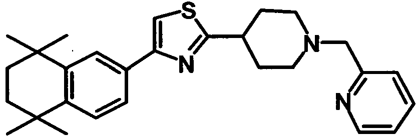 |
| "A78" | 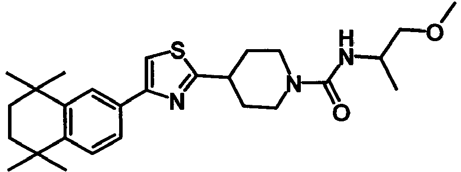 |
| "A79" | 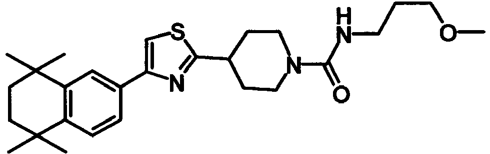 |
| "A80" | 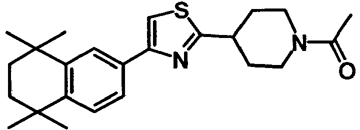 |
| "A81" | 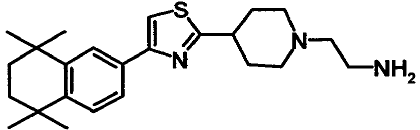 |
| "A82" | 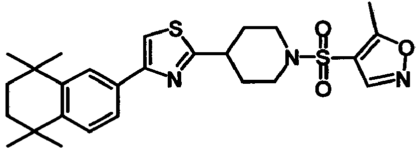 |
| "A83" | 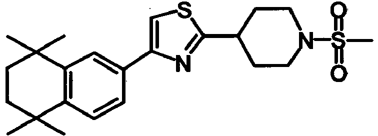 |
| "B1" | 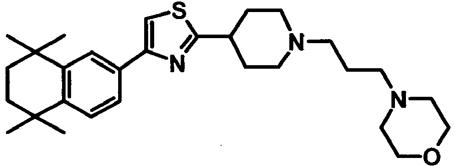 |
| "B2" | 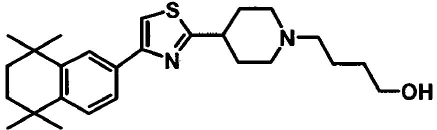 |
| "B3" | 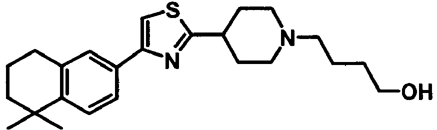 |
| "B4" | 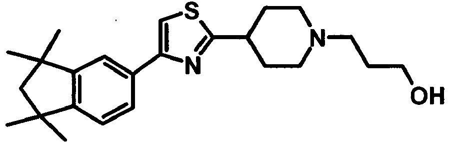 |
| "B5" | 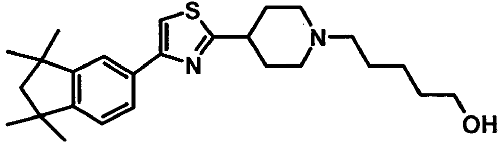 |
| "B6" | 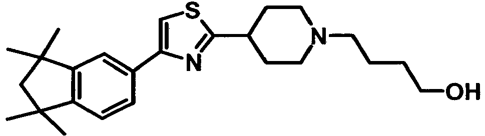 |
| "B7" | 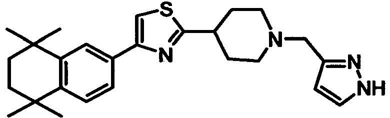 |
| "B8" | 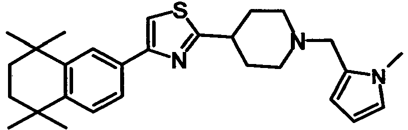 |
| "B9" | 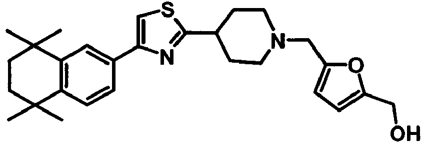 |
| "B10" | 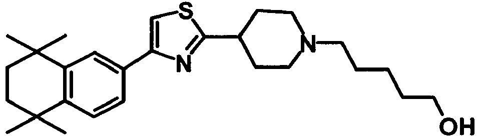 |
| "B11" | 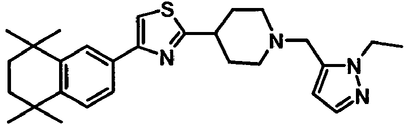 |
| "B12" | 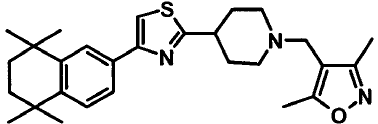 |
| "B13" | 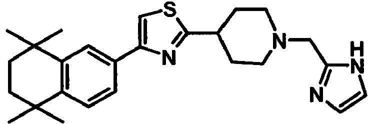 |
| "B14" | 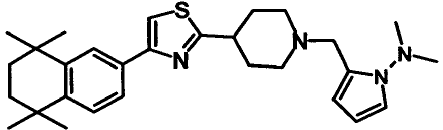 |
| "B15" | 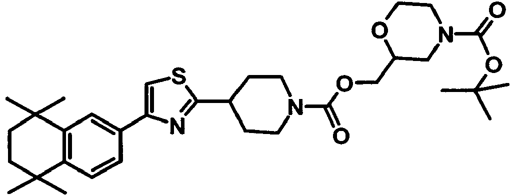 |
| "B16" | 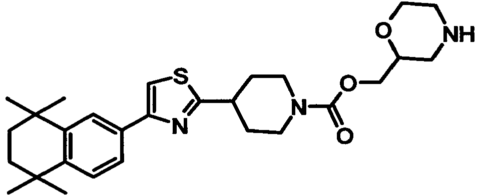 |
et de sels, tautomères et stéréoisomères pharmaceutiquement utilisables de ceux-ci, y compris des mélanges de ceux-ci en toutes proportions.

14. Composés de formule I 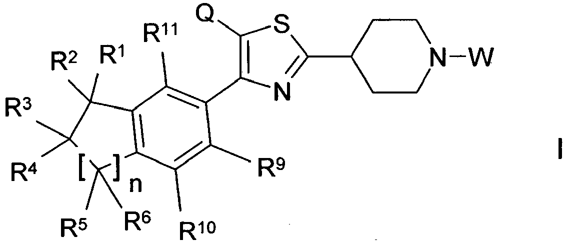 dans laquelle
R¹, R², R³, R⁴, R⁵, R⁶ désignent chacun, indépendamment les uns des autres, H ou A',
Q désigne H, A, Hal, COOR⁷ ou CON(R⁷R^{7'}),
W désigne [C(R⁸R⁸)]ₚZ, CO-[C(R⁸R⁸)]ₚZ, CO-N(R⁸)-[C(R⁸R⁸)]ₚZ, CO-O-[C(R⁸R^{8'})]pZ, SO₂-[C(R⁸R^{8'})]ₚZ, CO[C(R⁸R⁸)]ₚNHCOOA ou [C(R⁸R⁸)]ₚNHCOOA,
R⁷, R^{7'} désignent chacun, indépendamment l'un de l'autre, H ou A',
R⁸, R^{8'} désignent chacun, indépendamment l'un de l'autre, H, A, OA, NAA' ou Hét,
R⁹ désigne H ou A',
R¹⁰, R¹¹ désignent chacun, indépendamment l'un de l'autre, H, A' ou OH,
Z désigne Hét, Ar ou A,
A désigne alkyle non ramifié ou ramifié ayant 1-10 atomes de C, où 1-7 atomes de H peuvent être remplacés par F, Cl, Br, OH et/ou OCH₃,
et/ou où un ou deux groupements CH₂ non adjacents peuvent être remplacés par des groupements O, S, SO, SO₂, CO, COO, NR⁷, NR⁷CO, CONR⁷, CH=CH et/ou CH≡CH
ou
alkyle cyclique ayant 3-7 atomes de C,
Ar désigne phényle, naphtyle ou biphényle, chacun d'entre eux étant non substitué ou mono-, di-, tri-, tétra- ou pentasubstitué par Hal, A, [C(R⁷R^{7'})]ₚOR⁷, [C(R⁷R^{7'})]ₚN(R⁷)₂, SR⁷, NO₂, CN, CHO, COOR⁷, CON(R⁷R^{7'}), NR⁷COA, NR⁷SO₂A, SO₂N(R⁷R^{7'}), S(O)ₘA, O[C(R⁷R^{7'})]ₚN(R⁷)₂, O[C(R⁷R^{7'})₂]Hét, NHCOOA, NHCON(R⁷R^{7'}), NHCOO[C(R⁷R^{7'})]ₚN(R⁷)₂, NHCOO[C(R⁷R^{7'})]ₚHét, NHCONH[C(R⁷R^{7'})]ₚN(R⁷)₂, NHCONH[C(R⁷R⁷)]ₚHét, OCONH[C(R⁷R^{7'})]ₚN(R⁷)₂, OCONH[C(R⁷R⁷)]ₚHét, CONR⁷[C(R⁷R^{7'})]ₚN(R⁷)₂, CONR⁷[C(R⁷R⁷)]ₚHét, COA, [C(R⁷R^{7'})]ₚHét, [C(R⁷R^{7'})]ₚAr', CO[C(R⁷R^{7'})]ₚHét, CO[C(R⁷R⁷)]ₚAr', CONR⁷[C(R⁷R^{7'})]ₚAr', COO[C(R⁷R⁷)]ₚHét, COO[C(R⁷R^{7'})]ₚAr', S(O)ₘ[C(R⁷R^{7'})]ₚHét et/ou S(O)m[C(R⁷R^{7'})]ₚAr',
Ar' désigne phényle qui est non substitué ou mono-, di-, tri-, tétra- ou pentasubstitué par Hal, A, OH, OA', NH₂, NHA, NAA', CN, CHO, COR⁷, CON(R⁷R^{7'}), NR⁷COA, NR⁷SO₂A, SO₂N(R⁷R⁷), S(O)ₘA et/ou COA,
A' désigne alkyle non ramifié ou ramifié ayant 1-8 atomes de C, où 1-7 atomes de H peuvent être remplacés par F, Cl et/ou Br,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OR⁷, [C(R⁷R^{7'})]ₚAr, [C(R⁷R^{7'})]ₚHét', COA, CO[C(R⁷R^{7'})]ₚAr, CO[C(R⁷R^{7'})]ₚHét', CON(R⁸)[C(R⁷R⁷)]ₚAr, CON(R⁸)[C(R⁷R^{7'})]ₚHét', CON(R⁷R^{7'}), COOR⁷, COO[C(R⁷R^{7'})]ₚAr, COO[C(R⁷R^{7'})]ₚHét', S(O)ₘA, S(O)ₘAr, S(O)ₘHét', NO₂, CN, NR⁷COOA, NR⁷COOAr, NR⁷COOHét', OCONHA', OCONHAr, OCONHHét', NR⁷SO₂A, NR⁷SO₂Ar, NR⁷SO₂Hét', SO₂N(R⁷)A, SO₂N(R⁷)Ar, SO₂N(R⁷)Hét', CHO, =S, =NH, =NA, oxy (-O-) et/ou =O (oxygène du carbonyle),
Hét' désigne un hétérocycle monocyclique saturé ayant de 1 à 2 atomes de N et/ou O, qui peut être mono- ou disubstitué par A, OA, OH, Hal et/ou =O (oxygène du carbonyle),
m désigne 0, 1 ou 2,
n désigne 1, 2 ou 3,
p désigne 0, 1, 2, 3 ou 4,
et les sels, tautomères et stéréoisomères pharmaceutiquement utilisables de ceux-ci, y compris des mélanges de ceux-ci en toutes proportions;
où les composés de formule I dans laquelle
a) W = COCH₂Hét;
b) n = 2 et W = alkyle non substitué ayant 1-10 atomes de C ou cycloalkyle ayant 3-7 atomes de C,
sont exclus.

15. Composés selon la revendication 14, dans lesquels
R¹, R² désignent chacun, indépendamment l'un de l'autre, H ou A',
R³, R⁴ désignent H,
R⁵, R⁶ désignent chacun, indépendamment l'un de l'autre, H ou A',
et les sels, tautomères et stéréoisomères pharmaceutiquement utilisables de ceux-ci, y compris des mélanges de ceux-ci en toutes proportions.

16. Composés selon la revendication 14 ou 15, dans lesquels
Q désigne H,
et les sels, tautomères et stéréoisomères pharmaceutiquement utilisables de ceux-ci, y compris des mélanges de ceux-ci en toutes proportions.

17. Composés selon l'une ou plusieurs parmi les revendications 14-16, dans lesquels
W désigne [C(R⁸R^{8'})]ₚZ, CO-[C(R⁸R^{8'})]ₚZ, CO-N(R⁸)-[C(R⁸R^{8'})]ₚZ, CO-O-[C(R⁸R^{8'})]ₚZ, CO[C(R⁸R^{8'})]ₚNHCOOA ou [C(R⁸R^{8'})]ₚNHCOOA,
et les sels, tautomères et stéréoisomères pharmaceutiquement utilisables de ceux-ci, y compris des mélanges de ceux-ci en toutes proportions.

18. Composés selon l'une ou plusieurs parmi les revendications 14-16, dans lesquels
R⁷, R^{7'} désignent chacun, indépendamment l'un de l'autre, H ou A',
R⁸, R^{8'} désignent chacun, indépendamment l'un de l'autre, H, A ou Hét,
et les sels, tautomères et stéréoisomères pharmaceutiquement utilisables de ceux-ci, y compris des mélanges de ceux-ci en toutes proportions.

19. Composés selon l'une ou plusieurs parmi les revendications 14-18, dans lesquels
Z désigne Hét ou A,
et les sels, tautomères et stéréoisomères pharmaceutiquement utilisables de ceux-ci, y compris des mélanges de ceux-ci en toutes proportions.

20. Composés selon l'une ou plusieurs parmi les revendications 16-21, dans lesquels
A désigne alkyle non ramifié ou ramifié ayant 1-10 atomes de C, où 1-7 atomes de H peuvent être remplacés par F, Cl, Br, OH et/ou OCH₃,
et/ou où un ou deux groupements CH₂ non adjacents peuvent être remplacés par des groupements O, SO₂, NR⁷, NR⁷CO et/ou CONR⁷,
et les sels, tautomères et stéréoisomères pharmaceutiquement utilisables de ceux-ci, y compris des mélanges de ceux-ci en toutes proportions.

21. Composés selon l'une ou plusieurs parmi les revendications 14-20, dans lesquels
Ar désigne phényle qui est non substitué ou mono-, di-, tri-, tétra- ou pentasubstitué par Hal et/ou [C(R⁷R^{7'})]ₚOR⁷,
et les sels, tautomères et stéréoisomères pharmaceutiquement utilisables de ceux-ci, y compris des mélanges de ceux-ci en toutes proportions.

22. Composés selon l'une ou plusieurs parmi les revendications 14-21, dans lesquels
Hét désigne un hétérocycle mono- ou bicyclique saturé, insaturé ou aromatique ayant de 1 à 4 atomes de N, O et/ou S, qui peut être non substitué ou mono-, di- ou trisubstitué par A, OR⁷, [C(R⁷R^{7'})]ₚAr, COOR⁷ et/ou =O (oxygène du carbonyle),
et les sels, tautomères et stéréoisomères pharmaceutiquement utilisables de ceux-ci, y compris des mélanges de ceux-ci en toutes proportions.

23. Composés selon l'une ou plusieurs parmi les revendications 14-22, dans lesquels
Hét désigne pyrrolidinyle, pipéridinyle, morpholinyle, oxazolidinyle, tétrahydroquinazolinyle, pipérazinyle, thiazolyle, furyle, thiényle, pyrrolyle, imidazolyle, pyrazolyle, oxazolyle, isoxazolyle, isothiazolyle, pyridyle, pyrimidinyle, triazolyle, tétrazolyle, oxadiazolyle ou thiadiazolyle, chacun d'entre eux étant non substitué ou mono-, di- ou trisubstitué par A, OR⁷, [C(R⁷R^{T})]ₚAr, COOR⁷ et/ou =O (oxygène du carbonyle),
et les sels, tautomères et stéréoisomères pharmaceutiquement utilisables de ceux-ci, y compris des mélanges de ceux-ci en toutes proportions.

24. Composés selon l'une ou plusieurs parmi les revendications 14-23, dans lesquels
R¹, R² désignent chacun, indépendamment l'un de l'autre, H ou A',
R³, R⁴ désignent H,
R⁵, R⁶ désignent chacun, indépendamment l'un de l'autre, H ou A',
R⁷, R^{7'} désignent chacun, indépendamment l'un de l'autre, H ou A',
R⁸, R^{8'} désignent chacun, indépendamment l'un de l'autre, H, A ou Hét,
R⁹ désigne H ou A',
R¹⁰, R¹¹ désignent chacun, indépendamment l'un de l'autre, H, A' ou OH,
Q désigne H,
W désigne [C(R⁸R^{8'})]ₚZ, CO-[C(R⁸R⁸)]ₚZ, CO-N(R⁸)-[C(R⁸R⁸)]ₚZ, CO-O-[C(R⁸R^{8'})]ₚZ, CO[C(R⁸R⁸)]ₚNHCOOA ou [C(R⁸R⁸)]ₚNHCOOA,
Z désigne Hét ou A,
A désigne alkyle non ramifié ou ramifié ayant 1-10 atomes de C, où 1-7 atomes de H peuvent être remplacés par F, Cl, Br, OH et/ou OCH₃,
et/ou où un ou deux groupements CH₂ non adjacents peuvent être remplacés par des groupements O, NR⁷, SO₂, NR⁷CO et/ou CONR⁷,
A' désigne alkyle non ramifié ou ramifié ayant 1-8 atomes de C, où 1-7 atomes de H peuvent être remplacés par F, Cl et/ou Br,
Ar désigne phényle qui est non substitué ou mono-, di-, tri-, tétra- ou pentasubstitué par Hal et/ou [C(R⁷R^{7'})]ₚOR⁷,
Hét désigne un hétérocycle mono- ou bicyclique saturé, insaturé ou aromatique ayant de 1 à 4 atomes de N, O et/ou S, qui peut être non substitué ou mono-, di- ou trisubstitué par A, OR⁷, [C(R⁷R^{7'})]ₚAr, COOR⁷ et/ou =O (oxygène du carbonyle),
n désigne 1 ou 2,
p désigne 0, 1, 2, 3 ou 4,
et les sels, tautomères et stéréoisomères pharmaceutiquement utilisables de ceux-ci, y compris des mélanges de ceux-ci en toutes proportions;
où les composés de formule I dans laquelle
a) W = COCH₂Hét;
b) n = 2 et W = alkyle non substitué ayant 1-10 atomes de C ou cycloalkyle ayant 3-7 atomes de C,
sont exclus.

25. Composés selon la revendication 14, choisis dans le groupe constitué par
| n° | Structure et/ou nom |
|---|---|
| "A1" | 4-(2-Méthyl-3-{4-[4-(5,5,8,8-tétraméthyl-5,6,7,8-tétrahydro-naphtalén-2-yl)thiazol-2-yl]pipéridin-1-yl}-propyl)morpholine |
| "A2" | 1'-Éthyl-4-[4-(5,5,8,8-tétraméthyl-5,6,7,8-tétrahydronaphtalén-2-yl)thiazol-2-yl]-[1,3']bipipéridinyle |
| "A3" | 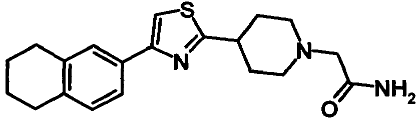 |
| "A4" | 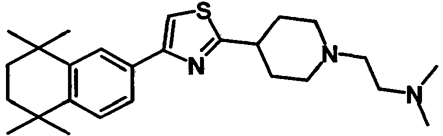 |
| "A5" | 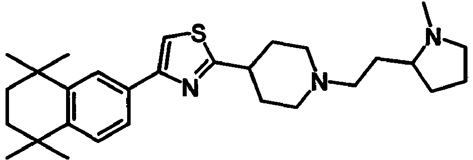 |
| "A6" | 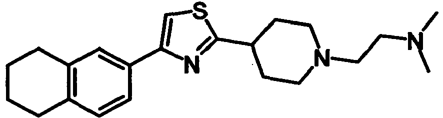 |
| "A7" | 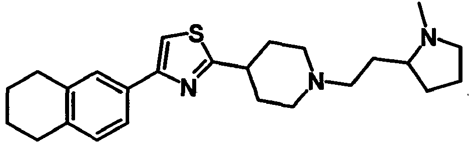 |
| "A8" | 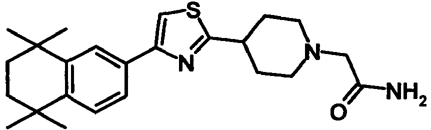 |
| "A9" | 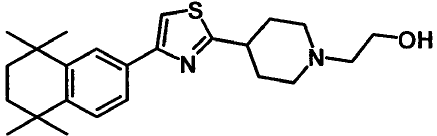 |
| "A10" | 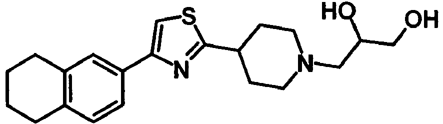 |
| "A11" | 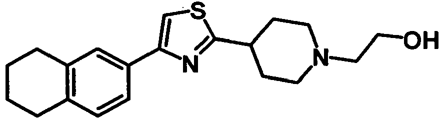 |
| "A12" | 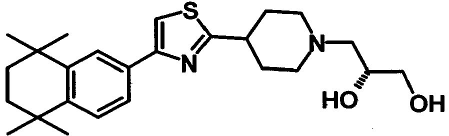 |
| "A13" | 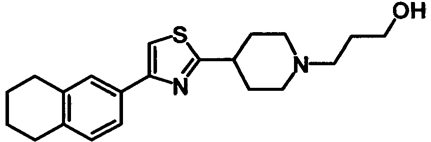 |
| "A14" | 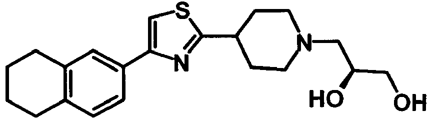 |
| "A15" | 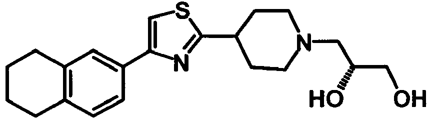 |
| "A16" | 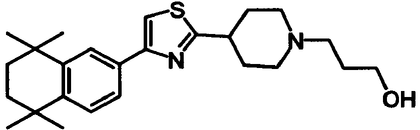 |
| "A17" | 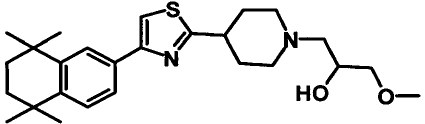 |
| "A18" | 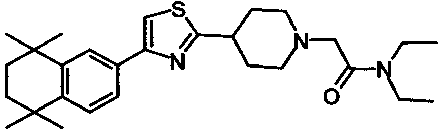 |
| "A19" | 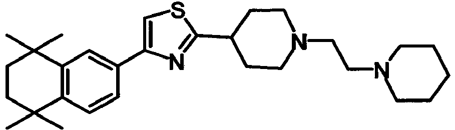 |
| "A20" | 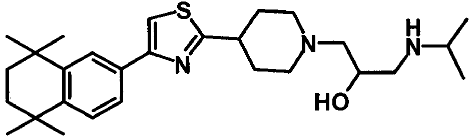 |
| "A21" | 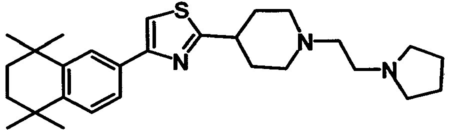 |
| "A22" | 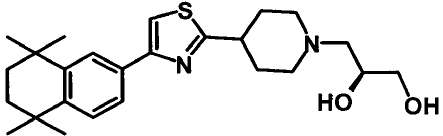 |
| "A23" | 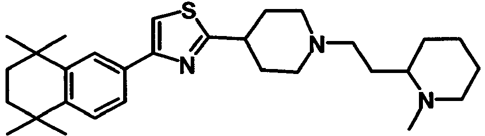 |
| "A24" | 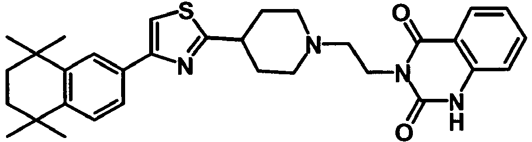 |
| "A25" | 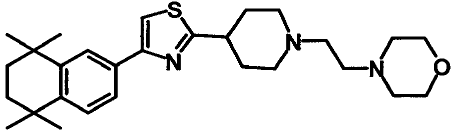 |
| "A26" | 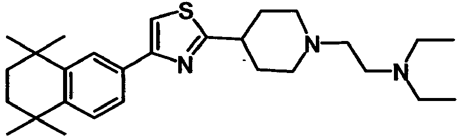 |
| "A27" | 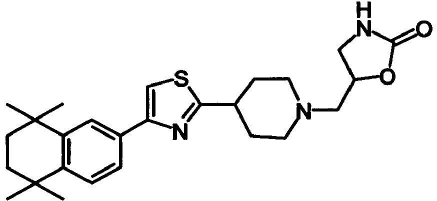 |
| "A28" | 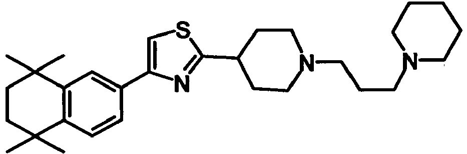 |
| "A29" | 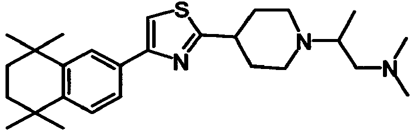 |
| "A30" | 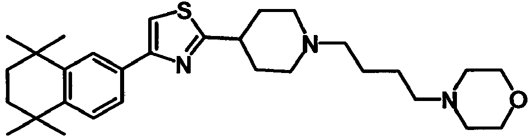 |
| "A31" | 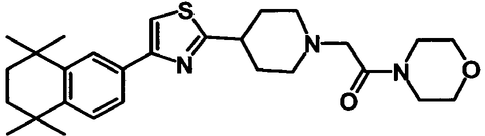 |
| "A32" | 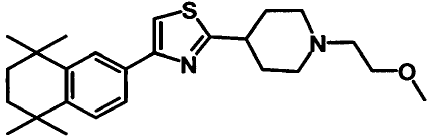 |
| "A33" | 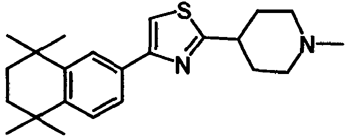 |
| "A34" | 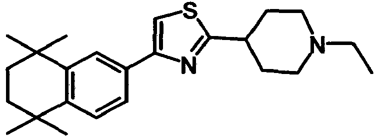 |
| "A35" | 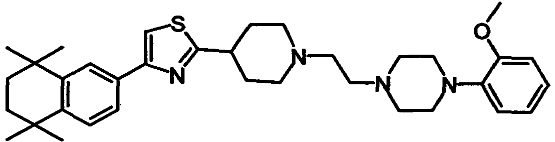 |
| "A36" | 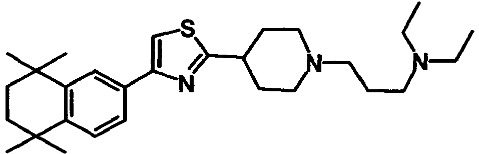 |
| "A37" | (2-{4-[4-(5,5,8,8-Tétraméthyl-5,6,7,8-tétrahydronaphtalén-2-yl)thiazol-2-yl]pipéridin-1-yl}éthyl)carbamate de tertio-butyle |
| "A38" | 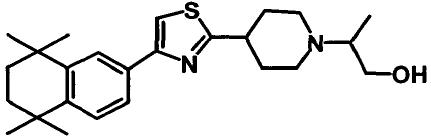 |
| "A39" | 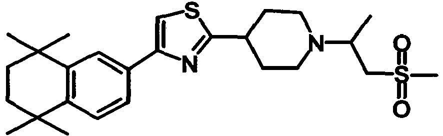 |
| "A40" | 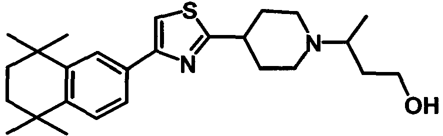 |
| "A41" | (2-{4-[4-(5,5,8,8-Tétraméthyl-5,6,7,8-tétrahydronaphtalén-2-yl)thiazol-2-yl]pipéridin-1-yl}éthyl)carbamate de tertio-butyle |
| "A42" | 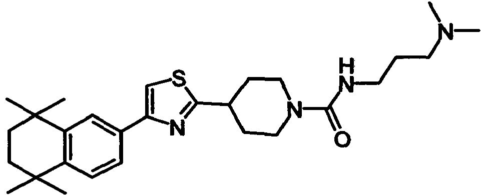 |
| "A43" | 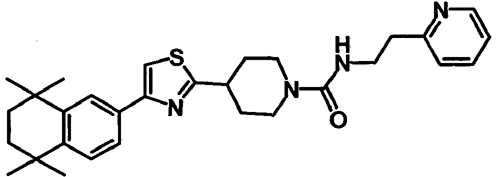 |
| "A44" | 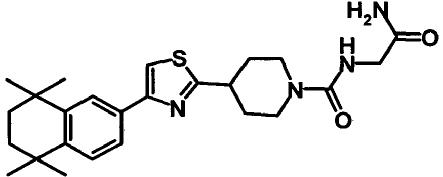 |
| "A45" | 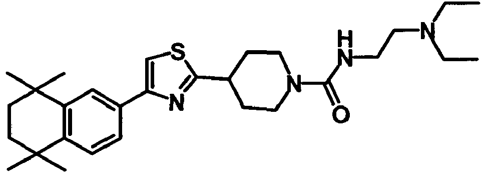 |
| "A46" | 4-[4-(5,5,8,8-Tétraméthyl-5,6,7,8-tétrahydronaphtalén-2-yl)thiazol-2-yl]pipéridine-1-carboxylate de 3-méthoxypropyle |
| "A47" | 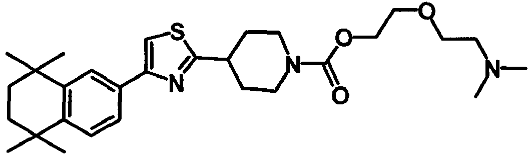 |
| "A48" | 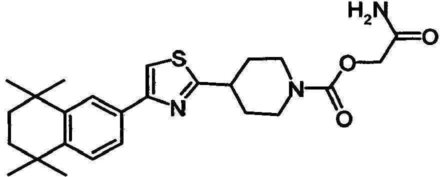 |
| "A49" | 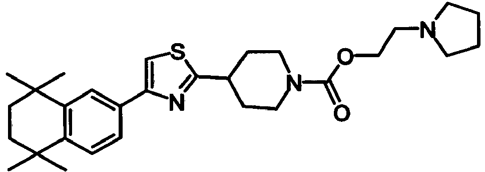 |
| "A50" | 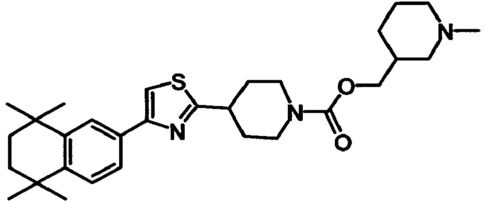 |
| "A51" | 2-Pyridin-4-yl-1-{4-[4-(5,5,8,8-tétraméthyl-5,6,7,8-tétrahydronaphtalén-2-yl)thiazol-2-yl]pipéridin-1-yl}-éthanone |
| "A52" | 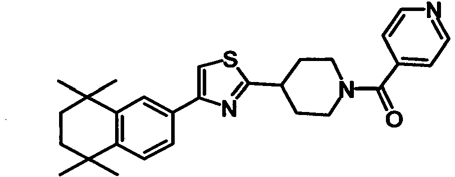 |
| "A53" | 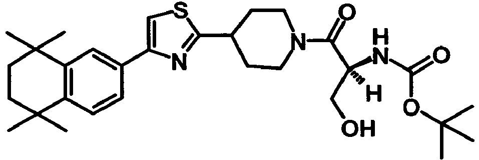 |
| "A54" | 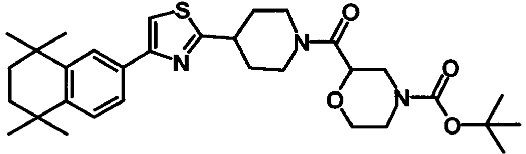 |
| "A55" | 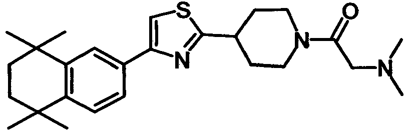 |
| "A56" | 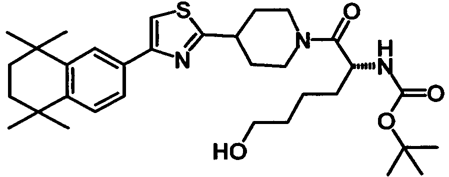 |
| "A57" | 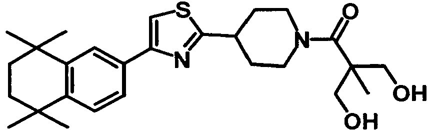 |
| "A58" | 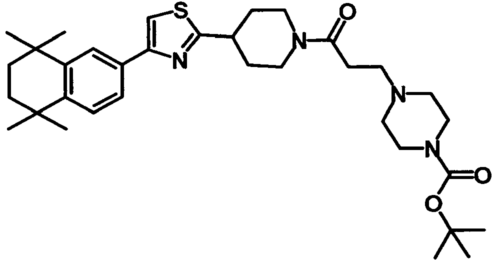 |
| "A59" | 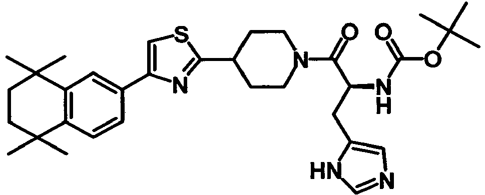 |
| "A60" | 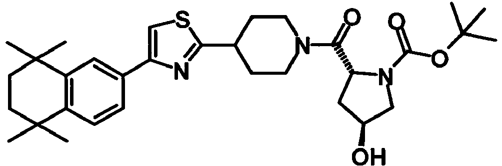 |
| "A61" | 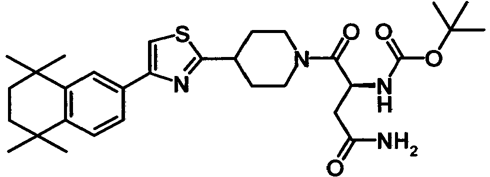 |
| "A62" | 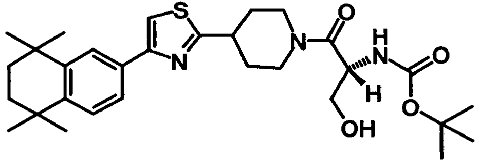 |
| "A63" | (S)-2-Amino-3-(3H-imidazol-4-yl)-1-{4-[4-(5,5,8,8-tétraméthyl-5,6,7,8-tétrahydronaphtalén-2-yl)thiazol-2-yl]-pipéridin-1-yl}propan-1-one |
| "A64" | 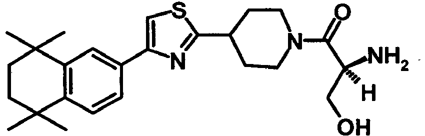 |
| "A65" | 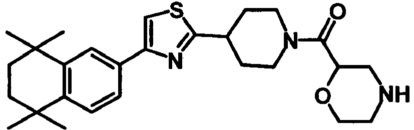 |
| "A66" | 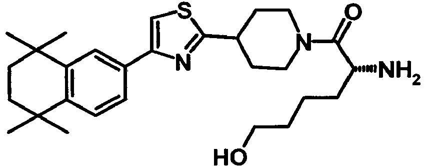 |
| "A67" | 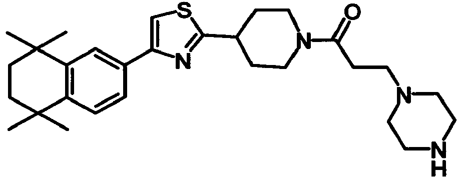 |
| "A68" | 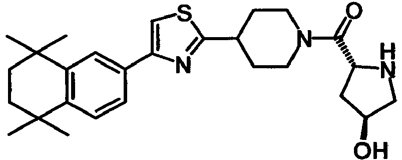 |
| "A69" | 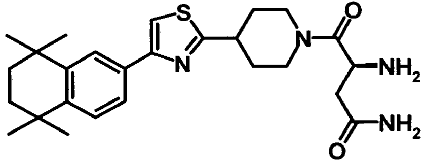 |
| "A70" | 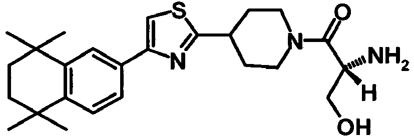 |
| "A71" | 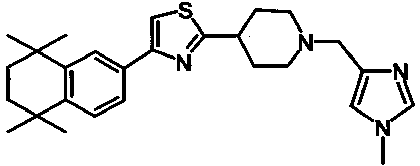 |
| "A72" | 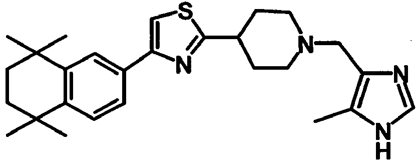 |
| "A73" | 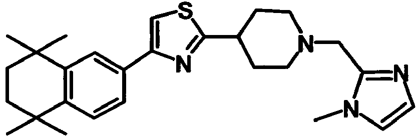 |
| "A74" | 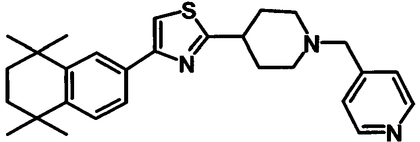 |
| "A75" | 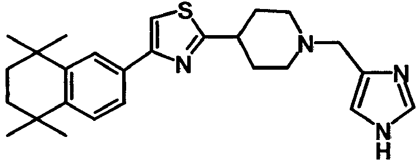 |
| "A76" | 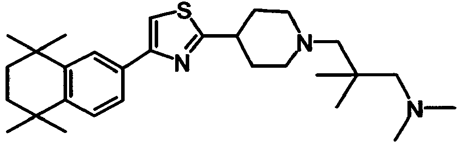 |
| "A77" | 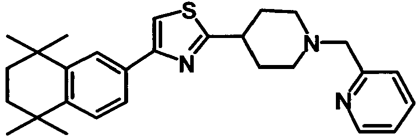 |
| "A78" | 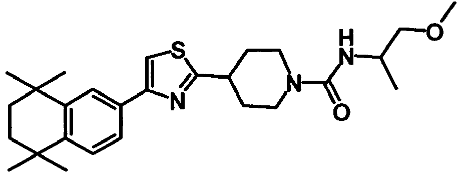 |
| "A79" | 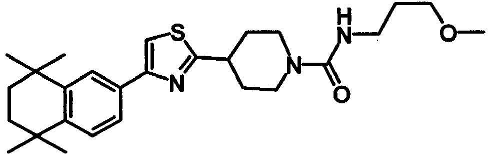 |
| "A80" | 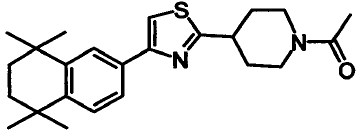 |
| "A81" | 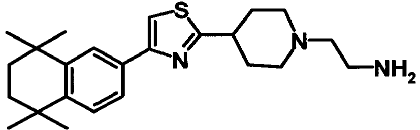 |
| "A82" | 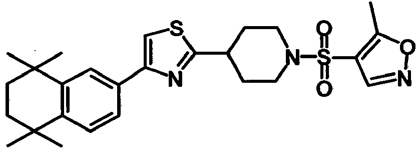 |
| "A83" | 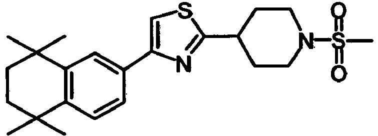 |
| "B1" | 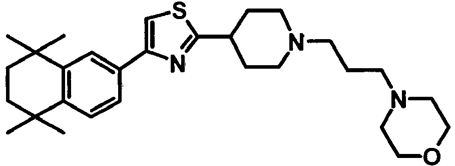 |
| "B2" | 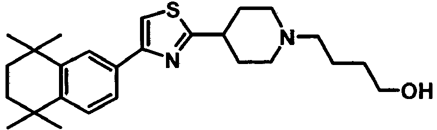 |
| "B3" | 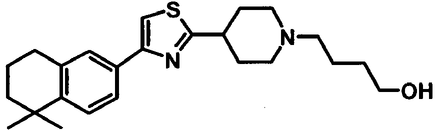 |
| "B4" | 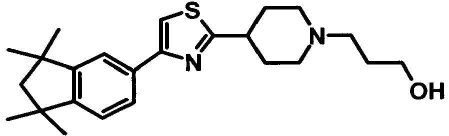 |
| "B5" | 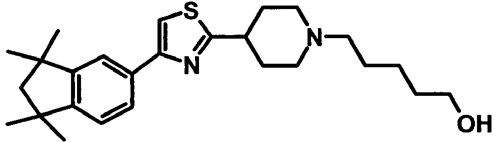 |
| "B6" | 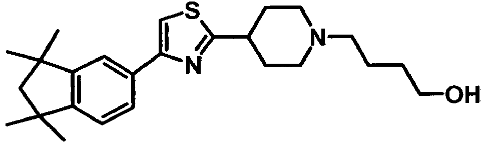 |
| "B7" | 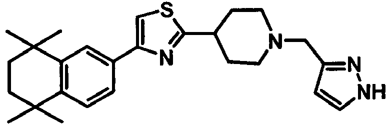 |
| "B8" | 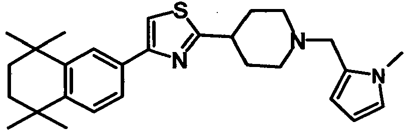 |
| "B9" | 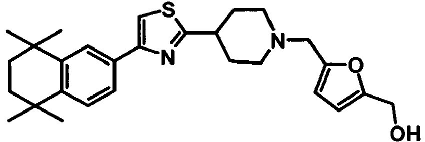 |
| "B10" | 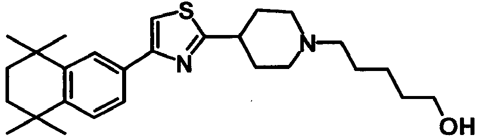 |
| "B11" | 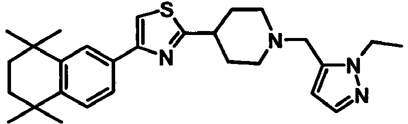 |
| "B12" | 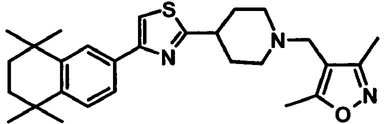 |
| "B13" | 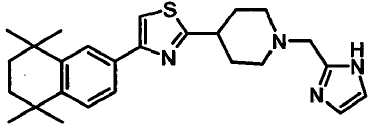 |
| "B14" | 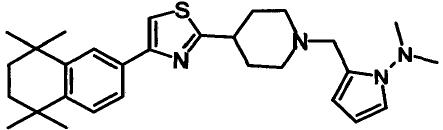 |
| "B15" | 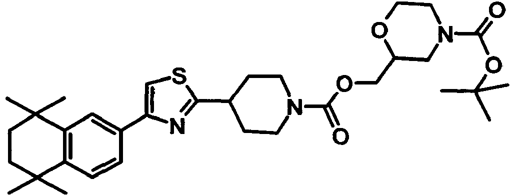 |
| "B16" | 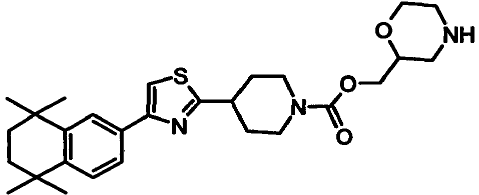 |
et les sels, tautomères et stéréoisomères pharmaceutiquement utilisables de ceux-ci, y compris des mélanges de ceux-ci en toutes proportions.

26. Procédé de préparation de composés de formule I selon les revendications 14-25 et de sels, tautomères et stéréoisomères pharmaceutiquement utilisables de ceux-ci, **caractérisé en ce que**
a) un composé de formule II 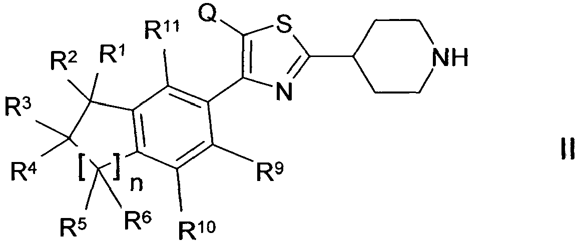 dans laquelle R¹, R², R³, R⁴, R⁵, R⁶, R⁹, R¹⁰, R¹¹, Q et n revêtent les significations indiquées selon la revendication 1,
est réagi avec un composé de formule III
L-W III,
dans laquelle W revêt la signification indiquée selon la revendication 1 et
L désigne Cl, Br, I ou un groupement OH libre ou modifié de manière fonctionnelle et réactive,
ou
b) de préparation de composés de formule I dans laquelle
W désigne [C(R⁸R^{8'})]ₚZ ou [C(R⁸R^{8'})]ₚNHCOOA,
R⁸ désigne H,
p désigne 1, 2, 3 ou 4,
R^{8'}, Z, A revêtent les significations indiquées selon la revendication 1,
un composé de formule II est réagi
i) avec un composé de formule IVa
R^{8'}-CO-[C(R⁸R^{8'})]ₚ₋₁Z IVa,
dans laquelle
p désigne 1, 2, 3 ou 4 et
R^{8'}, Z revêtent les significations indiquées selon la revendication 1,
dans une amination réductrice,
ou
ii) avec un composé de formule IVb
R^{8'}-CO-[C(R⁸R^{8'})]ₚ₋₁NHCOOA IVb,
dans laquelle
p désigne 1, 2, 3 ou 4 et
R^{8'}, A revêtent les significations indiquées selon la revendication 1,
dans une amination réductrice,
ou
c) en ce qu'ils sont libérés à partir de l'un de leurs dérivés fonctionnels par un traitement par un agent de solvolyse ou d'hydrogénolyse,
et/ou
une base ou un acide de formule I est converti(e) en l'un de ses sels.

27. Médicaments comprenant au moins un composé de formule I selon les revendications 14-25 et/ou des sels, tautomères et stéréoisomères pharmaceutiquement utilisables de celui-ci, y compris des mélanges de ceux-ci en toutes proportions, et éventuellement des excipients et/ou des adjuvants.

28. Médicaments comprenant au moins un composé de formule I selon l'une ou plusieurs parmi les revendications 14 à 25, et/ou des sels et stéréoisomères pharmaceutiquement utilisables de celui-ci, y compris des mélanges de ceux-ci en toutes proportions, et au moins un autre composé actif médicamenteux.

29. Ensemble (kit) constitué de conditionnements séparés
(a) d'une quantité efficace d'un composé de formule I selon l'une ou plusieurs parmi les revendications 14 à 25, et/ou de sels et stéréoisomères pharmaceutiquement utilisables de celui-ci, y compris des mélanges de ceux-ci en toutes proportions,
et
(b) d'une quantité efficace d'un autre composé actif médicamenteux.

30. Composés choisis dans le groupe constitué par
| n° | Structure et/ou nom |
|---|---|
| "C2" | 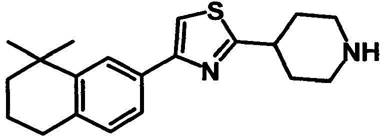 |
| "C3" | 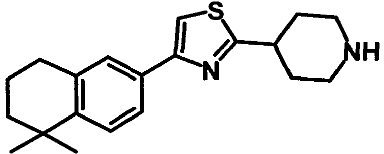 |
| "C4" | 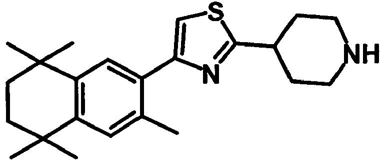 |
| "C6" | 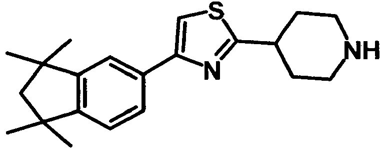 |
| "C7" | 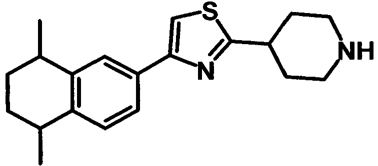 |
| "C8" | 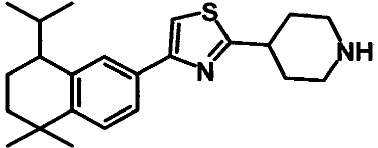 |
| "C9" | 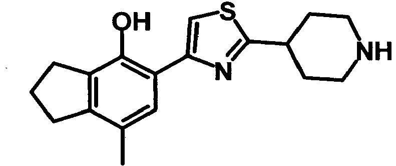 |
| "C10" | 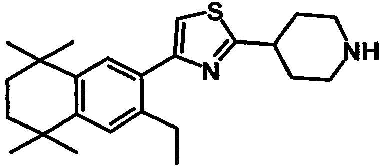 |
| "C11" | 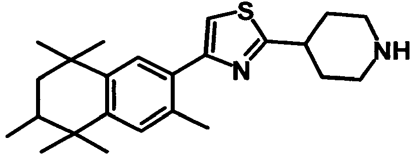 |
| "C12" | 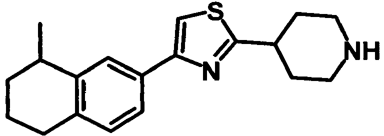 |
et les sels, tautomères et stéréoisomères pharmaceutiquement utilisables de ceux-ci, y compris des mélanges de ceux-ci en toutes proportions.

31. Utilisation de composés choisis dans le groupe constitué par
| n° | Structure et/ou nom |
|---|---|
| "C1" | 4-[4-(5,5,8,8-Tétraméthyl-5,6,7,8-tétrahydronaphtalén-2-yl)thiazol-2-yl]pipéridine |
| "C2" | 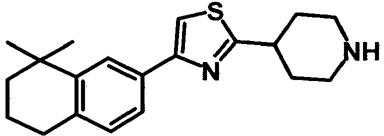 |
| "C3" | 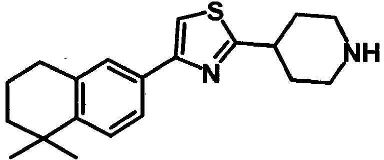 |
| "C4" | 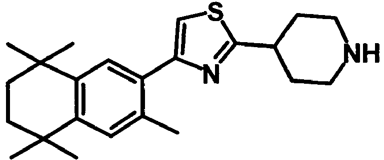 |
| "C5" | 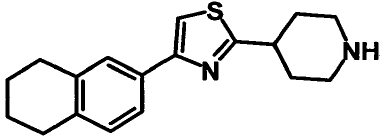 |
| "C6" | 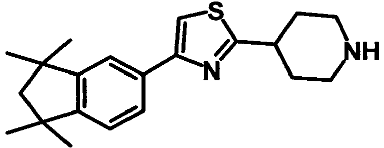 |
| "C7" | 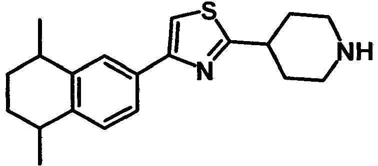 |
| "C8" | 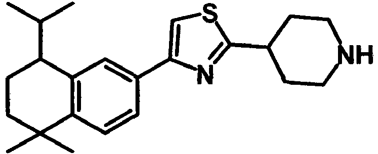 |
| "C9" | 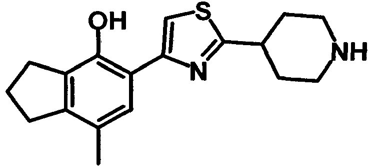 |
| "C10" | 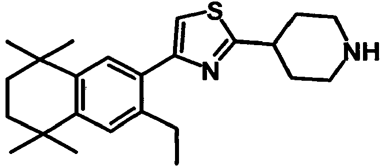 |
| "C11" | 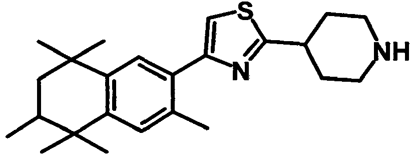 |
| "C12" | 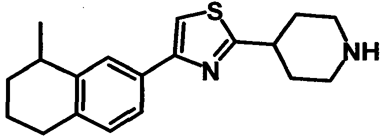 |
et de sels, tautomères et stéréoisomères pharmaceutiquement utilisables de ceux-ci, y compris des mélanges de ceux-ci en toutes proportions, pour la préparation d'un médicament destiné au traitement de maladies, où les maladies à traiter sont choisies dans le groupe constitué par
les maladies hyperprolifératives, les maladies inflammatoires, les maladies angiogènes, les maladies fibrotiques du poumon, du rein, du foie et du coeur.

32. Utilisation selon la revendication 31, dans laquelle la maladie hyper-proliférative est choisie dans le groupe constitué par
le cancer (maladie tumorale), l'athérosclérose, la resténose, la maladie proliférative des cellules mésangiales, le psoriasis.

33. Utilisation selon la revendication 32, dans laquelle la maladie tumorale est choisie dans le groupe constitué par
les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, de la peau, la leucémie monocytique, l'adénocarcinome du poumon, le carcinome du poumon à petites cellules, le cancer du pancréas, le glio-blastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

34. Utilisation selon la revendication 32, dans laquelle la maladie proliférative des cellules mésangiales est choisie dans le groupe constitué par
la glomérulonéphrite, la néphropathie diabétique, la néphrosclérose maligne, le syndrome de microangiopathie thrombotique, le rejet de greffe, la glomérulopathie.

35. Utilisation selon la revendication 31, dans laquelle les maladies inflammatoires sont choisies dans le groupe constitué par
l'affection abdominale inflammatoire, l'arthrite, l'athérosclérose, l'asthme, les allergies, les maladies inflammatoires rénales, la sclérose en plaques, la broncho-pneumopathie chronique obstructive, les maladies inflammatoires cutanées, les maladies parodontales, le psoriasis, les maladies immunitaires favorisées par les lymphocytes T.

36. Utilisation selon la revendication 35, dans laquelle l'affection abdominale inflammatoire est choisie dans le groupe constitué par
la rectocolite hémorragique, la maladie de Crohn, la colite non spécifique.

37. Utilisation selon la revendication 35, dans laquelle les maladies immunitaires favorisées par les lymphocytes T sont choisies dans le groupe constitué par
l'encéphalomyélite allergique, la névrite allergique, le rejet de greffe, la réaction du greffon contre l'hôte, la myocardite, la thyroïdite, la néphrite, le lupus érythémateux disséminé, le diabète sucré insulinodépendant.

38. Utilisation selon la revendication 35, dans laquelle la maladie arthritique est choisie dans le groupe constitué par
la polyarthrite rhumatoïde, l'arthrose, le syndrome de Caplan-Colinet, le syndrome de Felty, le syndrome de Sjögren, la spondylarthrite ankylosante, la maladie de Still, la chondrocalcinose, l'arthrite métabolique, le rhumatisme articulaire aigu, la maladie de Reiter, le syndrome de Wissler-Fanconi.

39. Utilisation selon la revendication 35, dans laquelle la maladie inflammatoire rénale est choisie dans le groupe constitué par
la glomérulonéphrite, les lésions glomérulaires, le syndrome néphrotique, la néphrite interstitielle, le lupus néphrétique, le syndrome de Goodpasture, la granulomatose de Wegener, la vascularite rénale, la néphropathie à IgA, la néphropathie glomérulaire idiopathique.

40. Utilisation selon la revendication 35, dans laquelle la maladie inflammatoire cutanée est choisie dans le groupe constitué par
le psoriasis, la dermite atopique, la sensibilité de contact, l'acné.

41. Utilisation selon la revendication 31, dans laquelle les maladies angiogènes sont choisies dans le groupe constitué par
la rétinopathie diabétique, l'arthrite, le cancer, le psoriasis, le sarcome de Kaposi, l'hémangiome, l'angiogenèse myocardique, la néovascularisation des plaques athérosclérotiques, les maladies oculaires angiogènes, la néovascularisation choroïdienne, la fibroplasie rétrolentale, la dégénérescence maculaire, le rejet de greffe cornéenne, la rubéose de l'iris, le glaucome neurovasculaire, le syndrome d'Osler-Weber.

42. Médicaments comprenant au moins un composé choisi dans le groupe constitué par
| n° | Structure et/ou nom |
|---|---|
| "C2" | 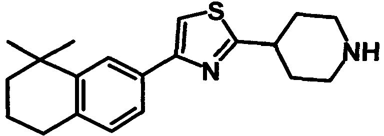 |
| "C3" | 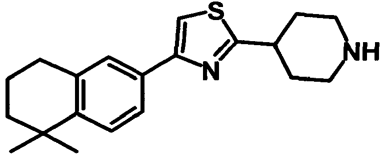 |
| "C4" | 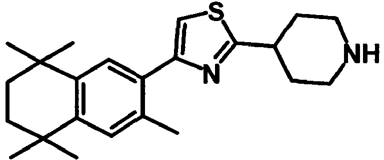 |
| "C6" | 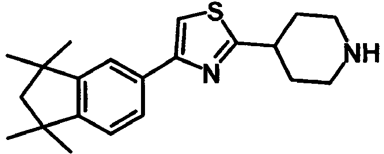 |
| "C7" | 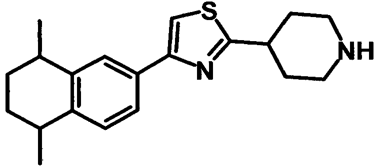 |
| "C8" | 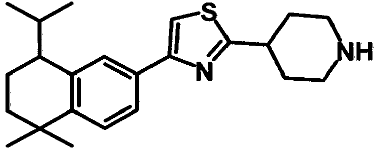 |
| "C9" | 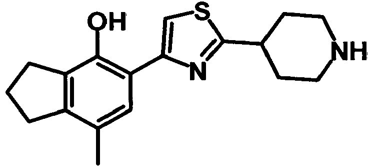 |
| "C10" | 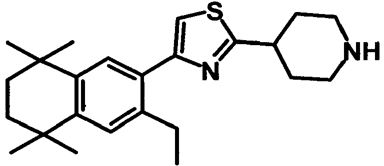 |
| "C11" | 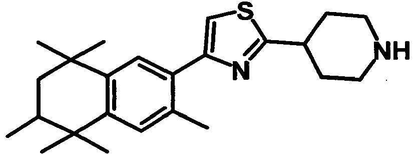 |
| "C12" | 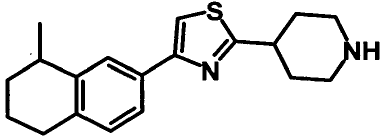 |
et/ou des sels, tautomères et stéréoisomères pharmaceutiquement utilisables de celui-ci, y compris des mélanges de ceux-ci en toutes proportions, et éventuellement des excipients et/ou des adjuvants.
